# EUROPEAN PATENT APPLICATION

(11) **EP 2 510 941 A2**
(43) Date of publication of application: **17.10.2012**
(21) Application number: 12171564.3
(22) Date of filing: 20.02.2008
(51) Int. Cl.: A61K 38/17, C07K 16/28, A61K 39/395

(54) **Methods of treating multiple sclerosis by administration of alpha-fetoprotein in combination with an integrin antagonist**

(30) Priority: 20.02.2007 US 902216 P
(62) Divisional of application: 08725824.0
(71) Applicant: Merrimack Pharmaceuticals, Inc., Cambridge, MA 02139 (US)
(72) Inventor: Stewart, Edward, J., Winchester, MA Massachusetts 01890 (US); Briskin, Michael, Lexington, MA Massachusetts 02421 (US)
(74) Representative: Harrison Goddard Foote

(57) **Abstract**

The present invention relates to methods for treating multiple sclerosis by administering therapeutically effective amounts of an alpha-fetoprotein polypeptide (or a biologically active fragment, derivative, or analog thereof) and an integrin antagonist (e.g., natalizumab) to a patient in need thereof. Also disclosed are compositions and kits that comprise therapeutically effective amounts of an alpha-fetoprotein polypeptide (or a biologically active fragment, derivative, or analog thereof) and an integrin antagonist (e.g., natalizumab).

SEQUENCE LISTING

<110>  Merrimack Pharmaceuticals, Inc. et al.

<120>  Methods of Treating Multiple Sclerosis by Administration of
       Alpha-Fetoprotein in Combination with an Integrin Antagonist

<130>  06727/025WO2

<140>  PCT/US08/02231
<141>  2008-02-20

<150>  US 60/902,216
<151>  2007-02-20

<160>  36

<170>  PatentIn version 3.3

<210>  1
<211>  591
<212>  PRT
<213>  Homo sapiens

<400>  1

Arg Thr Leu His Arg Asn Glu Tyr Gly Ile Ala Ser Ile Leu Asp Ser
1               5                   10                  15

Tyr Gln Cys Thr Ala Glu Ile Ser Leu Ala Asp Leu Ala Thr Ile Phe
            20                  25                  30

Phe Ala Gln Phe Val Gln Glu Ala Thr Tyr Lys Glu Val Ser Lys Met
        35                  40                  45

Val Lys Asp Ala Leu Thr Ala Ile Glu Lys Pro Thr Gly Asp Glu Gln
    50                  55                  60

Ser Ser Gly Cys Leu Glu Asn Gln Leu Pro Ala Phe Leu Glu Glu Leu
65                  70                  75                  80

Cys His Glu Lys Glu Ile Leu Glu Lys Tyr Gly His Ser Asp Cys Cys
                85                  90                  95

Ser Gln Ser Glu Glu Gly Arg His Asn Cys Phe Leu Ala His Lys Lys
            100                 105                 110

Pro Thr Pro Ala Ser Ile Pro Leu Phe Gln Val Pro Glu Pro Val Thr
        115                 120                 125

Ser Cys Glu Ala Tyr Glu Glu Asp Arg Glu Thr Phe Met Asn Lys Phe
    130                 135                 140

Ile Tyr Glu Ile Ala Arg Arg His Pro Phe Leu Tyr Ala Pro Thr Ile
145                 150                 155                 160

Leu Leu Trp Ala Ala Arg Tyr Asp Lys Ile Ile Pro Ser Cys Cys Lys
                165                 170                 175

Ala Glu Asn Ala Val Glu Cys Phe Gln Thr Lys Ala Ala Thr Val Thr
            180                 185                 190

Lys Glu Leu Arg Glu Ser Ser Leu Leu Asn Gln His Ala Cys Ala Val
        195                 200                 205

Met Lys Asn Phe Gly Thr Arg Thr Phe Gln Ala Ile Thr Val Thr Lys
    210                 215                 220

Leu Ser Gln Lys Phe Thr Lys Val Asn Phe Thr Glu Ile Gln Lys Leu
225                 230                 235                 240

Val Leu Asp Val Ala His Val His Glu His Cys Cys Arg Gly Asp Val
                245                 250                 255

Leu Asp Cys Leu Gln Asp Gly Glu Lys Ile Met Ser Tyr Ile Cys Ser
            260                 265                 270

Gln Gln Asp Thr Leu Ser Asn Lys Ile Thr Glu Cys Cys Lys Leu Thr
        275                 280                 285

Thr Leu Glu Arg Gly Gln Cys Ile Ile His Ala Glu Asn Asp Glu Lys
    290                 295                 300

Pro Glu Gly Leu Ser Pro Asn Leu Asn Arg Phe Leu Gly Asp Arg Asp
305                 310                 315                 320

Phe Asn Gln Phe Ser Ser Gly Glu Lys Asn Ile Phe Leu Ala Ser Phe
                325                 330                 335

Val His Glu Tyr Ser Arg Arg His Pro Gln Leu Ala Val Ser Val Ile
            340                 345                 350

Leu Arg Val Ala Lys Gly Tyr Gln Glu Leu Leu Glu Lys Cys Phe Gln
        355                 360                 365

Thr Glu Asn Pro Leu Glu Cys Gln Asp Lys Gly Glu Glu Glu Leu Gln
    370                 375                 380

Lys Tyr Ile Gln Glu Ser Gln Ala Leu Ala Lys Arg Ser Cys Gly Leu
385                 390                 395                 400

Phe Gln Lys Leu Gly Glu Tyr Tyr Leu Gln Asn Ala Phe Leu Val Ala
                405                 410                 415

Tyr Thr Lys Lys Ala Pro Gln Leu Thr Ser Ser Glu Leu Met Ala Ile
            420                 425                 430

Thr Arg Lys Met Ala Ala Thr Ala Ala Thr Cys Cys Gln Leu Ser Glu
        435                 440                 445

Asp Lys Leu Leu Ala Cys Gly Glu Gly Ala Ala Asp Ile Ile Ile Gly
    450                 455                 460

His Leu Cys Ile Arg His Glu Met Thr Pro Val Asn Pro Gly Val Gly
465                 470                 475                 480

Gln Cys Cys Thr Ser Ser Tyr Ala Asn Arg Arg Pro Cys Phe Ser Ser
                485                 490                 495

Leu Val Val Asp Glu Thr Tyr Val Pro Pro Ala Phe Ser Asp Asp Lys
            500                 505                 510

Phe Ile Phe His Lys Asp Leu Cys Gln Ala Gln Gly Val Ala Leu Gln
        515                 520                 525

Thr Met Lys Gln Glu Phe Leu Ile Asn Leu Val Lys Gln Lys Pro Gln
    530                 535                 540

Ile Thr Glu Glu Gln Leu Glu Ala Val Ile Ala Asp Phe Ser Gly Leu
545                 550                 555                 560

Leu Glu Lys Cys Cys Gln Gly Gln Glu Gln Glu Val Cys Phe Ala Glu
                565                 570                 575

Glu Gly Gln Lys Leu Ile Ser Lys Thr Arg Ala Ala Leu Gly Val
            580                 585                 590

<210>  2
<211>  2032
<212>  DNA
<213>  Homo sapiens

<400>  2
tccatattgt gcttccacca ctgccaataa caaaataact agcaaccatg aagtgggtgg      60

aatcaatttt tttaattttc ctactaaatt ttactgaatc cagaacactg catagaaatg     120

aatatggaat agcttccata ttggattctt accaatgtac tgcagagata agtttagctg     180

acctggctac catatttttt gcccagtttg ttcaagaagc cacttacaag gaagtaagca     240

aaatggtgaa agatgcattg actgcaattg agaaacccac tggagatgaa cagtcttcag     300

ggtgtttaga aaaccagcta cctgcctttc tggaagaact ttgccatgag aaagaaattt     360

tggagaagta cggacattca gactgctgca gccaaagtga agagggaaga cataactgtt     420

ttcttgcaca caaaaagccc actccagcat cgatcccact tttccaagtt ccagaacctg     480

tcacaagctg tgaagcatat gaagaagaca gggagacatt catgaacaaa ttcatttatg 540

agatagcaag aaggcatccc ttcctgtatg cacctacaat tcttctttgg gctgctcgct 600

atgacaaaat aattccatct tgctgcaaag ctgaaaatgc agttgaatgc ttccaaacaa 660

aggcagcaac agttacaaaa gaattaagag aaagcagctt gttaaatcaa catgcatgtg 720

cagtaatgaa aaattttggg acccgaactt tccaagccat aactgttact aaactgagtc 780

agaagtttac caaagttaat tttactgaaa tccagaaact agtcctggat gtggcccatg 840

tacatgagca ctgttgcaga ggagatgtgc tggattgtct gcaggatggg gaaaaaatca 900

tgtcctacat atgttctcaa caagacactc tgtcaaacaa aataacagaa tgctgcaaac 960

tgaccacgct ggaacgtggt caatgtataa ttcatgcaga aaatgatgaa aaacctgaag 1020

gtctatctcc aaatctaaac aggtttttag gagatagaga ttttaaccaa ttttcttcag 1080

gggaaaaaaa tatcttcttg gcaagttttg ttcatgaata ttcaagaaga catcctcagc 1140

ttgctgtctc agtaattcta agagttgcta aaggatacca ggagttattg gagaagtgtt 1200

tccagactga aaccctcttg gaatgccaag ataaaggaga agaagaatta cagaaataca 1260

tccaggagag ccaagcattg gcaaagcgaa gctgcggcct cttccagaaa ctaggagaat 1320

attacttaca aaatgcgttt ctcgttgctt acacaaagaa agccccccag ctgacctcgt 1380

cggagctgat ggccatcacc agaaaaatgg cagccacagc agccacttgt tgccaactca 1440

gtgaggacaa actattggcc tgtggcgagg gagcggctga cattattatc ggacacttat 1500

gtatcagaca tgaaatgact ccagtaaacc ctggtgttgg ccagtgctgc acttcttcat 1560

atgccaacag gaggccatgc ttcagcagct tggtggtgga tgaaacatat gtccctcctg 1620

cattctctga tgacaagttc attttccata aggatctgtg ccaagctcag ggtgtagcgc 1680

tgcaaacgat gaagcaagag tttctcatta accttgtgaa gcaaaagcca caaataacag 1740

aggaacaact tgaggctgtc attgcagatt tctcaggcct gttggagaaa tgctgccaag 1800

gccaggaaca ggaagtctgc tttgctgaag agggacaaaa actgatttca aaaactcgtg 1860

ctgctttggg agtttaaatt acttcagggg aagagaagac aaaacgagtc tttcattcgg 1920

tgtgaacttt tctctttaat tttaactgat ttaacacttt ttgtgaatta atgaaatgat 1980

aaagactttt atgtgagatt tccttatcac agaaataaaa tatctccaaa tg 2032

<210> 3
<211> 8
<212> PRT
<213> Homo sapiens

<400> 3

Glu Met Thr Pro Val Asn Pro Gly
1               5

<210>  4
<211>  34
<212>  PRT
<213>  Homo sapiens

<400>  4

Leu Ser Glu Asp Lys Leu Leu Ala Cys Gly Glu Gly Ala Ala Asp Ile
1               5                   10                  15

Ile Ile Gly His Leu Cys Ile Arg His Glu Met Thr Pro Val Asn Pro
            20                  25                  30

Gly Val

<210>  5
<211>  197
<212>  PRT
<213>  Homo sapiens

<400>  5

Thr Leu His Arg Asn Glu Tyr Gly Ile Ala Ser Ile Leu Asp Ser Tyr
1               5                   10                  15

Gln Cys Thr Ala Glu Ile Ser Leu Ala Asp Leu Ala Thr Ile Phe Phe
            20                  25                  30

Ala Gln Phe Val Gln Glu Ala Thr Tyr Lys Glu Val Ser Lys Met Val
        35                  40                  45

Lys Asp Ala Leu Thr Ala Ile Glu Lys Pro Thr Gly Asp Glu Gln Ser
    50                  55                  60

Ser Gly Cys Leu Glu Asn Gln Leu Pro Ala Phe Leu Glu Glu Leu Cys
65                  70                  75                  80

His Glu Lys Glu Ile Leu Glu Lys Tyr Gly His Ser Asp Cys Cys Ser
                85                  90                  95

Gln Ser Glu Glu Gly Arg His Asn Cys Phe Leu Ala His Lys Lys Pro
            100                 105                 110

Thr Pro Ala Ser Ile Pro Leu Phe Gln Val Pro Glu Pro Val Thr Ser
        115                 120                 125

Cys Glu Ala Tyr Glu Glu Asp Arg Glu Thr Phe Met Asn Lys Phe Ile
    130                 135                 140

Tyr Glu Ile Ala Arg Arg His Pro Phe Leu Tyr Ala Pro Thr Ile Leu
145                 150                 155                 160

Leu Trp Ala Ala Arg Tyr Asp Lys Ile Ile Pro Ser Cys Cys Lys Ala
                165                 170                 175

Glu Asn Ala Val Glu Cys Phe Gln Thr Lys Ala Ala Thr Val Thr Lys
            180                 185                 190

Glu Leu Arg Glu Ser
        195

<210>  6
<211>  192
<212>  PRT
<213>  Homo sapiens

<400>  6

Ser Leu Leu Asn Gln His Ala Cys Ala Val Met Lys Asn Phe Gly Thr
1               5                   10                  15

Arg Thr Phe Gln Ala Ile Thr Val Thr Lys Leu Ser Gln Lys Phe Thr
            20                  25                  30

Lys Val Asn Phe Thr Glu Ile Gln Lys Leu Val Leu Asp Val Ala His
        35                  40                  45

Val His Glu His Cys Cys Arg Gly Asp Val Leu Asp Cys Leu Gln Asp
    50                  55                  60

Gly Glu Lys Ile Met Ser Tyr Ile Cys Ser Gln Gln Asp Thr Leu Ser
65                  70                  75                  80

Asn Lys Ile Thr Glu Cys Cys Lys Leu Thr Thr Leu Glu Arg Gly Gln
                85                  90                  95

Cys Ile Ile His Ala Glu Asn Asp Glu Lys Pro Glu Gly Leu Ser Pro
            100                 105                 110

Asn Leu Asn Arg Phe Leu Gly Asp Arg Asp Phe Asn Gln Phe Ser Ser
        115                 120                 125

Gly Glu Lys Asn Ile Phe Leu Ala Ser Phe Val His Glu Tyr Ser Arg
    130                 135                 140

Arg His Pro Gln Leu Ala Val Ser Val Ile Leu Arg Val Ala Lys Gly
145                 150                 155                 160

Tyr Gln Glu Leu Leu Glu Lys Cys Phe Gln Thr Glu Asn Pro Leu Glu
                165                 170                 175

Cys Gln Asp Lys Gly Glu Glu Glu Leu Gln Lys Tyr Ile Gln Glu Ser

180                 185                 190

<210>  7
<211>  201
<212>  PRT
<213>  Homo sapiens

<400>  7

Gln Ala Leu Ala Lys Arg Ser Cys Gly Leu Phe Gln Lys Leu Gly Glu
1               5                   10                  15

Tyr Tyr Leu Gln Asn Ala Phe Leu Val Ala Tyr Thr Lys Lys Ala Pro
            20                  25                  30

Gln Leu Thr Ser Ser Glu Leu Met Ala Ile Thr Arg Lys Met Ala Ala
        35                  40                  45

Thr Ala Ala Thr Cys Cys Gln Leu Ser Glu Asp Lys Leu Leu Ala Cys
    50                  55                  60

Gly Glu Gly Ala Ala Asp Ile Ile Ile Gly His Leu Cys Ile Arg His
65                  70                  75                  80

Glu Met Thr Pro Val Asn Pro Gly Val Gly Gln Cys Cys Thr Ser Ser
                85                  90                  95

Tyr Ala Asn Arg Arg Pro Cys Phe Ser Ser Leu Val Val Asp Glu Thr
            100                 105                 110

Tyr Val Pro Pro Ala Phe Ser Asp Asp Lys Phe Ile Phe His Lys Asp
        115                 120                 125

Leu Cys Gln Ala Gln Gly Val Ala Leu Gln Thr Met Lys Gln Glu Phe
    130                 135                 140

Leu Ile Asn Leu Val Lys Gln Lys Pro Gln Ile Thr Glu Glu Gln Leu
145                 150                 155                 160

Glu Ala Val Ile Ala Asp Phe Ser Gly Leu Leu Glu Lys Cys Cys Gln
                165                 170                 175

Gly Gln Glu Gln Glu Val Cys Phe Ala Glu Glu Gly Gln Lys Leu Ile
            180                 185                 190

Ser Lys Thr Arg Ala Ala Leu Gly Val
        195                 200

<210>  8
<211>  389
<212>  PRT

<213>  Homo sapiens

<400>  8

Thr Leu His Arg Asn Glu Tyr Gly Ile Ala Ser Ile Leu Asp Ser Tyr
1               5                   10                  15

Gln Cys Thr Ala Glu Ile Ser Leu Ala Asp Leu Ala Thr Ile Phe Phe
            20                  25                  30

Ala Gln Phe Val Gln Glu Ala Thr Tyr Lys Glu Val Ser Lys Met Val
        35                  40                  45

Lys Asp Ala Leu Thr Ala Ile Glu Lys Pro Thr Gly Asp Glu Gln Ser
    50                  55                  60

Ser Gly Cys Leu Glu Asn Gln Leu Pro Ala Phe Leu Glu Glu Leu Cys
65                  70                  75                  80

His Glu Lys Glu Ile Leu Glu Lys Tyr Gly His Ser Asp Cys Cys Ser
                85                  90                  95

Gln Ser Glu Glu Gly Arg His Asn Cys Phe Leu Ala His Lys Lys Pro
            100                 105                 110

Thr Pro Ala Ser Ile Pro Leu Phe Gln Val Pro Glu Pro Val Thr Ser
        115                 120                 125

Cys Glu Ala Tyr Glu Glu Asp Arg Glu Thr Phe Met Asn Lys Phe Ile
    130                 135                 140

Tyr Glu Ile Ala Arg Arg His Pro Phe Leu Tyr Ala Pro Thr Ile Leu
145                 150                 155                 160

Leu Trp Ala Ala Arg Tyr Asp Lys Ile Ile Pro Ser Cys Cys Lys Ala
                165                 170                 175

Glu Asn Ala Val Glu Cys Phe Gln Thr Lys Ala Ala Thr Val Thr Lys
            180                 185                 190

Glu Leu Arg Glu Ser Ser Leu Leu Asn Gln His Ala Cys Ala Val Met
        195                 200                 205

Lys Asn Phe Gly Thr Arg Thr Phe Gln Ala Ile Thr Val Thr Lys Leu
    210                 215                 220

Ser Gln Lys Phe Thr Lys Val Asn Phe Thr Glu Ile Gln Lys Leu Val
225                 230                 235                 240

Leu Asp Val Ala His Val His Glu His Cys Cys Arg Gly Asp Val Leu

245                 250                 255

Asp Cys Leu Gln Asp Gly Glu Lys Ile Met Ser Tyr Ile Cys Ser Gln
            260                 265                 270

Gln Asp Thr Leu Ser Asn Lys Ile Thr Glu Cys Cys Lys Leu Thr Thr
        275                 280                 285

Leu Glu Arg Gly Gln Cys Ile Ile His Ala Glu Asn Asp Glu Lys Pro
    290                 295                 300

Glu Gly Leu Ser Pro Asn Leu Asn Arg Phe Leu Gly Asp Arg Asp Phe
305                 310                 315                 320

Asn Gln Phe Ser Ser Gly Glu Lys Asn Ile Phe Leu Ala Ser Phe Val
                325                 330                 335

His Glu Tyr Ser Arg Arg His Pro Gln Leu Ala Val Ser Val Ile Leu
            340                 345                 350

Arg Val Ala Lys Gly Tyr Gln Glu Leu Leu Glu Lys Cys Phe Gln Thr
        355                 360                 365

Glu Asn Pro Leu Glu Cys Gln Asp Lys Gly Glu Glu Glu Leu Gln Lys
    370                 375                 380

Tyr Ile Gln Glu Ser
385

<210>  9
<211>  393
<212>  PRT
<213>  Homo sapiens

<400>  9

Ser Leu Leu Asn Gln His Ala Cys Ala Val Met Lys Asn Phe Gly Thr
1               5                   10                  15

Arg Thr Phe Gln Ala Ile Thr Val Thr Lys Leu Ser Gln Lys Phe Thr
            20                  25                  30

Lys Val Asn Phe Thr Glu Ile Gln Lys Leu Val Leu Asp Val Ala His
        35                  40                  45

Val His Glu His Cys Cys Arg Gly Asp Val Leu Asp Cys Leu Gln Asp
    50                  55                  60

Gly Glu Lys Ile Met Ser Tyr Ile Cys Ser Gln Gln Asp Thr Leu Ser
65                  70                  75                  80

Asn Lys Ile Thr Glu Cys Cys Lys Leu Thr Thr Leu Glu Arg Gly Gln
                85                  90                  95

Cys Ile Ile His Ala Glu Asn Asp Glu Lys Pro Glu Gly Leu Ser Pro
            100                 105                 110

Asn Leu Asn Arg Phe Leu Gly Asp Arg Asp Phe Asn Gln Phe Ser Ser
        115                 120                 125

Gly Glu Lys Asn Ile Phe Leu Ala Ser Phe Val His Glu Tyr Ser Arg
    130                 135                 140

Arg His Pro Gln Leu Ala Val Ser Val Ile Leu Arg Val Ala Lys Gly
145                 150                 155                 160

Tyr Gln Glu Leu Leu Glu Lys Cys Phe Gln Thr Glu Asn Pro Leu Glu
                165                 170                 175

Cys Gln Asp Lys Gly Glu Glu Glu Leu Gln Lys Tyr Ile Gln Glu Ser
            180                 185                 190

Gln Ala Leu Ala Lys Arg Ser Cys Gly Leu Phe Gln Lys Leu Gly Glu
        195                 200                 205

Tyr Tyr Leu Gln Asn Ala Phe Leu Val Ala Tyr Thr Lys Lys Ala Pro
    210                 215                 220

Gln Leu Thr Ser Ser Glu Leu Met Ala Ile Thr Arg Lys Met Ala Ala
225                 230                 235                 240

Thr Ala Ala Thr Cys Cys Gln Leu Ser Glu Asp Lys Leu Leu Ala Cys
                245                 250                 255

Gly Glu Gly Ala Ala Asp Ile Ile Ile Gly His Leu Cys Ile Arg His
            260                 265                 270

Glu Met Thr Pro Val Asn Pro Gly Val Gly Gln Cys Cys Thr Ser Ser
        275                 280                 285

Tyr Ala Asn Arg Arg Pro Cys Phe Ser Ser Leu Val Val Asp Glu Thr
    290                 295                 300

Tyr Val Pro Pro Ala Phe Ser Asp Asp Lys Phe Ile Phe His Lys Asp
305                 310                 315                 320

Leu Cys Gln Ala Gln Gly Val Ala Leu Gln Thr Met Lys Gln Glu Phe
                325                 330                 335

Leu Ile Asn Leu Val Lys Gln Lys Pro Gln Ile Thr Glu Glu Gln Leu
            340                 345                 350

Glu Ala Val Ile Ala Asp Phe Ser Gly Leu Leu Glu Lys Cys Cys Gln
        355                 360                 365

Gly Gln Glu Gln Glu Val Cys Phe Ala Glu Glu Gly Gln Lys Leu Ile
    370                 375                 380

Ser Lys Thr Arg Ala Ala Leu Gly Val
385                 390

<210>  10
<211>  331
<212>  PRT
<213>  Homo sapiens

<400>  10

Gln Asp Gly Glu Lys Ile Met Ser Tyr Ile Cys Ser Gln Gln Asp Thr
1               5                   10                  15

Leu Ser Asn Lys Ile Thr Glu Cys Cys Lys Leu Thr Thr Leu Glu Arg
            20                  25                  30

Gly Gln Cys Ile Ile His Ala Glu Asn Asp Glu Lys Pro Glu Gly Leu
        35                  40                  45

Ser Pro Asn Leu Asn Arg Phe Leu Gly Asp Arg Asp Phe Asn Gln Phe
    50                  55                  60

Ser Ser Gly Glu Lys Asn Ile Phe Leu Ala Ser Phe Val His Glu Tyr
65                  70                  75                  80

Ser Arg Arg His Pro Gln Leu Ala Val Ser Val Ile Leu Arg Val Ala
                85                  90                  95

Lys Gly Tyr Gln Glu Leu Leu Glu Lys Cys Phe Gln Thr Glu Asn Pro
            100                 105                 110

Leu Glu Cys Gln Asp Lys Gly Glu Glu Glu Leu Gln Lys Tyr Ile Gln
        115                 120                 125

Glu Ser Gln Ala Leu Ala Lys Arg Ser Cys Gly Leu Phe Gln Lys Leu
    130                 135                 140

Gly Glu Tyr Tyr Leu Gln Asn Ala Phe Leu Val Ala Tyr Thr Lys Lys
145                 150                 155                 160

Ala Pro Gln Leu Thr Ser Ser Glu Leu Met Ala Ile Thr Arg Lys Met
                165                 170                 175

Ala Ala Thr Ala Ala Thr Cys Cys Gln Leu Ser Glu Asp Lys Leu Leu
            180                 185                 190

Ala Cys Gly Glu Gly Ala Ala Asp Ile Ile Ile Gly His Leu Cys Ile
        195                 200                 205

Arg His Glu Met Thr Pro Val Asn Pro Gly Val Gly Gln Cys Cys Thr
    210                 215                 220

Ser Ser Tyr Ala Asn Arg Arg Pro Cys Phe Ser Ser Leu Val Val Asp
225                 230                 235                 240

Glu Thr Tyr Val Pro Pro Ala Phe Ser Asp Asp Lys Phe Ile Phe His
                245                 250                 255

Lys Asp Leu Cys Gln Ala Gln Gly Val Ala Leu Gln Thr Met Lys Gln
            260                 265                 270

Glu Phe Leu Ile Asn Leu Val Lys Gln Lys Pro Gln Ile Thr Glu Glu
        275                 280                 285

Gln Leu Glu Ala Val Ile Ala Asp Phe Ser Gly Leu Leu Glu Lys Cys
    290                 295                 300

Cys Gln Gly Gln Glu Gln Glu Val Cys Phe Ala Glu Glu Gly Gln Lys
305                 310                 315                 320

Leu Ile Ser Lys Thr Arg Ala Ala Leu Gly Val
                325                 330

<210> 11
<211> 1032
<212> PRT
<213> Homo sapiens

<400> 11

<210>  12
<211>  6082
<212>  DNA
<213>  Homo sapiens

<400>  12
ataacgtctt tgtcactaaa atgttcccca ggggccttcg gcgagtcttt ttgtttggtt      60

ttttgttttt aatctgtggc tcttgataat ttatctagtg gttgcctaca cctgaaaaac     120

aagacacagt gtttaactat caacgaaaga actggacggc tccccgccgc agtcccactc     180

cccgagtttg tggctggcat ttgggccacg ccgggctggg cggtcacagc gaggggcgcg     240

cagtttgggg tcacacagct ccgcttctag gccccaacca ccgttaaaag gggaagcccg     300

tgccccatca ggtccgctct tgctgagccc agagccatcc cgcgctctgc gggctgggag 360

gcccgggcca ggacgcgagt cctgcgcagc cgaggttccc cagcgccccc tgcagccgcg 420

cgtaggcaga gacggagccc ggccctgcgc ctccgcacca cgcccgggac cccacccagc 480

ggcccgtacc cggagaagca gcgcgagcac ccgaagctcc cggctggcgg cagaaaccgg 540

gagtggggcc gggcgagtgc gcggcatccc aggccggccc gaacgctccg cccgcggtgg 600

gccgacttcc cctcctcttc cctctctcct tcctttagcc cgctggcgcc ggacacgctg 660

cgcctcatct cttggggcgt tcttccccgt tggccaaccg tcgcatcccg tgcaactttg 720

gggtagtggc cgtttagtgt tgaatgttcc ccaccgagag cgcatggctt gggaagcgag 780

gcgcgaaccc ggcccccgaa gggccgccgt ccgggagacg gtgatgctgt tgctgtgcct 840

gggggtcccg accggccgcc cctacaacgt ggacactgag agcgcgctgc tttaccaggg 900

cccccacaac acgctgttcg gctactcggt cgtgctgcac agccacgggg cgaaccgatg 960

gctcctagtg ggtgcgccca ctgccaactg gctcgccaac gcttcagtga tcaatcccgg 1020

ggcgatttac agatgcagga tcggaaagaa tcccggccag acgtgcgaac agctccagct 1080

gggtagccct aatggagaac cttgtggaaa gacttgtttg gaagagagag acaatcagtg 1140

gttgggggtc acactttcca gacagccagg agaaaatgga tccatcgtga cttgtgggca 1200

tagatggaaa aatatatttt acataaagaa tgaaaataag ctccccactg gtggttgcta 1260

tggagtgccc cctgatttac gaacagaact gagtaaaaga atagctccgt gttatcaaga 1320

ttatgtgaaa aaatttggag aaaattttgc atcatgtcaa gctggaatat ccagttttta 1380

cacaaaggat ttaattgtga tgggggcccc aggatcatct tactggactg gctctctttt 1440

tgtctacaat ataactacaa ataaatacaa ggctttttta gacaaacaaa atcaagtaaa 1500

atttggaagt tatttaggat attcagtcgg agctggtcat tttcggagcc agcatactac 1560

cgaagtagtc ggaggagctc ctcaacatga gcagattggt aaggcatata tattcagcat 1620

tgatgaaaaa gaactaaata tcttacatga aatgaaaggt aaaaagcttg gatcgtactt 1680

tggagcttct gtctgtgctg tggacctcaa tgcagatggc ttctcagatc tgctcgtggg 1740

agcacccatg cagagcacca tcagagagga aggaagagtg tttgtgtaca tcaactctgg 1800

ctcgggagca gtaatgaatg caatggaaac aaacctcgtt ggaagtgaca aatatgctgc 1860

aagatttggg gaatctatag ttaatcttgg cgacattgac aatgatggct ttgaagatgt 1920

tgctatcgga gctccacaag aagatgactt gcaaggtgct atttatattt acaatggccg 1980

tgcagatggg atctcgtcaa ccttctcaca gagaattgaa ggacttcaga tcagcaaatc 2040

gttaagtatg tttggacagt ctatatcagg acaaattgat gcagataata atggctatgt 2100

agatgtagca gttggtgctt ttcggtctga ttctgctgtc ttgctaagga caagacctgt 2160

agtaattgtt gacgcttctt taagccaccc tgagtcagta aatagaacga aatttgactg 2220

tgttgaaaat ggatggcctt ctgtgtgcat agatctaaca ctttgtttct catataaggg 2280

caaggaagtt ccaggttaca ttgttttgtt ttataacatg agtttggatg tgaacagaaa 2340

ggcagagtct ccaccaagat tctatttctc ttctaatgga acttctgacg tgattacagg 2400

aagcatacag gtgtccagca gagaagctaa ctgtagaaca catcaagcat ttatgcggaa 2460

agatgtgcgg gacatcctca ccccaattca gattgaagct gcttaccacc ttggtcctca 2520

tgtcatcagt aaacgaagta cagaggaatt cccaccactt cagccaattc ttcagcagaa 2580

gaaagaaaaa gacataatga aaaaaacaat aaactttgca aggttttgtg cccatgaaaa 2640

ttgttctgct gatttacagg tttctgcaaa gattgggttt ttgaagcccc atgaaaataa 2700

aacatatctt gctgttggga gtatgaagac attgatgttg aatgtgtcct tgtttaatgc 2760

tggagatgat gcatatgaaa cgactctaca tgtcaaacta cccgtgggtc tttatttcat 2820

taagatttta gagctggaag agaagcaaat aaactgtgaa gtcacagata actctggcgt 2880

ggtacaactt gactgcagta ttggctatat atatgtagat catctctcaa ggatagatat 2940

tagctttctc ctggatgtga gctcactcag cagagcggaa gaggacctca gtatcacagt 3000

gcatgctacc tgtgaaaatg aagaggaaat ggacaatcta aagcacagca gagtgactgt 3060

agcaatacct ttaaaatatg aggttaagct gactgttcat gggtttgtaa acccaacttc 3120

atttgtgtat ggatcaaatg atgaaaatga gcctgaaacg tgcatggtgg agaaaatgaa 3180

cttaactttc catgttatca acactggcaa tagtatggct cccaatgtta gtgtggaaat 3240

aatggtacca aattctttta gcccccaaac tgataagctg ttcaacattt tggatgtcca 3300

gactactact ggagaatgcc actttgaaaa ttatcaaaga gtgtgtgcat tagagcagca 3360

aaagagtgca atgcagacct tgaaaggcat agtccggttc ttgtccaaga ctgataagag 3420

gctattgtac tgcataaaag ctgatccaca ttgtttaaat ttcttgtgta attttgggaa 3480

aatggaaagt ggaaaagaag ccagtgttca tatccaactg gaaggccggc catccatttt 3540

agaaatggat gagacttcag cactcaagtt tgaaataaga gcaacaggtt ttccagagcc 3600

aaatccaaga gtaattgaac taaacaagga tgagaatgtt gcgcatgttc tactggaagg 3660

actacatcat caaagaccca aacgttattt caccatagtg attatttcaa gtagcttgct 3720

acttggactt attgtacttc tgttgatctc atatgttatg tggaaggctg gcttctttaa 3780

aagacaatac aaatctatcc tacaagaaga aaacagaaga gacagttgga gttatatcaa 3840

cagtaaaagc aatgatgatt aaggacttct ttcaaattga gagaatggaa aacagactca 3900

ggttgtagta aagaaattta aaagacactg tttacaagaa aaaatgaatt ttgtttggac 3960

ttcttttact catgatcttg tgacatatta tgtcttcatg caaggggaaa atctcagcaa 4020

tgattactct ttgagataga agaactgcaa aggtaataat acagccaaag ataatctctc 4080

agcttttaaa tgggtagaga aacactaaag cattcaattt attcaagaaa agtaagccct 4140

tgaagatatc ttgaaatgaa agtataactg agttaaatta tactggagaa gtcttagact 4200

tgaaatacta cttaccatat gtgcttgcct cagtaaaatg aaccccactg ggtgggcaga 4260

ggttcatttc aaatacatct ttgatacttg ttcaaaatat gttctttaaa aatataattt 4320

tttagagagc tgttcccaaa ttttctaacg agtggaccat tatcacttta aagcccttta 4380

tttataatac atttcctacg ggctgtgttc caacaaccat tttttttcag cagactatga 4440

atattatagt attataggcc aaactggcaa acttcagact gaacatgtac actggtttga 4500

gcttagtgaa attacttctg gataattatt tttttataat tatggatttc accatctttc 4560

tttctgtata tatacatgtg tttttatgta ggtatatatt taccattctt cctatctatt 4620

cttcctataa cacaccttta tcaagcatac ccaggagtaa tcttcaaatc ttttgttata 4680

ttctgaaaca aaagattgtg agtgttgcac tttacctgat acacgctgat ttagaaaata 4740

cagaaaccat acctcactaa taactttaaa atcaaagctg tgcaaagact agggggccta 4800

tacttcatat gtattatgta ctatgtaaaa tattgactat cacacaacta tttccttgga 4860

tgtaattctt tgttaccctt tacaagtata agtgttacct tacatggaaa cgaagaaaca 4920

aaattcataa atttaaattc ataaatttag ctgaaagata ctgattcaat ttgtatacag 4980

tgaatataaa tgagacgaca gcaaaatttt catgaaatgt aaaatatttt tatagtttgt 5040

tcatactata tgaggttcta ttttaaatga ctttctggat tttaaaaaat ttctttaaat 5100

acaatcattt ttgtaatatt tattttatgc ttatgatcta gataattgca gaatatcatt 5160

ttatctgact ctgccttcat aagagagctg tggccgaatt ttgaacatct gttataggga 5220

gtgatcaaat tagaaggcaa tgtggaaaaa caattctggg aaagatttct ttatatgaag 5280

tccctgccac tagccagcca tcctaattga tgaaagttat ctgttcacag gcctgcagtg 5340

atggtgagga atgttctgag atttgcgaag gcatttgagt agtgaaatgt aagcacaaaa 5400

cctcctgaac ccagagtgtg tatacacagg aataaacttt atgacattta tgtattttta 5460

aaaaactttg tatcgttata aaaaggctag tcattctttc aggagaacat ctaggatcat 5520

agatgaaaaa tcaagccccg atttagaact gtcttctcca ggatggtctc taaggaaatt 5580

tacatttggt tctttcctac tcagaactac tcagaaacaa ctatatattt caggttatct 5640

gagcacagtg aaagcagagt actatggttg tccaacacag gcctctcaga tacaagggga 5700

acacaattac atattgggct agattttgcc cagttcaaaa tagtatttgt tatcaactta 5760

ctttgttact tgtatcatga attttaaaac cctaccactt taagaagaca gggatgggtt 5820

attctttttt ggcaggtagg ctatataact atgtgatttt gaaatttaac tgctctggat 5880

tagggagcag tgaatcaagg cagacttatg aaatctgtat tatatttgta acagaatata 5940

ggaaatttaa cataattgat gagctcaaat cctgaaaaat gaaagaatcc aaattatttc 6000

agaattatct aggttaaata ttgatgtatt atgatggttg caaagttttt ttgtgtgtcc 6060

aataaacaca ttgtaaaaaa aa 6082

<210>  13
<211>  288
<212>  PRT
<213>  Homo sapiens

<400>  13

Met Gly His Thr Arg Arg Gln Gly Thr Ser Pro Ser Lys Cys Pro Tyr
1               5                   10                  15

Leu Asn Phe Phe Gln Leu Leu Val Leu Ala Gly Leu Ser His Phe Cys
            20                  25                  30

Ser Gly Val Ile His Val Thr Lys Glu Val Lys Glu Val Ala Thr Leu
        35                  40                  45

Ser Cys Gly His Asn Val Ser Val Glu Glu Leu Ala Gln Thr Arg Ile
    50                  55                  60

Tyr Trp Gln Lys Glu Lys Lys Met Val Leu Thr Met Met Ser Gly Asp
65                  70                  75                  80

Met Asn Ile Trp Pro Glu Tyr Lys Asn Arg Thr Ile Phe Asp Ile Thr
                85                  90                  95

Asn Asn Leu Ser Ile Val Ile Leu Ala Leu Arg Pro Ser Asp Glu Gly
            100                 105                 110

Thr Tyr Glu Cys Val Val Leu Lys Tyr Glu Lys Asp Ala Phe Lys Arg
        115                 120                 125

Glu His Leu Ala Glu Val Thr Leu Ser Val Lys Ala Asp Phe Pro Thr
    130                 135                 140

Pro Ser Ile Ser Asp Phe Glu Ile Pro Thr Ser Asn Ile Arg Arg Ile
145                 150                 155                 160

Ile Cys Ser Thr Ser Gly Gly Phe Pro Glu Pro His Leu Ser Trp Leu
                165                 170                 175

Glu Asn Gly Glu Glu Leu Asn Ala Ile Asn Thr Thr Val Ser Gln Asp
            180                 185                 190

Pro Glu Thr Glu Leu Tyr Ala Val Ser Ser Lys Leu Asp Phe Asn Met
        195                 200                 205

Thr Thr Asn His Ser Phe Met Cys Leu Ile Lys Tyr Gly His Leu Arg
    210                 215                 220

Val Asn Gln Thr Phe Asn Trp Asn Thr Thr Lys Gln Glu His Phe Pro
225                 230                 235                 240

Asp Asn Leu Leu Pro Ser Trp Ala Ile Thr Leu Ile Ser Val Asn Gly
                245                 250                 255

Ile Phe Val Ile Cys Cys Leu Thr Tyr Cys Phe Ala Pro Arg Cys Arg
            260                 265                 270

Glu Arg Arg Arg Asn Glu Arg Leu Arg Arg Glu Ser Val Arg Pro Val
        275                 280                 285

<210>  14
<211>  2757
<212>  DNA
<213>  Homo sapiens

<400>  14
gacaagtact gagtgaactc aaaccctctg taaagtaaca gaagttagaa ggggaaatgt      60

cgcctctctg aagattaccc aaagaaaaag tgatttgtca ttgctttata gactgtaaga     120

agagaacatc tcagaagtgg agtcttaccc tgaaatcaaa ggatttaaag aaaaagtgga     180

atttttcttc agcaagctgt gaaactaaat ccacaacctt tggagaccca ggaacaccct     240

ccaatctctg tgtgttttgt aaacatcact ggagggtctt ctacgtgagc aattggattg     300

tcatcagccc tgcctgtttt gcacctggga agtgccctgg tcttacttgg gtccaaattg     360

ttggctttca cttttgaccc taagcatctg aagccatggg ccacacacgg aggcagggaa     420

catcaccatc caagtgtcca tacctcaatt tctttcagct cttggtgctg gctggtcttt     480

ctcacttctg ttcaggtgtt atccacgtga ccaaggaagt gaaagaagtg gcaacgctgt     540

cctgtggtca caatgtttct gttgaagagc tggcacaaac tcgcatctac tggcaaaagg     600

agaagaaaat ggtgctgact atgatgtctg gggacatgaa tatatggccc gagtacaaga     660

accggaccat ctttgatatc actaataacc tctccattgt gatcctggct ctgcgcccat     720

ctgacgaggg cacatacgag tgtgttgttc tgaagtatga aaaagacgct ttcaagcggg     780

aacacctggc tgaagtgacg ttatcagtca aagctgactt ccctacacct agtatatctg     840

actttgaaat tccaacttct aatattagaa ggataatttg ctcaacctct ggaggttttc     900

cagagcctca cctctcctgg ttggaaaatg gagaagaatt aaatgccatc aacacaacag     960

tttcccaaga tcctgaaact gagctctatg ctgttagcag caaactggat ttcaatatga    1020

caaccaacca cagcttcatg tgtctcatca agtatggaca tttaagagtg aatcagacct    1080

tcaactggaa tacaaccaag caagagcatt ttcctgataa cctgctccca tcctgggcca    1140

ttaccttaat ctcagtaaat ggaatttttg tgatatgctg cctgacctac tgctttgccc    1200

caagatgcag agagagaagg aggaatgaga gattgagaag ggaaagtgta cgccctgtat    1260

aacagtgtcc gcagaagcaa ggggctgaaa agatctgaag gtcccacctc catttgcaat    1320

tgacctcttc tgggaacttc ctcagatgga caagattacc ccaccttgcc ctttacgtat    1380

ctgctcttag gtgcttcttc acttcagttg ctttgcagga agtgtctaga ggaatatggt 1440

gggcacagaa gtagctctgg tgaccttgat caaggtgttt tgaaatgcag aattcttgag 1500

ttctggaagg gactttagag aataccagtg ttattaatga caaaggcact gaggcccagg 1560

gaggtgaccc gaattataaa ggccagcgcc agaacccaga tttcctaact ctggtgctct 1620

ttccctttat cagtttgact gtggcctgtt aactggtata tacatatata tgtcaggcaa 1680

agtgctgctg gaagtagaat ttgtccaata acaggtcaac ttcagagact atctgatttc 1740

ctaatgtcag agtagaagat tttatgctgc tgtttacaaa agcccaatgt aatgcatagg 1800

aagtatggca tgaacatctt taggagacta atggaaatat tattggtgtt tacccagtat 1860

tccatttttt tcattgtgtt ctctattgct gctctctcac tcccccatga ggtacagcag 1920

aaaggagaac tatccaaaac taatttcctc tgacatgtaa gacgaatgat ttaggtacgt 1980

caaagcagta gtcaaggagg aaagggatag tccaaagact taactggttc atattggact 2040

gataatctct ttaaatggct ttatgctagt ttgacctcat ttgtaaaata tttatgagaa 2100

agttctcatt taaaatgaga tcgttgttta cagtgtatgt actaagcagt aagctatctt 2160

caaatgtcta aggtagtaac tttccatagg gcctccttag atccctaaga tggctttttc 2220

tccttggtat ttctgggtct ttctgacatc agcagagaac tggaaagaca tagccaactg 2280

ctgttcatgt tactcatgac tcctttctct aaaactgcct tccacaattc actagaccag 2340

aagtggacgc aacttaagct gggataatca cattatcatc tgaaaatctg gagttgaaca 2400

gcaaaagaag acaacatttc tcaaatgcac atctcatggc agctaagcca catggctggg 2460

atttaaagcc tttagagcca gcccatggct ttagctacct cactatgctg cttcacaaac 2520

cttgctcctg tgtaaaacta tattctcagt gtagggcaga gaggtctaac accaacataa 2580

ggtactagca gtgtttcccg tattgacagg aatacttaac tcaataattc ttttcttttc 2640

catttagtaa cagttgtgat gactatgttt ctattctaag taattcctgt attctacagc 2700

agatactttg tcagcaatac taagggaaga aacaaagttg aaccgtttct ttaataa 2757

<210> 15
<211> 830
<212> PRT
<213> Homo sapiens

<400> 15

Met Ala Asn Cys Gln Ile Ala Ile Leu Tyr Gln Arg Phe Gln Arg Val
1               5                   10                  15

Val Phe Gly Ile Ser Gln Leu Leu Cys Phe Ser Ala Leu Ile Ser Glu
            20                  25                  30

Leu Thr Asn Gln Lys Glu Val Ala Ala Trp Thr Tyr His Tyr Ser Thr
        35                  40                  45

Lys Ala Tyr Ser Trp Asn Ile Ser Arg Lys Tyr Cys Gln Asn Arg Tyr
    50                  55                  60

Thr Asp Leu Val Ala Ile Gln Asn Lys Asn Glu Ile Asp Tyr Leu Asn
65                  70                  75                  80

Lys Val Leu Pro Tyr Tyr Ser Ser Tyr Tyr Trp Ile Gly Ile Arg Lys
                85                  90                  95

Asn Asn Lys Thr Trp Thr Trp Val Gly Thr Lys Lys Ala Leu Thr Asn
            100                 105                 110

Glu Ala Glu Asn Trp Ala Asp Asn Glu Pro Asn Asn Lys Arg Asn Asn
        115                 120                 125

Glu Asp Cys Val Glu Ile Tyr Ile Lys Ser Pro Ser Ala Pro Gly Lys
    130                 135                 140

Trp Asn Asp Glu His Cys Leu Lys Lys Lys His Ala Leu Cys Tyr Thr
145                 150                 155                 160

Ala Ser Cys Gln Asp Met Ser Cys Ser Lys Gln Gly Glu Cys Leu Glu
                165                 170                 175

Thr Ile Gly Asn Tyr Thr Cys Ser Cys Tyr Pro Gly Phe Tyr Gly Pro
            180                 185                 190

Glu Cys Glu Tyr Val Arg Glu Cys Gly Glu Leu Glu Leu Pro Gln His
        195                 200                 205

Val Leu Met Asn Cys Ser His Pro Leu Gly Asn Phe Ser Phe Asn Ser
    210                 215                 220

Gln Cys Ser Phe His Cys Thr Asp Gly Tyr Gln Val Asn Gly Pro Ser
225                 230                 235                 240

Lys Leu Glu Cys Leu Ala Ser Gly Ile Trp Thr Asn Lys Pro Pro Gln
                245                 250                 255

Cys Leu Ala Ala Gln Cys Pro Pro Leu Lys Ile Pro Glu Arg Gly Asn
            260                 265                 270

Met Ile Cys Leu His Ser Ala Lys Ala Phe Gln His Gln Ser Ser Cys
        275                 280                 285

Ser Phe Ser Cys Glu Glu Gly Phe Ala Leu Val Gly Pro Glu Val Val
    290                 295                 300

Gln Cys Thr Ala Ser Gly Val Trp Thr Ala Pro Ala Pro Val Cys Lys
305                 310                 315                 320

Ala Val Gln Cys Gln His Leu Glu Ala Pro Ser Glu Gly Thr Met Asp
                325                 330                 335

Cys Val His Pro Leu Thr Ala Phe Ala Tyr Gly Ser Ser Cys Lys Phe
            340                 345                 350

Glu Cys Gln Pro Gly Tyr Arg Val Arg Gly Leu Asp Met Leu Arg Cys
        355                 360                 365

Ile Asp Ser Gly His Trp Ser Ala Pro Leu Pro Thr Cys Glu Ala Ile
    370                 375                 380

Ser Cys Glu Pro Leu Glu Ser Pro Val His Gly Ser Met Asp Cys Ser
385                 390                 395                 400

Pro Ser Leu Arg Ala Phe Gln Tyr Asp Thr Asn Cys Ser Phe Arg Cys
                405                 410                 415

Ala Glu Gly Phe Met Leu Arg Gly Ala Asp Ile Val Arg Cys Asp Asn
            420                 425                 430

Leu Gly Gln Trp Thr Ala Pro Ala Pro Val Cys Gln Ala Leu Gln Cys
        435                 440                 445

Gln Asp Leu Pro Val Pro Asn Glu Ala Arg Val Asn Cys Ser His Pro
    450                 455                 460

Phe Gly Ala Phe Arg Tyr Gln Ser Val Cys Ser Phe Thr Cys Asn Glu
465                 470                 475                 480

Gly Leu Leu Leu Val Gly Ala Ser Val Leu Gln Cys Leu Ala Thr Gly
                485                 490                 495

Asn Trp Asn Ser Val Pro Pro Glu Cys Gln Ala Ile Pro Cys Thr Pro
            500                 505                 510

Leu Leu Ser Pro Gln Asn Gly Thr Met Thr Cys Val Gln Pro Leu Gly
        515                 520                 525

Ser Ser Ser Tyr Lys Ser Thr Cys Gln Phe Ile Cys Asp Glu Gly Tyr
    530                 535                 540

Ser Leu Ser Gly Pro Glu Arg Leu Asp Cys Thr Arg Ser Gly Arg Trp
545                 550                 555                 560

Thr Asp Ser Pro Pro Met Cys Glu Ala Ile Lys Cys Pro Glu Leu Phe
                565                 570                 575

Ala Pro Glu Gln Gly Ser Leu Asp Cys Ser Asp Thr Arg Gly Glu Phe
            580                 585                 590

Asn Val Gly Ser Thr Cys His Phe Ser Cys Asn Asn Gly Phe Lys Leu
        595                 600                 605

Glu Gly Pro Asn Asn Val Glu Cys Thr Thr Ser Gly Arg Trp Ser Ala
    610                 615                 620

Thr Pro Pro Thr Cys Lys Gly Ile Ala Ser Leu Pro Thr Pro Gly Leu
625                 630                 635                 640

Gln Cys Pro Ala Leu Thr Thr Pro Gly Gln Gly Thr Met Tyr Cys Arg
                645                 650                 655

His His Pro Gly Thr Phe Gly Phe Asn Thr Thr Cys Tyr Phe Gly Cys
            660                 665                 670

Asn Ala Gly Phe Thr Leu Ile Gly Asp Ser Thr Leu Ser Cys Arg Pro
        675                 680                 685

Ser Gly Gln Trp Thr Ala Val Thr Pro Ala Cys Arg Ala Val Lys Cys
    690                 695                 700

Ser Glu Leu His Val Asn Lys Pro Ile Ala Met Asn Cys Ser Asn Leu
705                 710                 715                 720

Trp Gly Asn Phe Ser Tyr Gly Ser Ile Cys Ser Phe His Cys Leu Glu
                725                 730                 735

Gly Gln Leu Leu Asn Gly Ser Ala Gln Thr Ala Cys Gln Glu Asn Gly
            740                 745                 750

His Trp Ser Thr Thr Val Pro Thr Cys Gln Ala Gly Pro Leu Thr Ile
        755                 760                 765

Gln Glu Ala Leu Thr Tyr Phe Gly Gly Ala Val Ala Ser Thr Ile Gly
    770                 775                 780

Leu Ile Met Gly Gly Thr Leu Leu Ala Leu Leu Arg Lys Arg Phe Arg
785                 790                 795                 800

Gln Lys Asp Asp Gly Lys Cys Pro Leu Asn Pro His Ser His Leu Gly
                805                 810                 815

Thr Tyr Gly Val Phe Thr Asn Ala Ala Phe Asp Pro Ser Pro

820                 825                 830

<210>  16
<211>  3199
<212>  DNA
<213>  Homo sapiens

<400>  16
gtttttctaa acagcctgac actgagggga ggcagtgaga ctgtaagcag tctgggttgg      60

gcagaaggca gaaaaccagc agagtcacag aggagatggc caactgccaa atagccatct     120

tgtaccagag attccagaga gtggtctttg gaatttccca actcctttgc ttcagtgccc     180

tgatctctga actaacaaac cagaaagaag tggcagcatg gacttatcat tacagcacaa     240

aagcatactc atggaatatt tcccgtaaat actgccagaa tcgctacaca gacttagtgg     300

ccatccagaa taaaaatgaa attgattacc tcaataaggt cctaccctac tacagctcct     360

actactggat tgggatccga aagaacaata agacatggac atgggtggga accaaaaagg     420

ctctcaccaa cgaggctgag aactgggctg ataatgaacc taacaacaaa aggaacaacg     480

aggactgcgt ggagatatac atcaagagtc cgtcagcccc tggcaagtgg aatgatgagc     540

actgcttgaa gaaaaagcac gcattgtgtt acacagcctc ctgccaggac atgtcctgca     600

gcaaacaagg agagtgcctc gagaccatcg ggaactacac ctgctcctgt taccctggat     660

tctatgggcc agaatgtgaa tacgtgagag agtgtggaga acttgagctc cctcaacacg     720

tgctcatgaa ctgcagccac cctctgggaa acttctcttt taactcgcag tgcagcttcc     780

actgcactga cgggtaccaa gtaaatgggc ccagcaagct ggaatgcttg gcttctggaa     840

tctggacaaa taagcctcca cagtgtttag ctgcccagtg cccacccctg aagattcctg     900

aacgaggaaa catgatctgc cttcattctg caaaagcatt ccagcatcag tctagctgca     960

gcttcagttg tgaagaggga tttgcattag ttggaccgga agtggtgcaa tgcacagcct    1020

cgggggtatg gacagcccca gccccagtgt gtaaagctgt gcagtgtcag cacctggaag    1080

cccccagtga aggaaccatg gactgtgttc atccgctcac tgcttttgcc tatggctcca    1140

gctgcaaatt tgagtgccag cccggctaca gagtgagggg cttggacatg ctccgctgca    1200

ttgactctgg acactggtct gcacccttgc caacctgtga ggctatttcg tgtgagccgc    1260

tggagagtcc tgtccacgga agcatggatt gctctccatc cttgagagcg tttcagtatg    1320

acaccaactg tagcttccgc tgtgctgaag gtttcatgct gagaggagcc gatatagttc    1380

ggtgtgataa cttgggacag tggacagcac cagccccagt ctgtcaagct ttgcagtgcc    1440

aggatctccc agttccaaat gaggcccggg tgaactgctc ccaccccttc ggtgccttta    1500

ggtaccagtc agtctgcagc ttcacctgca atgaaggctt gctcctggtg ggagcaagtg    1560

tgctacagtg cttggctact ggaaactgga attctgttcc tccagaatgc caagccattc    1620

cctgcacacc tttgctaagc cctcagaatg gaacaatgac ctgtgttcaa cctcttggaa    1680

gttccagtta taaatccaca tgtcaattca tctgtgacga gggatattct ttgtctggac 1740

cagaaagatt ggattgtact cgatcgggac gctggacaga ctccccacca atgtgtgaag 1800

ccatcaagtg cccagaactc tttgccccag agcagggcag cctggattgt tctgacactc 1860

gtggagaatt caatgttggc tccacctgtc atttctcttg taacaatggc tttaagctgg 1920

aggggcccaa taatgtggaa tgcacaactt ctggaagatg gtcagctact ccaccaacct 1980

gcaaaggcat agcatcactt cctactccag ggttgcaatg tccagccctc accactcctg 2040

ggcagggaac catgtactgt aggcatcatc cgggaacctt tggttttaat accacttgtt 2100

actttggctg caacgctgga ttcacactca taggagacag cactctcagc tgcagacctt 2160

caggacaatg gacagcagta actccagcat gcagagctgt gaaatgctca gaactacatg 2220

ttaataagcc aatagcgatg aactgctcca acctctgggg aaacttcagt tatggatcaa 2280

tctgctcttt ccattgtcta gagggccagt tacttaatgg ctctgcacaa acagcatgcc 2340

aagagaatgg ccactggtca actaccgtgc caacctgcca agcaggacca ttgactatcc 2400

aggaagccct gacttacttt ggtggagcgg tggcttctac aataggtctg ataatgggtg 2460

ggacgctcct ggctttgcta agaaagcgtt tcagacaaaa agatgatggg aaatgcccct 2520

tgaatcctca cagccaccta ggaacatatg gagtttttac aaacgctgca tttgacccga 2580

gtccttaagg tttccataaa cacccatgaa tcaaagacat ggaattacct tagattagct 2640

ctggaccagc ctgttggacc cgctctggac caaccctgtt tcctgagttt gggattgtgg 2700

tacaatctca aattctcaac ctaccacccc ttcctgtccc acctcttctc ttcctgtaac 2760

acaagccaca gaagccagga gcaaatgttt ctgcagtagt ctctgtgctt tgactcacct 2820

gttacttgaa ataccagtga accaaagaga ctggagcatc tgactcacaa gaagaccaga 2880

ctgtggagaa ataaaaatac ctctttattt tttgattgaa ggaaggtttt ctccactttg 2940

ttggaaagca ggtggcatct ctaattggaa gaaattcctg tagcatcttc tggagtctcc 3000

agtggttgct gttgatgagg cctcttggac ctctgctctg aggcttccag agagtcctct 3060

ggatggcacc agaggctgca gaaggccaag aatcaagcta gaaggccaca tgtcaccgtg 3120

gaccttcctg ccaccagtca ctgtccctca aatgacccaa agaccaatat tcaaatgcgt 3180

aattaaaaga attttcccc 3199

<210> 17
<211> 381
<212> PRT
<213> Homo sapiens

<400> 17

Met Gly Pro Thr Ser Val Pro Leu Val Lys Ala His Arg Ser Ser Val
1               5                   10                  15

Ser Asp Tyr Val Asn Tyr Asp Ile Ile Val Arg His Tyr Asn Tyr Thr

20                  25                  30

Gly Lys Leu Asn Ile Ser Ala Asp Lys Glu Asn Ser Ile Lys Leu Thr
        35                  40                  45

Ser Val Val Phe Ile Leu Ile Cys Cys Phe Ile Ile Leu Glu Asn Ile
    50                  55                  60

Phe Val Leu Leu Thr Ile Trp Lys Thr Lys Lys Phe His Arg Pro Met
65                  70                  75                  80

Tyr Tyr Phe Ile Gly Asn Leu Ala Leu Ser Asp Leu Leu Ala Gly Val
                85                  90                  95

Ala Tyr Thr Ala Asn Leu Leu Leu Ser Gly Ala Thr Thr Tyr Lys Leu
            100                 105                 110

Thr Pro Ala Gln Trp Phe Leu Arg Glu Gly Ser Met Phe Val Ala Leu
        115                 120                 125

Ser Ala Ser Val Phe Ser Leu Leu Ala Ile Ala Ile Glu Arg Tyr Ile
    130                 135                 140

Thr Met Leu Lys Met Lys Leu His Asn Gly Ser Asn Asn Phe Arg Leu
145                 150                 155                 160

Phe Leu Leu Ile Ser Ala Cys Trp Val Ile Ser Leu Ile Leu Gly Gly
                165                 170                 175

Leu Pro Ile Met Gly Trp Asn Cys Ile Ser Ala Leu Ser Ser Cys Ser
            180                 185                 190

Thr Val Leu Pro Leu Tyr His Lys His Tyr Ile Leu Phe Cys Thr Thr
        195                 200                 205

Val Phe Thr Leu Leu Leu Leu Ser Ile Val Ile Leu Tyr Cys Arg Ile
    210                 215                 220

Tyr Ser Leu Val Arg Thr Arg Ser Arg Arg Leu Thr Phe Arg Lys Asn
225                 230                 235                 240

Ile Ser Lys Ala Ser Arg Ser Ser Glu Asn Val Ala Leu Leu Lys Thr
                245                 250                 255

Val Ile Ile Val Leu Ser Val Phe Ile Ala Cys Trp Ala Pro Leu Phe
            260                 265                 270

Ile Leu Leu Leu Leu Asp Val Gly Cys Lys Val Lys Thr Cys Asp Ile
        275                 280                 285

Leu Phe Arg Ala Glu Tyr Phe Leu Val Leu Ala Val Leu Asn Ser Gly
    290                 295                 300

Thr Asn Pro Ile Ile Tyr Thr Leu Thr Asn Lys Glu Met Arg Arg Ala
305                 310                 315                 320

Phe Ile Arg Ile Met Ser Cys Cys Lys Cys Pro Ser Gly Asp Ser Ala
                325                 330                 335

Gly Lys Phe Lys Arg Pro Ile Ile Ala Gly Met Glu Phe Ser Arg Ser
            340                 345                 350

Lys Ser Asp Asn Ser Ser His Pro Gln Lys Asp Glu Gly Asp Asn Pro
        355                 360                 365

Glu Thr Ile Met Ser Ser Gly Asn Val Asn Ser Ser Ser
    370                 375                 380

<210>  18
<211>  2811
<212>  DNA
<213>  Homo sapiens

<400>  18
ggggagtcgg gggcagcagc aagatgcgaa gcgagccgta cagatcccgg gctctccgaa      60

cgcaacttcg ccctgcttga gcgaggctgc ggtttccgag gccctctcca gccaaggaaa     120

agctacacaa aaagcctgga tcactcatcg aaccacccct gaagccagtg aaggctctct     180

cgcctcgccc tctagcgttc gtctggagta gcgccacccc ggcttcctgg ggacacaggt     240

ttggcaccat ggggcccacc agcgtcccgc tggtcaaggc ccaccgcagc tcggtctctg     300

actacgtcaa ctatgatatc atcgtccggc attacaacta cacgggaaag ctgaatatca     360

gcgcggacaa ggagaacagc attaaactga cctcggtggt gttcattctc atctgctgct     420

ttatcatcct ggagaacatc tttgtcttgc tgaccatttg gaaaaccaag aaattccacc     480

gacccatgta ctattttatt ggcaatctgg ccctctcaga cctgttggca ggagtagcct     540

acacagctaa cctgctcttg tctggggcca ccacctacaa gctcactccc gcccagtggt     600

ttctgcggga agggagtatg tttgtggccc tgtcagcctc cgtgttcagt ctcctcgcca     660

tcgccattga gcgctatatc acaatgctga aaatgaaact ccacaacggg agcaataact     720

tccgcctctt cctgctaatc agcgcctgct gggtcatctc cctcatcctg ggtggcctgc     780

ctatcatggg ctggaactgc atcagtgcgc tgtccagctg ctccaccgtg ctgccgctct     840

accacaagca ctatatcctc ttctgcacca cggtcttcac tctgcttctg ctctccatcg     900

tcattctgta ctgcagaatc tactccttgg tcaggactcg gagccgccgc ctgacgttcc     960

gcaagaacat ttccaaggcc agccgcagct ctgagaagtc gctggcgctg ctcaagaccg    1020

taattatcgt cctgagcgtc ttcatcgcct gctgggcacc gctcttcatc ctgctcctgc 1080

tggatgtggg ctgcaaggtg aagacctgtg acatcctctt cagagcggag tacttcctgg 1140

tgttagctgt gctcaactcc ggcaccaacc ccatcattta cactctgacc aacaaggaga 1200

tgcgtcgggc cttcatccgg atcatgtcct gctgcaagtg cccgagcgga gactctgctg 1260

gcaaattcaa gcgacccatc atcgccggca tggaattcag ccgcagcaaa tcggacaatt 1320

cctcccaccc ccagaaagac gaaggggaca acccagagac cattatgtct tctggaaacg 1380

tcaactcttc ttcctagaac tggaagctgt ccacccaccg gaagcgctct ttacttggtc 1440

gctggccacc ccagtgtttg gaaaaaaatc tctgggcttc gactgctgcc agggaggagc 1500

tgctgcaagc cagagggagg aagggggaga atacgaacag cctggtggtg tcgggtgttg 1560

gtgggtagag ttagttcctg tgaacaatgc actgggaagg gtggagatca ggtcccggcc 1620

tggaatatat tttctacccc cctggagctt tgattttgca ctgagccaaa ggtctagcat 1680

tgtcaagctc ctaaagggtt catttggccc ctcctcaaag actaatgtcc ccatgtgaaa 1740

gcgtctcttt gtctggagct ttgaggagat gttttccttc actttagttt caaacccaag 1800

tgagtgtgtg cacttctgct tctttaggga tgccctgtac atcccacacc ccaccctccc 1860

ttcccttcat acccctcctc aacgttcttt tactttatac tttaactacc tgagagttat 1920

cagagctggg gttgtggaat gatcgatcat ctatagcaaa taggctatgt tgagtacgta 1980

ggctgtggga agatgaagat ggtttggagg tgtaaaacaa tgtccttcgc tgaggccaaa 2040

gtttccatgt aagcgggatc cgttttttgg aatttggttg aagtcacttt gatttcttta 2100

aaaaacatct tttcaatgaa atgtgttacc atttcatatc cattgaagcc gaaatctgca 2160

taaggaagcc cactttatct aaatgatatt agccaggatc cttggtgtcc taggagaaac 2220

agacaagcaa aacaaagtga aaaccgaatg gattaacttt tgcaaaccaa gggagatttc 2280

ttagcaaatg agtctaacaa atatgacatc tgtctttggc acttttgttg atgtttattt 2340

cagaatgttg tgtgattcat ttcaagcaac aacatggttg tattttgttg tgttaaaagt 2400

acttttcttg atttttgaat gtatttgttt cagcagaagt cattttattg gatttttcta 2460

acccgtgtta acaccattga atgtgtattt cttaagaaaa taccaccctc ttgtgccctt 2520

aaaagcatta ctttaactgg tagggaacgc cagaaacttt tcagtccagc tattcattag 2580

atagtaattg aagatatgta taaatattac aaagaataaa aatatattac tgtctcttta 2640

gtatggtttt cagtgcaatt aaaccgagag atgtcttgtt tttttaaaaa gaatagtatt 2700

taataggttt ctgacttttg tggatcattt tgcacatagc tttatcaact tttaaacatt 2760

aataaactga tttttttaag gaaaaaaaaa aaaaaaaaaa aaaaaaaaaa a 2811

<210> 19
<211> 742
<212> PRT

<213>  Homo sapiens

<400>  19

<210>  20
<211>  3091
<212>  DNA

<213>  Homo sapiens

<400>  20
gggagaccca agcttctaga gatccctcga cctcgagatc cattgtgctc taaagagcgg      60

accccagcct ctgccaggtt cggtccgcca tcctcgtccc gtcctccgcc ggcccctgcc     120

ccgcgcccag ggatcctcca gctcctttcg cccgcgccct ccgttcgctc cggacaccat     180

ggacaagttt tggtggcacg cagcctgggg actctgcctc gtgccgctga gcctggcgca     240

gatcgatttg aatataacct gccgctttgc aggtgtattc cacgtggaga aaaatggtcg     300

ctacagcatc tctcggacgg aggccgctga cctctgcaag gctttcaata gcaccttgcc     360

cacaatggcc cagatggaga aagctctgag catcggattt gagacctgca ggtatgggtt     420

catagaaggg catgtggtga ttccccggat ccaccccaac tccatctgtg cagcaaacaa     480

cacaggggtg tacatcctca catacaacac ctcccagtat gacacatatt gcttcaatgc     540

ttcagctcca cctgaagaag attgtacatc agtcacagac ctgcccaatg cctttgatgg     600

accaattacc ataactattg ttaaccgtga tggcacccgc tatgtccaga aaggagaata     660

cagaacgaat cctgaagaca tctaccccag caaccctact gatgatgacg tgagcagcgg     720

ctcctccagt gaaaggagca gcacttcagg aggttacatc ttttacacct tttctactgt     780

acaccccatc ccagacgaag acagtccctg gatcaccgac agcacagaca gaatccctgc     840

taccactttg atgagcacta gtgctacagc aactgagaca gcaaccaaga ggcaagaagc     900

ctgggattgg ttttcatggt tgtttctacc atcagagtca aagaatcatc ttcacacaac     960

aacacaaatg gctggtacgt cttcaaatac catctcagca ggctgggagc caaatgaaga    1020

aaatgaagat gaaagagaca gacacctcag tttttctgga tcaggcattg atgatgatga    1080

agattttatc tccagcacca tttcaaccac accacgggcc tttgaccaca caaaacagaa    1140

ccaggactgg acccagtgga acccaagcca ttcaaatccg gaagtgctac ttcagacaac    1200

cacaaggatg actgatgtag acagaaatgg caccactgct tatgaaggaa actggaaccc    1260

agaagcacac cctcccctca ttcaccatga gcatcatgag gaagaagaga ccccacattc    1320

tacaagcaca atccaggcaa ctcctagtag tacaacggaa gaaacagcta cccagaagga    1380

acagtggttt ggcaacagat ggcatgaggg atatcgccaa acacccagag aagactccca    1440

ttcgacaaca gggacagctg cagcctcagc tcataccagc catccaatgc aaggaaggac    1500

aacaccaagc ccagaggaca gttcctggac tgatttcttc aacccaatct cacaccccat    1560

gggacgaggt catcaagcag gaagaaggat ggatatggac tccagtcata gtacaacgct    1620

tcagcctact gcaaatccaa acacaggttt ggtggaaaat ttggacagga caggacctct    1680

ttcaatgaca acgcagcaga gtaattctca gagcttctct acatcacatg aaggcttgga    1740

agaagataaa gaccatccaa caacttctac tctgacatca agcaatagga atgatgtcac    1800

aggtggaaga agagacccaa atcattctga aggctcaact actttactgg aaggttatac    1860

ctctcattac ccacacacga aggaaagcag gaccttcatc ccagtgacct cagctaagac 1920

tgggtccttt ggagttactg cagttactgt tggagattcc aactctaatg tcaatcgttc 1980

cttatcagga gaccaagaca cattccaccc cagtgggggg tcccatacca ctcatggatc 2040

tgaatcagat ggacactcac atgggagtca agaaggtgga gcaaacacaa cctctggtcc 2100

tataaggaca ccccaaattc cagaatggct gatcatcttg gcatccctct tggccttggc 2160

tttgattctt gcagtttgca ttgcagtcaa cagtcgaaga aggtgtgggc agaagaaaaa 2220

gctagtgatc aacagtggca atggagctgt ggaggacaga aagccaagtg gactcaacgg 2280

agaggccagc aagtctcagg aaatggtgca tttggtgaac aaggagtcgt cagaaactcc 2340

agaccagttt atgacagctg atgagacaag gaacctgcag aatgtggaca tgaagattgg 2400

ggtgtaacac ctacaccatt atcttggaaa gaaacaaccg ttggaaacat aaccattaca 2460

gggagctggg acacttaaca gatgcaatgt gctactgatt gtttcattgc gaatcttttt 2520

tagcataaaa ttttctactc tttttgtttt ttgtgttttg ttctttaaag tcaggtccaa 2580

tttgtaaaaa cagcattgct ttgtaaatta gggcccaatt aataatcagc aagaatttga 2640

tcgttcagtt ccacttggag gccttcatcc tcgggtgtgc tatggatggc ttctaacaaa 2700

aactacacat atgtattcct gatcgccaac ctttccccca ccagctaagg acatttccca 2760

gggttaatag ggcctggtcc ctgggaggaa atttgaatgg gtccattttg cccttccata 2820

gcctaatccc tgggcattgc tttccactga ggttggggtg tactagttac acatcttcaa 2880

cagaccccct ctagaaattt ttcagatgct tctgggagac accaaagggt gaagctattt 2940

atctgtagta aactatttat ctgtgttttt gaaatattaa accctggatc agtcctttga 3000

tcagtataat tttttaaagt tactttgtca gaggcacaaa agggtttaaa ctgattcata 3060

ataaatatct gtacttcttc gatcttcaaa a 3091

<210> 21
<211> 1170
<212> PRT
<213> Homo sapiens

<400> 21

Met Lys Asp Ser Cys Ile Thr Val Met Ala Met Ala Leu Leu Ser Gly
1               5                   10                  15

Phe Phe Phe Phe Ala Pro Ala Ser Ser Tyr Asn Leu Asp Val Arg Gly
            20                  25                  30

Ala Arg Ser Phe Ser Pro Pro Arg Ala Gly Arg His Phe Gly Tyr Arg
        35                  40                  45

Val Leu Gln Val Gly Asn Gly Val Ile Val Gly Ala Pro Gly Glu Gly
    50                  55                  60

Asn Ser Thr Gly Ser Leu Tyr Gln Cys Gln Ser Gly Thr Gly His Cys
65                  70                  75                  80

Leu Pro Val Thr Leu Arg Gly Ser Asn Tyr Thr Ser Lys Tyr Leu Gly
                85                  90                  95

Met Thr Leu Ala Thr Asp Pro Thr Asp Gly Ser Ile Leu Ala Cys Asp
            100                 105                 110

Pro Gly Leu Ser Arg Thr Cys Asp Gln Asn Thr Tyr Leu Ser Gly Leu
        115                 120                 125

Cys Tyr Leu Phe Arg Gln Asn Leu Gln Gly Pro Met Leu Gln Gly Arg
    130                 135                 140

Pro Gly Phe Gln Glu Cys Ile Lys Gly Asn Val Asp Leu Val Phe Leu
145                 150                 155                 160

Phe Asp Gly Ser Met Ser Leu Gln Pro Asp Glu Phe Gln Lys Ile Leu
                165                 170                 175

Asp Phe Met Lys Asp Val Met Lys Lys Leu Ser Asn Thr Ser Tyr Gln
            180                 185                 190

Phe Ala Ala Val Gln Phe Ser Thr Ser Tyr Lys Thr Glu Phe Asp Phe
        195                 200                 205

Ser Asp Tyr Val Lys Arg Lys Asp Pro Asp Ala Leu Leu Lys His Val
    210                 215                 220

Lys His Met Leu Leu Leu Thr Asn Thr Phe Gly Ala Ile Asn Tyr Val
225                 230                 235                 240

Ala Thr Glu Val Phe Arg Glu Glu Leu Gly Ala Arg Pro Asp Ala Thr
                245                 250                 255

Lys Val Leu Ile Ile Ile Thr Asp Gly Glu Ala Thr Asp Ser Gly Asn
            260                 265                 270

Ile Asp Ala Ala Lys Asp Ile Ile Arg Tyr Ile Ile Gly Ile Gly Lys
        275                 280                 285

His Phe Gln Thr Lys Glu Ser Gln Glu Thr Leu His Lys Phe Ala Ser
    290                 295                 300

Lys Pro Ala Ser Glu Phe Val Lys Ile Leu Asp Thr Phe Glu Lys Leu
305                 310                 315                 320

Lys Asp Leu Phe Thr Glu Leu Gln Lys Lys Ile Tyr Val Ile Glu Gly
                325                 330                 335

Thr Ser Lys Gln Asp Leu Thr Ser Phe Asn Met Glu Leu Ser Ser Ser
            340                 345                 350

Gly Ile Ser Ala Asp Leu Ser Arg Gly His Ala Val Val Gly Ala Val
        355                 360                 365

Gly Ala Lys Asp Trp Ala Gly Gly Phe Leu Asp Leu Lys Ala Asp Leu
    370                 375                 380

Gln Asp Asp Thr Phe Ile Gly Asn Glu Pro Leu Thr Pro Glu Val Arg
385                 390                 395                 400

Ala Gly Tyr Leu Gly Tyr Thr Val Thr Trp Leu Pro Ser Arg Gln Lys
                405                 410                 415

Thr Ser Leu Leu Ala Ser Gly Ala Pro Arg Tyr Gln His Met Gly Arg
            420                 425                 430

Val Leu Leu Phe Gln Glu Pro Gln Gly Gly Gly His Trp Ser Gln Val
        435                 440                 445

Gln Thr Ile His Gly Thr Gln Ile Gly Ser Tyr Phe Gly Gly Glu Leu
    450                 455                 460

Cys Gly Val Asp Val Asp Gln Asp Gly Glu Thr Glu Leu Leu Leu Ile
465                 470                 475                 480

Gly Ala Pro Leu Phe Tyr Gly Glu Gln Arg Gly Gly Arg Val Phe Ile
                485                 490                 495

Tyr Gln Arg Arg Gln Leu Gly Phe Glu Glu Val Ser Glu Leu Gln Gly
            500                 505                 510

Asp Pro Gly Tyr Pro Leu Gly Arg Phe Gly Glu Ala Ile Thr Ala Leu
        515                 520                 525

Thr Asp Ile Asn Gly Asp Gly Leu Val Asp Val Ala Val Gly Ala Pro
    530                 535                 540

Leu Glu Glu Gln Gly Ala Val Tyr Ile Phe Asn Gly Arg His Gly Gly
545                 550                 555                 560

Leu Ser Pro Gln Pro Ser Gln Arg Ile Glu Gly Thr Gln Val Leu Ser
                565                 570                 575

Gly Ile Gln Trp Phe Gly Arg Ser Ile His Gly Val Lys Asp Leu Glu

580                 585                 590

Gly Asp Gly Leu Ala Asp Val Ala Val Gly Ala Glu Ser Gln Met Ile
        595                 600                 605

Val Leu Ser Ser Arg Pro Val Val Asp Met Val Thr Leu Met Ser Phe
    610                 615                 620

Ser Pro Ala Glu Ile Pro Val His Glu Val Glu Cys Ser Tyr Ser Thr
625                 630                 635                 640

Ser Asn Lys Met Lys Glu Gly Val Asn Ile Thr Ile Cys Phe Gln Ile
                645                 650                 655

Lys Ser Leu Ile Pro Gln Phe Gln Gly Arg Leu Val Ala Asn Leu Thr
            660                 665                 670

Tyr Thr Leu Gln Leu Asp Gly His Arg Thr Arg Arg Arg Gly Leu Phe
        675                 680                 685

Pro Gly Gly Arg His Glu Leu Arg Arg Asn Ile Ala Val Thr Thr Ser
    690                 695                 700

Met Ser Cys Thr Asp Phe Ser Phe His Phe Pro Val Cys Val Gln Asp
705                 710                 715                 720

Leu Ile Ser Pro Ile Asn Val Ser Leu Asn Phe Ser Leu Trp Glu Glu
                725                 730                 735

Glu Gly Thr Pro Arg Asp Gln Arg Ala Gln Gly Lys Asp Ile Pro Pro
            740                 745                 750

Ile Leu Arg Pro Ser Leu His Ser Glu Thr Trp Glu Ile Pro Phe Glu
        755                 760                 765

Lys Asn Cys Gly Glu Asp Lys Lys Cys Glu Ala Asn Leu Arg Val Ser
    770                 775                 780

Phe Ser Pro Ala Arg Ser Arg Ala Leu Arg Leu Thr Ala Phe Ala Ser
785                 790                 795                 800

Leu Ser Val Glu Leu Ser Leu Ser Asn Leu Glu Glu Asp Ala Tyr Trp
                805                 810                 815

Val Gln Leu Asp Leu His Phe Pro Pro Gly Leu Ser Phe Arg Lys Val
            820                 825                 830

Glu Met Leu Lys Pro His Ser Gln Ile Pro Val Ser Cys Glu Glu Leu
        835                 840                 845

Pro Glu Glu Ser Arg Leu Leu Ser Arg Ala Leu Ser Cys Asn Val Ser
    850                 855                 860

Ser Pro Ile Phe Lys Ala Gly His Ser Val Ala Leu Gln Met Met Phe
865                 870                 875                 880

Asn Thr Leu Val Asn Ser Ser Trp Gly Asp Ser Val Glu Leu His Ala
                885                 890                 895

Asn Val Thr Cys Asn Asn Glu Asp Ser Asp Leu Leu Glu Asp Asn Ser
            900                 905                 910

Ala Thr Thr Ile Ile Pro Ile Leu Tyr Pro Ile Asn Ile Leu Ile Gln
        915                 920                 925

Asp Gln Glu Asp Ser Thr Leu Tyr Val Ser Phe Thr Pro Lys Gly Pro
    930                 935                 940

Lys Ile His Gln Val Lys His Met Tyr Gln Val Arg Ile Gln Pro Ser
945                 950                 955                 960

Ile His Asp His Asn Ile Pro Thr Leu Glu Ala Val Val Gly Val Pro
                965                 970                 975

Gln Pro Pro Ser Glu Gly Pro Ile Thr His Gln Trp Ser Val Gln Met
            980                 985                 990

Glu Pro Pro Val Pro Cys His Tyr  Glu Asp Leu Glu Arg  Leu Pro Asp
        995                 1000                1005

Ala Ala  Glu Pro Cys Leu Pro  Gly Ala Leu Phe Arg  Cys Pro Val
    1010                1015                1020

Val Phe  Arg Gln Glu Ile Leu  Val Gln Val Ile Gly  Thr Leu Glu
    1025                1030                1035

Leu Val  Gly Glu Ile Glu Ala  Ser Ser Met Phe Ser  Leu Cys Ser
    1040                1045                1050

Ser Leu  Ser Ile Ser Phe Asn  Ser Ser Lys His Phe  His Leu Tyr
    1055                1060                1065

Gly Ser  Asn Ala Ser Leu Ala  Gln Val Val Met Lys  Val Asp Val
    1070                1075                1080

Val Tyr  Glu Lys Gln Met Leu  Tyr Leu Tyr Val Leu  Ser Gly Ile
    1085                1090                1095

Gly Gly Leu Leu Leu Leu Leu Leu Ile Phe Ile Val Leu Tyr Lys
    1100                1105                1110

Val Gly Phe Phe Lys Arg Asn Leu Lys Glu Lys Met Glu Ala Gly
    1115                1120                1125

Arg Gly Val Pro Asn Gly Ile Pro Ala Glu Asp Ser Glu Gln Leu
    1130                1135                1140

Ala Ser Gly Gln Glu Ala Gly Asp Pro Gly Cys Leu Lys Pro Leu
    1145                1150                1155

His Glu Lys Asp Ser Glu Ser Gly Gly Gly Lys Asp
    1160                1165                1170

<210> 22
<211> 769
<212> PRT
<213> Homo sapiens

<400> 22

Met Leu Gly Leu Arg Pro Pro Leu Leu Ala Leu Val Gly Leu Leu Ser
1               5                   10                  15

Leu Gly Cys Val Leu Ser Gln Glu Cys Thr Lys Phe Lys Val Ser Ser
            20                  25                  30

Cys Arg Glu Cys Ile Glu Ser Gly Pro Gly Cys Thr Trp Cys Gln Lys
        35                  40                  45

Leu Asn Phe Thr Gly Pro Gly Asp Pro Asp Ser Ile Arg Cys Asp Thr
    50                  55                  60

Arg Pro Gln Leu Leu Met Arg Gly Cys Ala Ala Asp Asp Ile Met Asp
65                  70                  75                  80

Pro Thr Ser Leu Ala Glu Thr Gln Glu Asp His Asn Gly Gly Gln Lys
                85                  90                  95

Gln Leu Ser Pro Gln Lys Val Thr Leu Tyr Leu Arg Pro Gly Gln Ala
            100                 105                 110

Ala Ala Phe Asn Val Thr Phe Arg Arg Ala Lys Gly Tyr Pro Ile Asp
        115                 120                 125

Leu Tyr Tyr Leu Met Asp Leu Ser Tyr Ser Met Leu Asp Asp Leu Arg
    130                 135                 140

Asn Val Lys Lys Leu Gly Gly Asp Leu Leu Arg Ala Leu Asn Glu Ile

145                 150                 155                 160

Thr Glu Ser Gly Arg Ile Gly Phe Gly Ser Phe Val Asp Lys Thr Val
                165                 170                 175

Leu Pro Phe Val Asn Thr His Pro Asp Lys Leu Arg Asn Pro Cys Pro
            180                 185                 190

Asn Lys Glu Lys Glu Cys Gln Pro Pro Phe Ala Phe Arg His Val Leu
        195                 200                 205

Lys Leu Thr Asn Asn Ser Asn Gln Phe Gln Thr Glu Val Gly Lys Gln
    210                 215                 220

Leu Ile Ser Gly Asn Leu Asp Ala Pro Glu Gly Gly Leu Asp Ala Met
225                 230                 235                 240

Met Gln Val Ala Ala Cys Pro Glu Glu Ile Gly Trp Arg Asn Val Thr
                245                 250                 255

Arg Leu Leu Val Phe Ala Thr Asp Asp Gly Phe His Phe Ala Gly Asp
            260                 265                 270

Gly Lys Leu Gly Ala Ile Leu Thr Pro Asn Asp Gly Arg Cys His Leu
        275                 280                 285

Glu Asp Asn Leu Tyr Lys Arg Ser Asn Glu Phe Asp Tyr Pro Ser Val
    290                 295                 300

Gly Gln Leu Ala His Lys Leu Ala Glu Asn Asn Ile Gln Pro Ile Phe
305                 310                 315                 320

Ala Val Thr Ser Arg Met Val Lys Thr Tyr Glu Lys Leu Thr Glu Ile
                325                 330                 335

Ile Pro Lys Ser Ala Val Gly Glu Leu Ser Glu Asp Ser Ser Asn Val
            340                 345                 350

Val Gln Leu Ile Lys Asn Ala Tyr Asn Lys Leu Ser Ser Arg Val Phe
        355                 360                 365

Leu Asp His Asn Ala Leu Pro Asp Thr Leu Lys Val Thr Tyr Asp Ser
    370                 375                 380

Phe Cys Ser Asn Gly Val Thr His Arg Asn Gln Pro Arg Gly Asp Cys
385                 390                 395                 400

Asp Gly Val Gln Ile Asn Val Pro Ile Thr Phe Gln Val Lys Val Thr
                405                 410                 415

Ala Thr Glu Cys Ile Gln Glu Gln Ser Phe Val Ile Arg Ala Leu Gly
            420                 425                 430

Phe Thr Asp Ile Val Thr Val Gln Val Leu Pro Gln Cys Glu Cys Arg
        435                 440                 445

Cys Arg Asp Gln Ser Arg Asp Arg Ser Leu Cys His Gly Lys Gly Phe
    450                 455                 460

Leu Glu Cys Gly Ile Cys Arg Cys Asp Thr Gly Tyr Ile Gly Lys Asn
465                 470                 475                 480

Cys Glu Cys Gln Thr Gln Gly Arg Ser Ser Gln Glu Leu Glu Gly Ser
                485                 490                 495

Cys Arg Lys Asp Asn Asn Ser Ile Ile Cys Ser Gly Leu Gly Asp Cys
            500                 505                 510

Val Cys Gly Gln Cys Leu Cys His Thr Ser Asp Val Pro Gly Lys Leu
        515                 520                 525

Ile Tyr Gly Gln Tyr Cys Glu Cys Asp Thr Ile Asn Cys Glu Arg Tyr
    530                 535                 540

Asn Gly Gln Val Cys Gly Gly Pro Gly Arg Gly Leu Cys Phe Cys Gly
545                 550                 555                 560

Lys Cys Arg Cys His Pro Gly Phe Glu Gly Ser Ala Cys Gln Cys Glu
                565                 570                 575

Arg Thr Thr Glu Gly Cys Leu Asn Pro Arg Arg Val Glu Cys Ser Gly
            580                 585                 590

Arg Gly Arg Cys Arg Cys Asn Val Cys Glu Cys His Ser Gly Tyr Gln
        595                 600                 605

Leu Pro Leu Cys Gln Glu Cys Pro Gly Cys Pro Ser Pro Cys Gly Lys
    610                 615                 620

Tyr Ile Ser Cys Ala Glu Cys Leu Lys Phe Glu Lys Gly Pro Phe Gly
625                 630                 635                 640

Lys Asn Cys Ser Ala Ala Cys Pro Gly Leu Gln Leu Ser Asn Asn Pro
                645                 650                 655

Val Lys Gly Arg Thr Cys Lys Glu Arg Asp Ser Glu Gly Cys Trp Val
            660                 665                 670

Ala Tyr Thr Leu Glu Gln Gln Asp Gly Met Asp Arg Tyr Leu Ile Tyr
      675                 680                 685

Val Asp Glu Ser Arg Glu Cys Val Ala Gly Pro Asn Ile Ala Ala Ile
    690                 695                 700

Val Gly Gly Thr Val Ala Gly Ile Val Leu Ile Gly Ile Leu Leu Leu
705                 710                 715                 720

Val Ile Trp Lys Ala Leu Ile His Leu Ser Asp Leu Arg Glu Tyr Arg
                725                 730                 735

Arg Phe Glu Lys Glu Lys Leu Lys Ser Gln Trp Asn Asn Asp Asn Pro
            740                 745                 750

Leu Phe Lys Ser Ala Thr Thr Thr Val Met Asn Pro Lys Phe Ala Glu
      755                 760                 765

Ser

<210>  23
<211>  5133
<212>  DNA
<213>  Homo sapiens

<400>  23
cctctttcac cctgtctagg ttgccagcaa atcccacggg cctcctgacg ctgcccctgg      60

ggccacaggt ccctcgagtg ctggaaggat gaaggattcc tgcatcactg tgatggccat     120

ggcgctgctg tctgggttct ttttcttcgc gccggcctcg agctacaacc tggacgtgcg     180

gggcgcgcgg agcttctccc caccgcgcgc cgggaggcac tttggatacc gcgtcctgca     240

ggtcggaaac ggggtcatcg tgggagctcc aggggagggg aacagcacag gaagcctcta     300

tcagtgccag tcgggcacag gacactgcct gccagtcacc ctgagaggtt ccaactatac     360

ctccaagtac ttgggaatga ccttggcaac agaccccaca gatggaagca ttttggcctg     420

tgaccctggg ctgtctcgaa cgtgtgacca gaacacctat ctgagtggcc tgtgttacct     480

cttccgccag aatctgcagg gtcccatgct gcaggggcgc cctggttttc aggaatgtat     540

caagggcaac gtagacctgg tatttctgtt tgatggttcg atgagcttgc agccagatga     600

atttcagaaa attctggact tcatgaagga tgtgatgaag aaactcagca acacttcgta     660

ccagtttgct gctgttcagt tttccacaag ctacaaaaca gaatttgatt tctcagatta     720

tgttaaatgg aaggaccctg atgctctgct gaagcatgta aagcacatgt tgctgttgac     780

caataccttt ggtgccatca attatgtcgc gacagaggtg ttccgggagg agctgggggc     840

ccggccagat gccaccaaag tgcttatcat catcacggat ggggaggcca ctgacagtgg     900

caacatcgat gcggccaaag acatcatccg ctacatcatc gggattggaa agcattttca 960
gaccaaggag agtcaggaga ccctccacaa atttgcatca aaacccgcga gcgagtttgt 1020
gaaaattctg gacacatttg agaagctgaa agatctattc actgagctgc agaagaagat 1080
ctatgtcatt gagggcacaa gcaaacagga cctgacttcc ttcaacatgg agctgtcctc 1140
cagcggcatc agtgctgacc tcagcagggg ccatgcagtc gtgggggcag taggagccaa 1200
ggactgggct gggggctttc ttgacctgaa ggcagacctg caggatgaca catttattgg 1260
gaatgaacca ttgacaccag aagtgagagc aggctatttg ggttacaccg tgacctggct 1320
gccctcccgg caaaagactt cgttgctggc ctcgggagcc cctcgatacc agcacatggg 1380
ccgagtgctg ctgttccaag agccacaggg cggaggacac tggagccagg tccagacaat 1440
ccatgggacc cagattggct cttatttcgg tggggagctg tgtggcgtcg acgtggacca 1500
agatggggag acagagctgc tgctgattgg tgccccactg ttctatgggg agcagagagg 1560
aggccgggtg tttatctacc agagaagaca gttggggttt gaagaagtct cagagctgca 1620
gggggacccc ggctacccac tcgggcggtt tggagaagcc atcactgctc tgacagacat 1680
caacggcgat gggctggtag acgtggctgt gggggcccct ctggaggagc agggggctgt 1740
gtacatcttc aatgggaggc acggggggct tagtccccag ccaagtcagc ggatagaagg 1800
gacccaagtg ctctcaggaa ttcagtggtt tggacgctcc atccatgggg tgaaggacct 1860
tgaaggggat ggcttggcag atgtggctgt gggggctgag agccagatga tcgtgctgag 1920
ctcccggccc gtggtggata tggtcaccct gatgtccttc tctccagctg agatcccagt 1980
gcatgaagtg gagtgctcct attcaaccag taacaagatg aaagaaggag ttaatatcac 2040
aatctgtttc cagatcaagt ctctctaccc ccagttccaa ggccgcctgg ttgccaatct 2100
cacttacact ctgcagctgg atggccaccg gaccagaaga cgggggttgt tcccaggagg 2160
gagacatgaa ctcagaagga atatagctgt caccaccagc atgtcatgca ctgacttctc 2220
atttcatttc ccggtatgtg ttcaagacct catctccccc atcaatgttt ccctgaattt 2280
ctctctttgg gaggaggaag ggacaccgag ggaccaaagg gcgcagggca aggacatacc 2340
gcccatcctg agaccctccc tgcactcgga aacctgggag atcccttttg agaagaactg 2400
tggggaggac aagaagtgtg aggcaaactt gagagtgtcc ttctctcctg caagatccag 2460
agccctgcgt ctaactgctt ttgccagcct ctctgtggag ctgagcctga gtaacttgga 2520
agaagatgct tactgggtcc agctggacct gcacttcccc ccgggactct ccttccgcaa 2580
ggtggagatg ctgaagcccc atagccagat acctgtgagc tgcgaggagc ttcctgaaga 2640
gtccaggctt ctgtccaggg cattatcttg caatgtgagc tctcccatct tcaaagcagg 2700
ccactcggtt gctctgcaga tgatgtttaa tacactggta aacagctcct ggggggactc 2760
ggttgaattg cacgccaatg tgacctgtaa caatgaggac tcagacctcc tggaggacaa 2820
ctcagccact accatcatcc ccatcctgta ccccatcaac atcctcatcc aggaccaaga 2880

agactccaca ctctatgtca gtttcacccc caaaggcccc aagatccacc aagtcaagca 2940

catgtaccag gtgaggatcc agccttccat ccacgaccac aacataccca ccctggaggc 3000

tgtggttggg gtgccacagc ctcccagcga ggggcccatc acacaccagt ggagcgtgca 3060

gatggagcct cccgtgccct gccactatga ggatctggag aggctcccgg atgcagctga 3120

gccttgtctc cccggagccc tgttccgctg ccctgttgtc ttcaggcagg agatcctcgt 3180

ccaagtgatc gggactctgg agctggtggg agagatcgag gcctcttcca tgttcagcct 3240

ctgcagctcc ctctccatct ccttcaacag cagcaagcat ttccacctct atggcagcaa 3300

cgcctccctg gcccaggttg tcatgaaggt tgacgtggtg tatgagaagc agatgctcta 3360

cctctacgtg ctgagcggca tcggggggct gctgctgctg ctgctcattt tcatagtgct 3420

gtacaaggtt ggtttcttca aacggaacct gaaggagaag atggaggctg gcagaggtgt 3480

cccgaatgga atccctgcag aagactctga gcagctggca tctgggcaag aggctgggga 3540

tcccggctgc ctgaagcccc tccatgagaa ggactctgag agtggtggtg gcaaggactg 3600

agtccaggcc tgtgaggtgc agagtgccca gaactggact caggatgccc agggccactc 3660

tgcctctgcc tgcattctgc cgtgtgccct cgggcgagtc actgcctctc cctggccctc 3720

agtttcccta tctcgaacat ggaactcatt cctgaatgtc tcctttgcag gctcataggg 3780

aagacctgct gagggaccag ccaagagggc tgcaaaagtg agggcttgtc attaccagac 3840

ggttcaccag cctctcttgg ttccttcctt ggaagagaat gtctgatcta aatgtggaga 3900

aactgtagtc tcaggaccta gggatgttct ggccctcacc cctgccctgg gatgtccaca 3960

gatgcctcca ccccccagaa cctgtccttg cacactcccc tgcactggag tccagtctct 4020

tctgctggca gaaagcaaat gtgacctgtg tcactacgtg actgtggcac acgccttgtt 4080

cttggccaaa gaccaaattc cttggcatgc cttccagcac cctgcaaaat gagaccctcg 4140

tggccttccc cagcctcttc tagagccgtg atgcctccct gttgaagctc tggtgacacc 4200

agcctttctc ccaggccagg ctccttcctg tcttcctgca ttcacccaga cagctccctc 4260

tgcctgaacc ttccatctcg cccacccctc cttccttgac cagcagatcc cagctcacgt 4320

cacacacttg gttgggtcct cacatctttc acacttccac caccctgcac tactccctca 4380

aagcacacgt catgtttctt catccggcag cctggatgtt ttttccctgt ttaatgattg 4440

acgtacttag cagctatctc tcagtgaact gtgagggtaa aggctatact tgtcttgttc 4500

accttgggat gacgccgcat gatatgtcag ggcgtgggac atctagtagg tgcttgacat 4560

aatttcactg aattaatgac agagccagtg ggaagataca gaaaaagagg gccggggctg 4620

ggcgcggtgg ttcacgcctg taatcccagc actttgggag gccaaggagg gtggatcacc 4680

tgaggtcagg agttagaggc cagcctggcg aaaccccatc tctactaaaa atacaaaatc 4740

caggcgtggt ggcacacacc tgtagtccca gctactcagg aggttgaggt aggagaattg 4800

cttgaacctg ggaggtggag gttgcagtga gccaagattg cgccattgca ctccagcctg 4860

ggcaacacag cgagactccg tctcaaggaa aaaataaaaa taaaaagcgg gcacgggccc 4920

ggacatcccc acccttggag gctgtcttct caggctctgc cctgccctag ctccacaccc 4980

tctcccagga cccatcacgc ctgtgcagtg gcccccacag aaagactgag ctcaaggtgg 5040

gaaccacgtc tgctaacttg gagccccagt gccaagcaca gtgcctgcat gtatttatcc 5100

aataaatgtg aaattctgtc caaaaaaaaa aaa 5133

<210> 24
<211> 2291
<212> DNA
<213> Homo sapiens

<400> 24
   ctcgccctgg tggggctgct ctccctcggg tgcgtcctct ctcaggagtg cacgaagttc 60
   aaggtcagca gctgccggga atgcatcgag tcggggcccg gctgcacctg gtgccagaag 120
   ctgaacttca cagggccggg ggatcctgac tccattcgct gcgacacccg gccacagctg 180
   ctcatgaggg gctgtgcggc tgacgacatc atggacccca caagcctcgc tgaaacccag 240
   gaagaccaca atgggggcca gaagcagctg tccccacaaa aagtgacgct ttacctgcga 300
   ccaggccagg cagcagcgtt caacgtgacc ttccggcggg ccaagggcta ccccatcgac 360
   ctgtactatc tgatggacct ctcctactcc atgcttgatg acctcaggaa tgtcaagaag 420
   ctaggtggcg acctgctccg ggccctcaac gagatcaccg agtccggccg cattggcttc 480
   gggtccttcg tggacaagac cgtgctgccg ttcgtgaaca cgcaccctga taagctgcga 540
   aacccatgcc ccaacaagga gaaagagtgc ccgcccccgt ttgccttcag gcacgtgctg 600
   aagctgacca acaactccaa ccagtttcag accgaggtcg ggaagcagct gatttccgga 660
   aacctggatg cacccgaggg tgggctggac gccatgatgc aggtcgccgc ctgcccggag 720
   gaaatcggct ggcgcaacgt cacgcggctg ctggtgtttg ccactgatga cggcttccat 780
   ttcgcgggcg acggaaagct gggcgccatc ctgaccccca acgacggccg ctgtcacctg 840
   gaggacaact tgtacaagag gagcaacgaa ttcgactacc catcggtggg ccagctggcg 900
   cacaagctgg ctgaaaacaa catccagccc atcttcgcgg tgaccagtag gatggtgaag 960
   acctacgaga aactcaccga gatcatcccc aagtcagccg tgggggagct gtctgaggac 1020
   tccagcaatg tggtccatct cattaagaat gcttacaata aactctcctc cagggtcttc 1080
   ctggatcaca acgccctccc cgacaccctg aaagtcacct acgactcctt ctgcagcaat 1140
   ggagtgacgc acaggaacca gcccagaggt gactgtgatg gcgtgcagat caatgtcccg 1200
   atcaccttcc aggtgaaggt cacggccaca gagtgcatcc aggagcagtc gtttgtcatc 1260
   cgggcgctgg gcttcacgga catagtgacc gtgcaggtcc ttccccagtg tgagtgccgg 1320
   tgccgggacc agagcagaga ccgcagcctc tgccatggca agggcttctt ggagtgcggc 1380
   atctgcaggt gtgacactgg ctacattggg aaaaactgtg agtgccagac acagggccgg 1440
   agcagccagg agctggaagg aagctgccgg aaggacaaca actccatcat ctgctcaggg 1500
   ctgggggact gtgtctgcgg gcagtgcctg tgccacacca gcgacgtccc cggcaagctg 1560
   atatacgggc agtactgcga gtgtgacacc atcaactgtg agcgctacaa cggccaggtc 1620
   tgcggcggcc cggggagggg gctctgcttc tgcgggaagt gccgctgcca cccgggcttt 1680
   gagggctcag cgtgccagtg cgagaggacc actgagggct gcctgaaccc gcggcgtgtt 1740
   gagtgtagtg gtcgtggccg gtgccgctgc aacgtatgcg agtgccattc aggctaccag 1800
   ctgcctctgt gccaggagtg ccccggctgc ccctcaccct gtggcaagta catctcctgc 1860
   gccgagtgcc tgaagttcga aaagggcccc tttgggaaga actgcagcgc ggcgtgtccg 1920
   ggcctgcagc tgtcgaacaa ccccgtgaag ggcaggacct gcaaggagag ggactcagag 1980
   ggctgctggg tggcctacac gctggagcag caggacggga tggaccgcta cctcatctat 2040
   gtggatgaga gccgagagtg tgtggcaggc cccaacatcg ccgccatcgt cgggggcacc 2100
   gtggcaggca tcgtgctgat cggcattctc ctgctggtca tctggaaggc tctgatccac 2160
   ctgagcgacc tccgggagta caggcgcttt gagaaggaga agctcaagtc ccagtggaac 2220
   aatgataatc cccttttcaa gagcgccacc acgacggtca tgaaccccaa gtttgctgag 2280
   agttaggagc a 2291

<210> 25
<211> 1170
<212> PRT
<213> Homo sapiens

<400> 25

Met Lys Asp Ser Cys Ile Thr Val Met Ala Met Ala Leu Leu Ser Gly
1               5                   10                  15

Phe Phe Phe Phe Ala Pro Ala Ser Ser Tyr Asn Leu Asp Val Arg Gly
            20                  25                  30

Ala Arg Ser Phe Ser Pro Pro Arg Ala Gly Arg His Phe Gly Tyr Arg
        35                  40                  45

Val Leu Gln Val Gly Asn Gly Val Ile Val Gly Ala Pro Gly Glu Gly
    50                  55                  60

Asn Ser Thr Gly Ser Leu Tyr Gln Cys Gln Ser Gly Thr Gly His Cys
65                  70                  75                  80

Leu Pro Val Thr Leu Arg Gly Ser Asn Tyr Thr Ser Lys Tyr Leu Gly
                85                  90                  95

Met Thr Leu Ala Thr Asp Pro Thr Asp Gly Ser Ile Leu Ala Cys Asp

            100                 105                 110

Pro Gly Leu Ser Arg Thr Cys Asp Gln Asn Thr Tyr Leu Ser Gly Leu
        115                 120                 125

Cys Tyr Leu Phe Arg Gln Asn Leu Gln Gly Pro Met Leu Gln Gly Arg
    130                 135                 140

Pro Gly Phe Gln Glu Cys Ile Lys Gly Asn Val Asp Leu Val Phe Leu
145                 150                 155                 160

Phe Asp Gly Ser Met Ser Leu Gln Pro Asp Glu Phe Gln Lys Ile Leu
                165                 170                 175

Asp Phe Met Lys Asp Val Met Lys Lys Leu Ser Asn Thr Ser Tyr Gln
            180                 185                 190

Phe Ala Ala Val Gln Phe Ser Thr Ser Tyr Lys Thr Glu Phe Asp Phe
        195                 200                 205

Ser Asp Tyr Val Lys Arg Lys Asp Pro Asp Ala Leu Leu Lys His Val
    210                 215                 220

Lys His Met Leu Leu Leu Thr Asn Thr Phe Gly Ala Ile Asn Tyr Val
225                 230                 235                 240

Ala Thr Glu Val Phe Arg Glu Glu Leu Gly Ala Arg Pro Asp Ala Thr
                245                 250                 255

Lys Val Leu Ile Ile Ile Thr Asp Gly Glu Ala Thr Asp Ser Gly Asn
            260                 265                 270

Ile Asp Ala Ala Lys Asp Ile Ile Arg Tyr Ile Ile Gly Ile Gly Lys
        275                 280                 285

His Phe Gln Thr Lys Glu Ser Gln Glu Thr Leu His Lys Phe Ala Ser
    290                 295                 300

Lys Pro Ala Ser Glu Phe Val Lys Ile Leu Asp Thr Phe Glu Lys Leu
305                 310                 315                 320

Lys Asp Leu Phe Thr Glu Leu Gln Lys Lys Ile Tyr Val Ile Glu Gly
                325                 330                 335

Thr Ser Lys Gln Asp Leu Thr Ser Phe Asn Met Glu Leu Ser Ser Ser
            340                 345                 350

Gly Ile Ser Ala Asp Leu Ser Arg Gly His Ala Val Val Gly Ala Val
        355                 360                 365

Gly Ala Lys Asp Trp Ala Gly Gly Phe Leu Asp Leu Lys Ala Asp Leu
    370                 375                 380

Gln Asp Asp Thr Phe Ile Gly Asn Glu Pro Leu Thr Pro Glu Val Arg
385                 390                 395                 400

Ala Gly Tyr Leu Gly Tyr Thr Val Thr Trp Leu Pro Ser Arg Gln Lys
                405                 410                 415

Thr Ser Leu Leu Ala Ser Gly Ala Pro Arg Tyr Gln His Met Gly Arg
            420                 425                 430

Val Leu Leu Phe Gln Glu Pro Gln Gly Gly Gly His Trp Ser Gln Val
        435                 440                 445

Gln Thr Ile His Gly Thr Gln Ile Gly Ser Tyr Phe Gly Gly Glu Leu
    450                 455                 460

Cys Gly Val Asp Val Asp Gln Asp Gly Glu Thr Glu Leu Leu Leu Ile
465                 470                 475                 480

Gly Ala Pro Leu Phe Tyr Gly Glu Gln Arg Gly Gly Arg Val Phe Ile
                485                 490                 495

Tyr Gln Arg Arg Gln Leu Gly Phe Glu Glu Val Ser Glu Leu Gln Gly
            500                 505                 510

Asp Pro Gly Tyr Pro Leu Gly Arg Phe Gly Glu Ala Ile Thr Ala Leu
        515                 520                 525

Thr Asp Ile Asn Gly Asp Gly Leu Val Asp Val Ala Val Gly Ala Pro
    530                 535                 540

Leu Glu Glu Gln Gly Ala Val Tyr Ile Phe Asn Gly Arg His Gly Gly
545                 550                 555                 560

Leu Ser Pro Gln Pro Ser Gln Arg Ile Glu Gly Thr Gln Val Leu Ser
                565                 570                 575

Gly Ile Gln Trp Phe Gly Arg Ser Ile His Gly Val Lys Asp Leu Glu
            580                 585                 590

Gly Asp Gly Leu Ala Asp Val Ala Val Gly Ala Glu Ser Gln Met Ile
        595                 600                 605

Val Leu Ser Ser Arg Pro Val Val Asp Met Val Thr Leu Met Ser Phe
    610                 615                 620

Ser Pro Ala Glu Ile Pro Val His Glu Val Glu Cys Ser Tyr Ser Thr
625                 630                 635                 640

Ser Asn Lys Met Lys Glu Gly Val Asn Ile Thr Ile Cys Phe Gln Ile
                645                 650                 655

Lys Ser Leu Ile Pro Gln Phe Gln Gly Arg Leu Val Ala Asn Leu Thr
            660                 665                 670

Tyr Thr Leu Gln Leu Asp Gly His Arg Thr Arg Arg Arg Gly Leu Phe
        675                 680                 685

Pro Gly Gly Arg His Glu Leu Arg Arg Asn Ile Ala Val Thr Thr Ser
    690                 695                 700

Met Ser Cys Thr Asp Phe Ser Phe His Phe Pro Val Cys Val Gln Asp
705                 710                 715                 720

Leu Ile Ser Pro Ile Asn Val Ser Leu Asn Phe Ser Leu Trp Glu Glu
                725                 730                 735

Glu Gly Thr Pro Arg Asp Gln Arg Ala Gln Gly Lys Asp Ile Pro Pro
            740                 745                 750

Ile Leu Arg Pro Ser Leu His Ser Glu Thr Trp Glu Ile Pro Phe Glu
        755                 760                 765

Lys Asn Cys Gly Glu Asp Lys Lys Cys Glu Ala Asn Leu Arg Val Ser
    770                 775                 780

Phe Ser Pro Ala Arg Ser Arg Ala Leu Arg Leu Thr Ala Phe Ala Ser
785                 790                 795                 800

Leu Ser Val Glu Leu Ser Leu Ser Asn Leu Glu Glu Asp Ala Tyr Trp
                805                 810                 815

Val Gln Leu Asp Leu His Phe Pro Pro Gly Leu Ser Phe Arg Lys Val
            820                 825                 830

Glu Met Leu Lys Pro His Ser Gln Ile Pro Val Ser Cys Glu Glu Leu
        835                 840                 845

Pro Glu Glu Ser Arg Leu Leu Ser Arg Ala Leu Ser Cys Asn Val Ser
    850                 855                 860

Ser Pro Ile Phe Lys Ala Gly His Ser Val Ala Leu Gln Met Met Phe
865                 870                 875                 880

Asn Thr Leu Val Asn Ser Ser Trp Gly Asp Ser Val Glu Leu His Ala
                885                 890                 895

Asn Val Thr Cys Asn Asn Glu Asp Ser Asp Leu Leu Glu Asp Asn Ser
            900                 905                 910

Ala Thr Thr Ile Ile Pro Ile Leu Tyr Pro Ile Asn Ile Leu Ile Gln
        915                 920                 925

Asp Gln Glu Asp Ser Thr Leu Tyr Val Ser Phe Thr Pro Lys Gly Pro
    930                 935                 940

Lys Ile His Gln Val Lys His Met Tyr Gln Val Arg Ile Gln Pro Ser
945                 950                 955                 960

Ile His Asp His Asn Ile Pro Thr Leu Glu Ala Val Val Gly Val Pro
                965                 970                 975

Gln Pro Pro Ser Glu Gly Pro Ile Thr His Gln Trp Ser Val Gln Met
            980                 985                 990

Glu Pro Pro Val Pro Cys His Tyr  Glu Asp Leu Glu Arg  Leu Pro Asp
        995                 1000                1005

Ala Ala  Glu Pro Cys Leu Pro  Gly Ala Leu Phe Arg  Cys Pro Val
    1010                1015                1020

Val Phe  Arg Gln Glu Ile Leu  Val Gln Val Ile Gly  Thr Leu Glu
    1025                1030                1035

Leu Val  Gly Glu Ile Glu Ala  Ser Ser Met Phe Ser  Leu Cys Ser
    1040                1045                1050

Ser Leu  Ser Ile Ser Phe Asn  Ser Ser Lys His Phe  His Leu Tyr
    1055                1060                1065

Gly Ser  Asn Ala Ser Leu Ala  Gln Val Val Met Lys  Val Asp Val
    1070                1075                1080

Val Tyr  Glu Lys Gln Met Leu  Tyr Leu Tyr Val Leu  Ser Gly Ile
    1085                1090                1095

Gly Gly  Leu Leu Leu Leu Leu  Leu Ile Phe Ile Val  Leu Tyr Lys
    1100                1105                1110

Val Gly  Phe Phe Lys Arg Asn  Leu Lys Glu Lys Met  Glu Ala Gly
    1115                1120                1125

Arg Gly  Val Pro Asn Gly Ile  Pro Ala Glu Asp Ser  Glu Gln Leu

1130                 1135                 1140

Ala Ser Gly Gln Glu Ala Gly Asp Pro Gly Cys Leu Lys Pro Leu
    1145                 1150                 1155

His Glu Lys Asp Ser Glu Ser Gly Gly Gly Lys Asp
    1160                 1165                 1170

<210>  26
<211>  1152
<212>  PRT
<213>  Homo sapiens

<400>  26

Met Ala Leu Arg Val Leu Leu Leu Thr Ala Leu Thr Leu Cys His Gly
1               5                   10                  15

Phe Asn Leu Asp Thr Glu Asn Ala Met Thr Phe Gln Glu Asn Ala Arg
            20                  25                  30

Gly Phe Gly Gln Ser Val Val Gln Leu Gln Gly Ser Arg Val Val Val
        35                  40                  45

Gly Ala Pro Gln Glu Ile Val Ala Ala Asn Gln Arg Gly Ser Leu Tyr
    50                  55                  60

Gln Cys Asp Tyr Ser Thr Gly Ser Cys Glu Pro Ile Arg Leu Gln Val
65                  70                  75                  80

Pro Val Glu Ala Val Asn Met Ser Leu Gly Leu Ser Leu Ala Ala Thr
                85                  90                  95

Thr Ser Pro Pro Gln Leu Leu Ala Cys Gly Pro Thr Val His Gln Thr
            100                 105                 110

Cys Ser Glu Asn Thr Tyr Val Lys Gly Leu Cys Phe Leu Phe Gly Ser
        115                 120                 125

Asn Leu Arg Gln Gln Pro Gln Lys Phe Pro Glu Ala Leu Arg Gly Cys
    130                 135                 140

Pro Gln Glu Asp Ser Asp Ile Ala Phe Leu Ile Asp Gly Ser Gly Ser
145                 150                 155                 160

Ile Ile Pro His Asp Phe Arg Arg Met Lys Glu Phe Val Ser Thr Val
                165                 170                 175

Met Glu Gln Leu Lys Lys Ser Lys Thr Leu Phe Ser Leu Met Gln Tyr
            180                 185                 190

Ser Glu Glu Phe Arg Ile His Phe Thr Phe Lys Glu Phe Gln Asn Asn
        195                 200                 205

Pro Asn Pro Arg Ser Leu Val Lys Pro Ile Thr Gln Leu Leu Gly Arg
    210                 215                 220

Thr His Thr Ala Thr Gly Ile Arg Lys Val Val Arg Glu Leu Phe Asn
225                 230                 235                 240

Ile Thr Asn Gly Ala Arg Lys Asn Ala Phe Lys Ile Leu Val Val Ile
                245                 250                 255

Thr Asp Gly Glu Lys Phe Gly Asp Pro Leu Gly Tyr Glu Asp Val Ile
            260                 265                 270

Pro Glu Ala Asp Arg Glu Gly Val Ile Arg Tyr Val Ile Gly Val Gly
        275                 280                 285

Asp Ala Phe Arg Ser Glu Lys Ser Arg Gln Glu Leu Asn Thr Ile Ala
    290                 295                 300

Ser Lys Pro Pro Arg Asp His Val Phe Gln Val Asn Asn Phe Glu Ala
305                 310                 315                 320

Leu Lys Thr Ile Gln Asn Gln Leu Arg Glu Lys Ile Phe Ala Ile Glu
                325                 330                 335

Gly Thr Gln Thr Gly Ser Ser Ser Ser Phe Glu His Glu Met Ser Gln
            340                 345                 350

Glu Gly Phe Ser Ala Ala Ile Thr Ser Asn Gly Pro Leu Leu Ser Thr
        355                 360                 365

Val Gly Ser Tyr Asp Trp Ala Gly Gly Val Phe Leu Tyr Thr Ser Lys
    370                 375                 380

Glu Lys Ser Thr Phe Ile Asn Met Thr Arg Val Asp Ser Asp Met Asn
385                 390                 395                 400

Asp Ala Tyr Leu Gly Tyr Ala Ala Ala Ile Ile Leu Arg Asn Arg Val
                405                 410                 415

Gln Ser Leu Val Leu Gly Ala Pro Arg Tyr Gln His Ile Gly Leu Val
            420                 425                 430

Ala Met Phe Arg Gln Asn Thr Gly Met Trp Glu Ser Asn Ala Asn Val
        435                 440                 445

Lys Gly Thr Gln Ile Gly Ala Tyr Phe Gly Ala Ser Leu Cys Ser Val
    450                 455                 460

Asp Val Asp Ser Asn Gly Ser Thr Asp Leu Val Leu Ile Gly Ala Pro
465                 470                 475                 480

His Tyr Tyr Glu Gln Thr Arg Gly Gly Gln Val Ser Val Cys Pro Leu
                485                 490                 495

Pro Arg Gly Arg Ala Arg Trp Gln Cys Asp Ala Val Leu Tyr Gly Glu
            500                 505                 510

Gln Gly Gln Pro Trp Gly Arg Phe Gly Ala Ala Leu Thr Val Leu Gly
        515                 520                 525

Asp Val Asn Gly Asp Lys Leu Thr Asp Val Ala Ile Gly Ala Pro Gly
    530                 535                 540

Glu Glu Asp Asn Arg Gly Ala Val Tyr Leu Phe His Gly Thr Ser Gly
545                 550                 555                 560

Ser Gly Ile Ser Pro Ser His Ser Gln Arg Ile Ala Gly Ser Lys Leu
                565                 570                 575

Ser Pro Arg Leu Gln Tyr Phe Gly Gln Ser Leu Ser Gly Gly Gln Asp
            580                 585                 590

Leu Thr Met Asp Gly Leu Val Asp Leu Thr Val Gly Ala Gln Gly His
        595                 600                 605

Val Leu Leu Leu Arg Ser Gln Pro Val Leu Arg Val Lys Ala Ile Met
    610                 615                 620

Glu Phe Asn Pro Arg Glu Val Ala Arg Asn Val Phe Glu Cys Asn Asp
625                 630                 635                 640

Gln Val Val Lys Gly Lys Glu Ala Gly Glu Val Arg Val Cys Leu His
                645                 650                 655

Val Gln Lys Ser Thr Arg Asp Arg Leu Arg Glu Gly Gln Ile Gln Ser
            660                 665                 670

Val Val Thr Tyr Asp Leu Ala Leu Asp Ser Gly Arg Pro His Ser Arg
        675                 680                 685

Ala Val Phe Asn Glu Thr Lys Asn Ser Thr Arg Arg Gln Thr Gln Val
    690                 695                 700

Leu Gly Leu Thr Gln Thr Cys Glu Thr Leu Lys Leu Gln Leu Pro Asn

705                 710                 715                 720

Cys Ile Glu Asp Pro Val Ser Pro Ile Val Leu Arg Leu Asn Phe Ser
                725                 730                 735

Leu Val Gly Thr Pro Leu Ser Ala Phe Gly Asn Leu Arg Pro Val Leu
            740                 745                 750

Ala Glu Asp Ala Gln Arg Leu Phe Thr Ala Leu Phe Pro Phe Glu Lys
        755                 760                 765

Asn Cys Gly Asn Asp Asn Ile Cys Gln Asp Asp Leu Ser Ile Thr Phe
    770                 775                 780

Ser Phe Met Ser Leu Asp Cys Leu Val Val Gly Gly Pro Arg Glu Phe
785                 790                 795                 800

Asn Val Thr Val Thr Val Arg Asn Asp Gly Glu Asp Ser Tyr Arg Thr
                805                 810                 815

Gln Val Thr Phe Phe Phe Pro Leu Asp Leu Ser Tyr Arg Lys Val Ser
            820                 825                 830

Thr Leu Gln Asn Gln Arg Ser Gln Arg Ser Trp Arg Leu Ala Cys Glu
        835                 840                 845

Ser Ala Ser Ser Thr Glu Val Ser Gly Ala Leu Lys Ser Thr Ser Cys
    850                 855                 860

Ser Ile Asn His Pro Ile Phe Pro Glu Asn Ser Glu Val Thr Phe Asn
865                 870                 875                 880

Ile Thr Phe Asp Val Asp Ser Lys Ala Ser Leu Gly Asn Lys Leu Leu
                885                 890                 895

Leu Lys Ala Asn Val Thr Ser Glu Asn Asn Met Pro Arg Thr Asn Lys
            900                 905                 910

Thr Glu Phe Gln Leu Glu Leu Pro Val Lys Tyr Ala Val Tyr Met Val
        915                 920                 925

Val Thr Ser His Gly Val Ser Thr Lys Tyr Leu Asn Phe Thr Ala Ser
    930                 935                 940

Glu Asn Thr Ser Arg Val Met Gln His Gln Tyr Gln Val Ser Asn Leu
945                 950                 955                 960

Gly Gln Arg Ser Leu Pro Ile Ser Leu Val Phe Leu Val Pro Val Arg
                965                 970                 975

Leu Asn Gln Thr Val Ile Trp Asp Arg Pro Gln Val Thr Phe Ser Glu
            980                 985                 990

Asn Leu Ser Ser Thr Cys His Thr  Lys Glu Arg Leu Pro  Ser His Ser
        995                 1000                 1005

Asp Phe  Leu Ala Glu Leu Arg  Lys Ala Pro Val Val  Asn Cys Ser
    1010                 1015                 1020

Ile Ala  Val Cys Gln Arg Ile  Gln Cys Asp Ile Pro  Phe Phe Gly
    1025                 1030                 1035

Ile Gln  Glu Glu Phe Asn Ala  Thr Leu Lys Gly Asn  Leu Ser Phe
    1040                 1045                 1050

Asp Trp  Tyr Ile Lys Thr Ser  His Asn His Leu Leu  Ile Val Ser
    1055                 1060                 1065

Thr Ala  Glu Ile Leu Phe Asn  Asp Ser Val Phe Thr  Leu Leu Pro
    1070                 1075                 1080

Gly Gln  Gly Ala Phe Val Arg  Ser Gln Thr Glu Thr  Lys Val Glu
    1085                 1090                 1095

Pro Phe  Glu Val Pro Asn Pro  Leu Pro Leu Ile Val  Gly Ser Ser
    1100                 1105                 1110

Val Gly  Gly Leu Leu Leu Leu  Ala Leu Ile Thr Ala  Ala Leu Tyr
    1115                 1120                 1125

Lys Leu  Gly Phe Phe Lys Arg  Gln Tyr Lys Asp Met  Met Ser Glu
    1130                 1135                 1140

Gly Gly  Pro Pro Gly Ala Glu  Pro Gln
    1145                 1150

<210>  27
<211>  1163
<212>  PRT
<213>  Homo sapiens

<400>  27

Met Thr Arg Thr Arg Ala Ala Leu Leu Leu Phe Thr Ala Leu Ala Thr
1               5                   10                  15

Ser Leu Gly Phe Asn Leu Asp Thr Glu Glu Leu Thr Ala Phe Arg Val
            20                  25                  30

Asp Ser Ala Gly Phe Gly Asp Ser Val Val Gln Tyr Ala Asn Ser Trp
        35                  40                  45

Val Val Val Gly Ala Pro Gln Lys Ile Thr Ala Ala Asn Gln Thr Gly
    50                  55                  60

Gly Leu Tyr Gln Cys Gly Tyr Ser Thr Gly Ala Cys Glu Pro Ile Gly
65                  70                  75                  80

Leu Gln Val Pro Pro Glu Ala Val Asn Met Ser Leu Gly Leu Ser Leu
                85                  90                  95

Ala Ser Thr Thr Ser Pro Ser Gln Leu Leu Ala Cys Gly Pro Thr Val
            100                 105                 110

His His Glu Cys Gly Arg Asn Met Tyr Leu Thr Gly Leu Cys Phe Leu
        115                 120                 125

Leu Gly Pro Thr Gln Leu Thr Gln Arg Leu Pro Val Ser Arg Gln Glu
    130                 135                 140

Cys Pro Arg Gln Glu Gln Asp Ile Val Phe Leu Ile Asp Gly Ser Gly
145                 150                 155                 160

Ser Ile Ser Ser Arg Asn Phe Ala Thr Met Met Asn Phe Val Arg Ala
                165                 170                 175

Val Ile Ser Gln Phe Gln Arg Pro Ser Thr Gln Phe Ser Leu Met Gln
            180                 185                 190

Phe Ser Asn Lys Phe Gln Thr His Phe Thr Phe Glu Glu Phe Arg Arg
        195                 200                 205

Thr Ser Asn Pro Leu Ser Leu Leu Ala Ser Val His Gln Leu Gln Gly
    210                 215                 220

Phe Thr Tyr Thr Ala Thr Ala Ile Gln Asn Val Val His Arg Leu Phe
225                 230                 235                 240

His Ala Ser Tyr Gly Ala Arg Arg Asp Ala Thr Lys Ile Leu Ile Val
                245                 250                 255

Ile Thr Asp Gly Lys Lys Glu Gly Asp Ser Leu Asp Tyr Lys Asp Val
            260                 265                 270

Ile Pro Met Ala Asp Ala Ala Gly Ile Ile Arg Tyr Ala Ile Gly Val
        275                 280                 285

Gly Leu Ala Phe Gln Asn Arg Asn Ser Trp Lys Glu Leu Asn Asp Ile

290                 295                 300

Ala Ser Lys Pro Ser Gln Glu His Ile Phe Lys Val Glu Asp Phe Asp
305                 310                 315                 320

Ala Leu Lys Asp Ile Gln Asn Gln Leu Lys Glu Lys Ile Phe Ala Ile
                325                 330                 335

Glu Gly Thr Glu Thr Thr Ser Ser Ser Ser Phe Glu Leu Glu Met Ala
            340                 345                 350

Gln Glu Gly Phe Ser Ala Val Phe Thr Pro Asp Gly Pro Val Leu Gly
        355                 360                 365

Ala Val Gly Ser Phe Thr Trp Ser Gly Gly Ala Phe Leu Tyr Pro Pro
    370                 375                 380

Asn Met Ser Pro Thr Phe Ile Asn Met Ser Gln Glu Asn Val Asp Met
385                 390                 395                 400

Arg Asp Ser Tyr Leu Gly Tyr Ser Thr Glu Leu Ala Leu Trp Lys Gly
                405                 410                 415

Val Gln Ser Leu Val Leu Gly Ala Pro Arg Tyr Gln His Thr Gly Lys
            420                 425                 430

Ala Val Ile Phe Thr Gln Val Ser Arg Gln Trp Arg Met Lys Ala Glu
        435                 440                 445

Val Thr Gly Thr Gln Ile Gly Ser Tyr Phe Gly Ala Ser Leu Cys Ser
    450                 455                 460

Val Asp Val Asp Thr Asp Gly Ser Thr Asp Leu Val Leu Ile Gly Ala
465                 470                 475                 480

Pro His Tyr Tyr Glu Gln Thr Arg Gly Gly Gln Val Ser Val Cys Pro
                485                 490                 495

Leu Pro Arg Gly Trp Arg Arg Trp Trp Cys Asp Ala Val Leu Tyr Gly
            500                 505                 510

Glu Gln Gly His Pro Trp Gly Arg Phe Gly Ala Ala Leu Thr Val Leu
        515                 520                 525

Gly Asp Val Asn Gly Asp Lys Leu Thr Asp Val Val Ile Gly Ala Pro
    530                 535                 540

Gly Glu Glu Glu Asn Arg Gly Ala Val Tyr Leu Phe His Gly Val Leu
545                 550                 555                 560

Gly Pro Ser Ile Ser Pro Ser His Ser Gln Arg Ile Ala Gly Ser Gln
                565                 570                 575

Leu Ser Ser Arg Leu Gln Tyr Phe Gly Gln Ala Leu Ser Gly Gly Gln
            580                 585                 590

Asp Leu Thr Gln Asp Gly Leu Val Asp Leu Ala Val Gly Ala Arg Gly
        595                 600                 605

Gln Val Leu Leu Leu Arg Thr Arg Pro Val Leu Trp Val Gly Val Ser
    610                 615                 620

Met Gln Phe Ile Pro Ala Glu Ile Pro Arg Ser Ala Phe Glu Cys Arg
625                 630                 635                 640

Glu Gln Val Val Ser Glu Gln Thr Leu Val Gln Ser Asn Ile Cys Leu
                645                 650                 655

Tyr Ile Asp Lys Arg Ser Lys Asn Leu Leu Gly Ser Arg Asp Leu Gln
            660                 665                 670

Ser Ser Val Thr Leu Asp Leu Ala Leu Asp Pro Gly Arg Leu Ser Pro
        675                 680                 685

Arg Ala Thr Phe Gln Glu Thr Lys Asn Arg Ser Leu Ser Arg Val Arg
    690                 695                 700

Val Leu Gly Leu Lys Ala His Cys Glu Asn Phe Asn Leu Leu Leu Pro
705                 710                 715                 720

Ser Cys Val Glu Asp Ser Val Thr Pro Ile Thr Leu Arg Leu Asn Phe
                725                 730                 735

Thr Leu Val Gly Lys Pro Leu Leu Ala Phe Arg Asn Leu Arg Pro Met
            740                 745                 750

Leu Ala Ala Asp Ala Gln Arg Tyr Phe Thr Ala Ser Leu Pro Phe Glu
        755                 760                 765

Lys Asn Cys Gly Ala Asp His Ile Cys Gln Asp Asn Leu Gly Ile Ser
    770                 775                 780

Phe Ser Phe Pro Gly Leu Lys Ser Leu Leu Val Gly Ser Asn Leu Glu
785                 790                 795                 800

Leu Asn Ala Glu Val Met Val Trp Asn Asp Gly Glu Asp Ser Tyr Gly
                805                 810                 815

Thr Thr Ile Thr Phe Ser His Pro Ala Gly Leu Ser Tyr Arg Tyr Val
            820                 825                 830

Ala Glu Gly Gln Lys Gln Gly Gln Leu Arg Ser Leu His Leu Thr Cys
        835                 840                 845

Asp Ser Ala Pro Val Gly Ser Gln Gly Thr Trp Ser Thr Ser Cys Arg
    850                 855                 860

Ile Asn His Leu Ile Phe Arg Gly Gly Ala Gln Ile Thr Phe Leu Ala
865                 870                 875                 880

Thr Phe Asp Val Ser Pro Lys Ala Val Leu Gly Asp Arg Leu Leu Leu
                885                 890                 895

Thr Ala Asn Val Ser Ser Glu Asn Asn Thr Pro Arg Thr Ser Lys Thr
            900                 905                 910

Thr Phe Gln Leu Glu Leu Pro Val Lys Tyr Ala Val Tyr Thr Val Val
        915                 920                 925

Ser Ser His Glu Gln Phe Thr Lys Tyr Leu Asn Phe Ser Glu Ser Glu
    930                 935                 940

Glu Lys Glu Ser His Val Ala Met His Arg Tyr Gln Val Asn Asn Leu
945                 950                 955                 960

Gly Gln Arg Asp Leu Pro Val Ser Ile Asn Phe Trp Val Pro Val Glu
                965                 970                 975

Leu Asn Gln Glu Ala Val Trp Met Asp Val Glu Val Ser His Pro Gln
            980                 985                 990

Asn Pro Ser Leu Arg Cys Ser Ser  Glu Lys Ile Ala Pro  Pro Ala Ser
        995                 1000                 1005

Asp Phe  Leu Ala His Ile Gln  Lys Asn Pro Val Leu  Asp Cys Ser
    1010                 1015                 1020

Ile Ala  Gly Cys Leu Arg Phe  Arg Cys Asp Val Pro  Ser Phe Ser
    1025                 1030                 1035

Val Gln  Glu Glu Leu Asp Phe  Thr Leu Lys Gly Asn  Leu Ser Phe
    1040                 1045                 1050

Gly Trp  Val Arg Gln Ile Leu  Gln Lys Lys Val Ser  Val Val Ser
    1055                 1060                 1065

Val Ala Glu Ile Thr Phe Asp Thr Ser Val Tyr Ser Gln Leu Pro
    1070                1075                1080

Gly Gln Glu Ala Phe Met Arg Ala Gln Thr Thr Thr Val Leu Glu
    1085                1090                1095

Lys Tyr Lys Val His Asn Pro Thr Pro Leu Ile Val Gly Ser Ser
    1100                1105                1110

Ile Gly Gly Leu Leu Leu Leu Ala Leu Ile Thr Ala Val Leu Tyr
    1115                1120                1125

Lys Val Gly Phe Phe Lys Arg Gln Tyr Lys Glu Met Met Glu Glu
    1130                1135                1140

Ala Asn Gly Gln Ile Ala Pro Glu Asn Gly Thr Gln Thr Pro Ser
    1145                1150                1155

Pro Pro Ser Glu Lys
    1160

<210> 28
<211> 769
<212> PRT
<213> Homo sapiens

<400> 28

Met Leu Gly Leu Arg Pro Pro Leu Leu Ala Leu Val Gly Leu Leu Ser
1               5                   10                  15

Leu Gly Cys Val Leu Ser Gln Glu Cys Thr Lys Phe Lys Val Ser Ser
            20                  25                  30

Cys Arg Glu Cys Ile Glu Ser Gly Pro Gly Cys Thr Trp Cys Gln Lys
        35                  40                  45

Leu Asn Phe Thr Gly Pro Gly Asp Pro Asp Ser Ile Arg Cys Asp Thr
    50                  55                  60

Arg Pro Gln Leu Leu Met Arg Gly Cys Ala Ala Asp Asp Ile Met Asp
65                  70                  75                  80

Pro Thr Ser Leu Ala Glu Thr Gln Glu Asp His Asn Gly Gly Gln Lys
                85                  90                  95

Gln Leu Ser Pro Gln Lys Val Thr Leu Tyr Leu Arg Pro Gly Gln Ala
            100                 105                 110

Ala Ala Phe Asn Val Thr Phe Arg Arg Ala Lys Gly Tyr Pro Ile Asp
        115                 120                 125

Leu Tyr Tyr Leu Met Asp Leu Ser Tyr Ser Met Leu Asp Asp Leu Arg
    130                 135                 140

Asn Val Lys Lys Leu Gly Gly Asp Leu Leu Arg Ala Leu Asn Glu Ile
145                 150                 155                 160

Thr Glu Ser Gly Arg Ile Gly Phe Gly Ser Phe Val Asp Lys Thr Val
                165                 170                 175

Leu Pro Phe Val Asn Thr His Pro Asp Lys Leu Arg Asn Pro Cys Pro
            180                 185                 190

Asn Lys Glu Lys Glu Cys Gln Pro Pro Phe Ala Phe Arg His Val Leu
        195                 200                 205

Lys Leu Thr Asn Asn Ser Asn Gln Phe Gln Thr Glu Val Gly Lys Gln
    210                 215                 220

Leu Ile Ser Gly Asn Leu Asp Ala Pro Glu Gly Gly Leu Asp Ala Met
225                 230                 235                 240

Met Gln Val Ala Ala Cys Pro Glu Glu Ile Gly Trp Arg Asn Val Thr
                245                 250                 255

Arg Leu Leu Val Phe Ala Thr Asp Asp Gly Phe His Phe Ala Gly Asp
            260                 265                 270

Gly Lys Leu Gly Ala Ile Leu Thr Pro Asn Asp Gly Arg Cys His Leu
        275                 280                 285

Glu Asp Asn Leu Tyr Lys Arg Ser Asn Glu Phe Asp Tyr Pro Ser Val
    290                 295                 300

Gly Gln Leu Ala His Lys Leu Ala Glu Asn Asn Ile Gln Pro Ile Phe
305                 310                 315                 320

Ala Val Thr Ser Arg Met Val Lys Thr Tyr Glu Lys Leu Thr Glu Ile
                325                 330                 335

Ile Pro Lys Ser Ala Val Gly Glu Leu Ser Glu Asp Ser Ser Asn Val
            340                 345                 350

Val Gln Leu Ile Lys Asn Ala Tyr Asn Lys Leu Ser Ser Arg Val Phe
        355                 360                 365

Leu Asp His Asn Ala Leu Pro Asp Thr Leu Lys Val Thr Tyr Asp Ser
    370                 375                 380

Phe Cys Ser Asn Gly Val Thr His Arg Asn Gln Pro Arg Gly Asp Cys
385                 390                 395                 400

Asp Gly Val Gln Ile Asn Val Pro Ile Thr Phe Gln Val Lys Val Thr
                405                 410                 415

Ala Thr Glu Cys Ile Gln Glu Gln Ser Phe Val Ile Arg Ala Leu Gly
            420                 425                 430

Phe Thr Asp Ile Val Thr Val Gln Val Leu Pro Gln Cys Glu Cys Arg
        435                 440                 445

Cys Arg Asp Gln Ser Arg Asp Arg Ser Leu Cys His Gly Lys Gly Phe
    450                 455                 460

Leu Glu Cys Gly Ile Cys Arg Cys Asp Thr Gly Tyr Ile Gly Lys Asn
465                 470                 475                 480

Cys Glu Cys Gln Thr Gln Gly Arg Ser Ser Gln Glu Leu Glu Gly Ser
                485                 490                 495

Cys Arg Lys Asp Asn Asn Ser Ile Ile Cys Ser Gly Leu Gly Asp Cys
            500                 505                 510

Val Cys Gly Gln Cys Leu Cys His Thr Ser Asp Val Pro Gly Lys Leu
        515                 520                 525

Ile Tyr Gly Gln Tyr Cys Glu Cys Asp Thr Ile Asn Cys Glu Arg Tyr
    530                 535                 540

Asn Gly Gln Val Cys Gly Gly Pro Gly Arg Gly Leu Cys Phe Cys Gly
545                 550                 555                 560

Lys Cys Arg Cys His Pro Gly Phe Glu Gly Ser Ala Cys Gln Cys Glu
                565                 570                 575

Arg Thr Thr Glu Gly Cys Leu Asn Pro Arg Arg Val Glu Cys Ser Gly
            580                 585                 590

Arg Gly Arg Cys Arg Cys Asn Val Cys Glu Cys His Ser Gly Tyr Gln
        595                 600                 605

Leu Pro Leu Cys Gln Glu Cys Pro Gly Cys Pro Ser Pro Cys Gly Lys
    610                 615                 620

Tyr Ile Ser Cys Ala Glu Cys Leu Lys Phe Glu Lys Gly Pro Phe Gly
625                 630                 635                 640

Lys Asn Cys Ser Ala Ala Cys Pro Gly Leu Gln Leu Ser Asn Asn Pro
                645                 650                 655

Val Lys Gly Arg Thr Cys Lys Glu Arg Asp Ser Glu Gly Cys Trp Val
            660                 665                 670

Ala Tyr Thr Leu Glu Gln Gln Asp Gly Met Asp Arg Tyr Leu Ile Tyr
        675                 680                 685

Val Asp Glu Ser Arg Glu Cys Val Ala Gly Pro Asn Ile Ala Ala Ile
    690                 695                 700

Val Gly Gly Thr Val Ala Gly Ile Val Leu Ile Gly Ile Leu Leu Leu
705                 710                 715                 720

Val Ile Trp Lys Ala Leu Ile His Leu Ser Asp Leu Arg Glu Tyr Arg
                725                 730                 735

Arg Phe Glu Lys Glu Lys Leu Lys Ser Gln Trp Asn Asn Asp Asn Pro
            740                 745                 750

Leu Phe Lys Ser Ala Thr Thr Thr Val Met Asn Pro Lys Phe Ala Glu
        755                 760                 765

Ser

<210> 29
<211> 5133
<212> DNA
<213> Homo sapiens

<400> 29
cctctttcac cctgtctagg ttgccagcaa atcccacggg cctcctgacg ctgcccctgg 60

ggccacaggt ccctcgagtg ctggaaggat gaaggattcc tgcatcactg tgatggccat 120

ggcgctgctg tctgggttct ttttcttcgc gccggcctcg agctacaacc tggacgtgcg 180

gggcgcgcgg agcttctccc caccgcgcgc cgggaggcac tttggatacc gcgtcctgca 240

ggtcggaaac ggggtcatcg tgggagctcc aggggagggg aacagcacag gaagcctcta 300

tcagtgccag tcgggcacag gacactgcct gccagtcacc ctgagaggtt ccaactatac 360

ctccaagtac ttgggaatga ccttggcaac agaccccaca gatggaagca ttttggcctg 420

tgaccctggg ctgtctcgaa cgtgtgacca gaacacctat ctgagtggcc tgtgttacct 480

cttccgccag aatctgcagg gtcccatgct gcaggggcgc cctggttttc aggaatgtat 540

caagggcaac gtagacctgg tatttctgtt tgatggttcg atgagcttgc agccagatga 600

atttcagaaa attctggact tcatgaagga tgtgatgaag aaactcagca acacttcgta 660

ccagtttgct gctgttcagt tttccacaag ctacaaaaca gaatttgatt tctcagatta 720

tgttaaatgg aaggaccctg atgctctgct gaagcatgta aagcacatgt tgctgttgac 780

caataccttt ggtgccatca attatgtcgc gacagaggtg ttccgggagg agctgggggc 840

ccggccagat gccaccaaag tgcttatcat catcacggat ggggaggcca ctgacagtgg 900

caacatcgat gcggccaaag acatcatccg ctacatcatc gggattggaa agcattttca 960

gaccaaggag agtcaggaga ccctccacaa atttgcatca aaacccgcga gcgagtttgt 1020

gaaaattctg gacacatttg agaagctgaa agatctattc actgagctgc agaagaagat 1080

ctatgtcatt gagggcacaa gcaaacagga cctgacttcc ttcaacatgg agctgtcctc 1140

cagcggcatc agtgctgacc tcagcagggg ccatgcagtc gtgggggcag taggagccaa 1200

ggactgggct gggggctttc ttgacctgaa ggcagacctg caggatgaca catttattgg 1260

gaatgaacca ttgacaccag aagtgagagc aggctatttg ggttacaccg tgacctggct 1320

gccctcccgg caaaagactt cgttgctggc ctcgggagcc cctcgatacc agcacatggg 1380

ccgagtgctg ctgttccaag agccacaggg cggaggacac tggagccagg tccagacaat 1440

ccatgggacc cagattggct cttatttcgg tggggagctg tgtggcgtcg acgtggacca 1500

agatggggag acagagctgc tgctgattgg tgccccactg ttctatgggg agcagagagg 1560

aggccgggtg tttatctacc agagaagaca gttggggttt gaagaagtct cagagctgca 1620

gggggacccc ggctacccac tcgggcggtt tggagaagcc atcactgctc tgacagacat 1680

caacggcgat gggctggtag acgtggctgt gggggcccct ctggaggagc agggggctgt 1740

gtacatcttc aatgggaggc acggggggct tagtccccag ccaagtcagc ggatagaagg 1800

gacccaagtg ctctcaggaa ttcagtggtt tggacgctcc atccatgggg tgaaggacct 1860

tgaaggggat ggcttggcag atgtggctgt gggggctgag agccagatga tcgtgctgag 1920

ctcccggccc gtggtggata tggtcaccct gatgtccttc tctccagctg agatcccagt 1980

gcatgaagtg gagtgctcct attcaaccag taacaagatg aaagaaggag ttaatatcac 2040

aatctgtttc cagatcaagt ctctctaccc ccagttccaa ggccgcctgg ttgccaatct 2100

cacttacact ctgcagctgg atggccaccg gaccagaaga cgggggttgt tcccaggagg 2160

gagacatgaa ctcagaagga atatagctgt caccaccagc atgtcatgca ctgacttctc 2220

atttcatttc ccggtatgtg ttcaagacct catctccccc atcaatgttt ccctgaattt 2280

ctctctttgg gaggaggaag ggacaccgag ggaccaaagg gcgcagggca aggacatacc 2340

gcccatcctg agaccctccc tgcactcgga aacctgggag atcccttttg agaagaactg 2400

tggggaggac aagaagtgtg aggcaaactt gagagtgtcc ttctctcctg caagatccag 2460

agccctgcgt ctaactgctt ttgccagcct ctctgtggag ctgagcctga gtaacttgga 2520

agaagatgct tactgggtcc agctggacct gcacttcccc ccgggactct ccttccgcaa 2580

ggtggagatg ctgaagcccc atagccagat acctgtgagc tgcgaggagc ttcctgaaga 2640

gtccaggctt ctgtccaggg cattatcttg caatgtgagc tctcccatct tcaaagcagg 2700

ccactcggtt gctctgcaga tgatgtttaa tacactggta aacagctcct ggggggactc 2760

ggttgaattg cacgccaatg tgacctgtaa caatgaggac tcagacctcc tggaggacaa 2820

ctcagccact accatcatcc ccatcctgta ccccatcaac atcctcatcc aggaccaaga 2880

agactccaca ctctatgtca gtttcacccc caaaggcccc aagatccacc aagtcaagca 2940

catgtaccag gtgaggatcc agccttccat ccacgaccac aacataccca ccctggaggc 3000

tgtggttggg gtgccacagc ctcccagcga ggggcccatc acacaccagt ggagcgtgca 3060

gatggagcct cccgtgccct gccactatga ggatctggag aggctcccgg atgcagctga 3120

gccttgtctc cccggagccc tgttccgctg ccctgttgtc ttcaggcagg agatcctcgt 3180

ccaagtgatc gggactctgg agctggtggg agagatcgag gcctcttcca tgttcagcct 3240

ctgcagctcc ctctccatct ccttcaacag cagcaagcat ttccacctct atggcagcaa 3300

cgcctccctg gcccaggttg tcatgaaggt tgacgtggtg tatgagaagc agatgctcta 3360

cctctacgtg ctgagcggca tcggggggct gctgctgctg ctgctcattt tcatagtgct 3420

gtacaaggtt ggtttcttca aacggaacct gaaggagaag atggaggctg gcagaggtgt 3480

cccgaatgga atccctgcag aagactctga gcagctggca tctgggcaag aggctgggga 3540

tcccggctgc ctgaagcccc tccatgagaa ggactctgag agtggtggtg gcaaggactg 3600

agtccaggcc tgtgaggtgc agagtgccca gaactggact caggatgccc agggccactc 3660

tgcctctgcc tgcattctgc cgtgtgccct cgggcgagtc actgcctctc cctggccctc 3720

agtttcccta tctcgaacat ggaactcatt cctgaatgtc tcctttgcag gctcataggg 3780

aagacctgct gagggaccag ccaagagggc tgcaaaagtg agggcttgtc attaccagac 3840

ggttcaccag cctctcttgg ttccttcctt ggaagagaat gtctgatcta aatgtggaga 3900

aactgtagtc tcaggaccta gggatgttct ggccctcacc cctgccctgg gatgtccaca 3960

gatgcctcca ccccccagaa cctgtccttg cacactcccc tgcactggag tccagtctct 4020

tctgctggca gaaagcaaat gtgacctgtg tcactacgtg actgtggcac acgccttgtt 4080

cttggccaaa gaccaaattc cttggcatgc cttccagcac cctgcaaaat gagaccctcg 4140

tggccttccc cagcctcttc tagagccgtg atgcctccct gttgaagctc tggtgacacc 4200

agcctttctc ccaggccagg ctccttcctg tcttcctgca ttcacccaga cagctccctc 4260

tgcctgaacc ttccatctcg cccacccctc cttccttgac cagcagatcc cagctcacgt 4320

cacacacttg gttgggtcct cacatctttc acacttccac caccctgcac tactccctca 4380

aagcacacgt catgtttctt catccggcag cctggatgtt ttttccctgt ttaatgattg 4440

acgtacttag cagctatctc tcagtgaact gtgagggtaa aggctatact tgtcttgttc 4500

accttgggat gacgccgcat gatatgtcag ggcgtgggac atctagtagg tgcttgacat 4560

aatttcactg aattaatgac agagccagtg ggaagataca gaaaaagagg gccggggctg 4620

ggcgcggtgg ttcacgcctg taatcccagc actttgggag gccaaggagg gtggatcacc 4680

tgaggtcagg agttagaggc cagcctggcg aaaccccatc tctactaaaa atacaaaatc 4740

caggcgtggt ggcacacacc tgtagtccca gctactcagg aggttgaggt aggagaattg 4800

cttgaacctg ggaggtggag gttgcagtga gccaagattg cgccattgca ctccagcctg 4860

ggcaacacag cgagactccg tctcaaggaa aaaataaaaa taaaaagcgg gcacgggccc 4920

ggacatcccc acccttggag gctgtcttct caggctctgc cctgccctag ctccacaccc 4980

tctcccagga cccatcacgc ctgtgcagtg gcccccacag aaagactgag ctcaaggtgg 5040

gaaccacgtc tgctaacttg gagccccagt gccaagcaca gtgcctgcat gtatttatcc 5100

aataaatgtg aaattctgtc caaaaaaaaa aaa 5133

<210> 30
<211> 4742
<212> DNA
<213> Homo sapiens

<400> 30
ttttctgccc ttctttgctt tggtggcttc cttgtggttc ctcagtggtg cctgcaaccc 60

ctggttcacc tccttccagg ttctggctcc ttccagccat ggctctcaga gtccttctgt 120

taacagcctt gaccttatgt catgggttca acttggacac tgaaaacgca atgaccttcc 180

aagagaacgc aaggggcttc gggcagagcg tggtccagct tcagggatcc agggtggtgg 240

ttggagcccc ccaggagata gtggctgcca accaaagggg cagcctctac cagtgcgact 300

acagcacagg ctcatgcgag cccatccgcc tgcaggtccc cgtggaggcc gtgaacatgt 360

ccctgggcct gtccctggca gccaccacca gcccccctca gctgctggcc tgtggtccca 420

ccgtgcacca gacttgcagt gagaacacgt atgtgaaagg gctctgcttc ctgtttggat 480

ccaacctacg gcagcagccc cagaagttcc cagaggccct ccgagggtgt cctcaagagg 540

atagtgacat tgccttcttg attgatggct ctggtagcat catcccacat gactttcggc 600

ggatgaagga gtttgtctca actgtgatgg agcaattaaa aaagtccaaa accttgttct 660

ctttgatgca gtactctgaa gaattccgga ttcactttac cttcaaagag ttccagaaca 720

accctaaccc aagatcactg gtgaagccaa taacgcagct gcttgggcgg acacacacgg 780

ccacgggcat ccgcaaagtg gtacgagagc tgtttaacat caccaacgga gcccgaaaga 840

atgcctttaa gatcctagtt gtcatcacgg atggagaaaa gtttggcgat cccttgggat 900

atgaggatgt catccctgag gcagacagag agggagtcat tcgctacgtc attggggtgg 960

gagatgcctt ccgcagtgag aaatcccgcc aagagcttaa taccatcgca tccaagccgc 1020

ctcgtgatca cgtgttccag gtgaataact ttgaggctct gaagaccatt cagaaccagc 1080

ttcgggagaa gatctttgcg atcgagggta ctcagacagg aagtagcagc tcctttgagc 1140

atgagatgtc tcaggaaggc ttcagcgctg ccatcacctc taatggcccc ttgctgagca 1200

ctgtggggag ctatgactgg gctggtggag tctttctata tacatcaaag gagaaaagca 1260
ccttcatcaa catgaccaga gtggattcag acatgaatga tgcttacttg ggttatgctg 1320
ccgccatcat cttacggaac cgggtgcaaa gcctggttct gggggcacct cgatatcagc 1380
acatcggcct ggtagcgatg ttcaggcaga acactggcat gtgggagtcc aacgctaatg 1440
tcaagggcac ccagatcggc gcctacttcg gggcctccct ctgctccgtg gacgtggaca 1500
gcaacggcag caccgacctg gtcctcatcg gggcccccca ttactacgag cagacccgag 1560
ggggccaggt gtccgtgtgc cccttgccca gggggagggc tcggtggcag tgtgatgctg 1620
ttctctacgg ggagcagggc caaccctggg gccgctttgg ggcagcccta acagtgctgg 1680
gggacgtaaa tggggacaag ctgacggacg tggccattgg ggccccagga gaggaggaca 1740
accggggtgc tgtttacctg tttcacggaa cctcaggatc tggcatcagc ccctcccata 1800
gccagcggat agcaggctcc aagctctctc ccaggctcca gtattttggt cagtcactga 1860
gtgggggcca ggacctcaca atggatggac tggtagacct gactgtagga gcccaggggc 1920
acgtgctgct gctcaggtcc cagccagtac tgagagtcaa ggcaatcatg gagttcaatc 1980
ccagggaagt ggcaaggaat gtatttgagt gtaatgatca ggtggtgaaa ggcaaggaag 2040
ccggagaggt cagagtctgc ctccatgtcc agaagagcac acgggatcgg ctaagagaag 2100
gacagatcca gagtgttgtg acttatgacc tggctctgga ctccggccgc ccacattccc 2160
gcgccgtctt caatgagaca aagaacagca cacgcagaca gacacaggtc ttggggctga 2220
cccagacttg tgagaccctg aaactacagt tgccgaattg catcgaggac ccagtgagcc 2280
ccattgtgct gcgcctgaac ttctctctgg tgggaacgcc attgtctgct ttcgggaacc 2340
tccggccagt gctggcggag gatgctcaga gactcttcac agccttgttt ccctttgaga 2400
agaattgtgg caatgacaac atctgccagg atgacctcag catcaccttc agtttcatga 2460
gcctggactg cctcgtggtg ggtgggcccc gggagttcaa cgtgacagtg actgtgagaa 2520
atgatggtga ggactcctac aggacacagg tcaccttctt cttcccgctt gacctgtcct 2580
accggaaggt gtccacgctc cagaaccagc gctcacagcg atcctggcgc ctggcctgtg 2640
agtctgcctc ctccaccgaa gtgtctgggg ccttgaagag caccagctgc agcataaacc 2700
accccatctt cccggaaaac tcagaggtca cctttaatat cacgtttgat gtagactcta 2760
aggcttccct tggaaacaaa ctgctcctca aggccaatgt gaccagtgag aacaacatgc 2820
ccagaaccaa caaaaccgaa ttccaactgg agctgccggt gaaatatgct gtctacatgg 2880
tggtcaccag ccatggggtc tccactaaat atctcaactt cacggcctca gagaatacca 2940
gtcgggtcat gcagcatcaa tatcaggtca gcaacctggg gcagaggagc ctccccatca 3000
gcctggtgtt cttggtgccc gtccggctga accagactgt catatgggac cgcccccagg 3060
tcaccttctc cgagaacctc tcgagtacgt gccacaccaa ggagcgcttg ccctctcact 3120

ccgactttct ggctgagctt cggaaggccc ccgtggtgaa ctgctccatc gctgtctgcc 3180

agagaatcca gtgtgacatc ccgttctttg gcatccagga agaattcaat gctaccctca 3240

aaggcaacct ctcgtttgac tggtacatca agacctcgca taaccacctc ctgatcgtga 3300

gcacagctga gatcttgttt aacgattccg tgttcaccct gctgccggga cagggggcgt 3360

ttgtgaggtc ccagacggag accaaagtgg agccgttcga ggtccccaac cccctgccgc 3420

tcatcgtggg cagctctgtc gggggactgc tgctcctggc cctcatcacc gccgcgctgt 3480

acaagctcgg cttcttcaag cggcaataca aggacatgat gagtgaaggg ggtcccccgg 3540

gggccgaacc ccagtagcgg ctccttcccg acagagctgc ctctcggtgg ccagcaggac 3600

tctgcccaga ccacacgtag cccccaggct gctggacacg tcggacagcg aagtatcccc 3660

gacaggacgg gcttgggctt ccatttgtgt gtgtgcaagt gtgtatgtgc gtgtgtgcaa 3720

gtgtctgtgt gcaagtgtgt gcacatgtgt gcgtgtgcgt gcatgtgcac ttgcacgccc 3780

atgtgtgagt gtgtgcaagt atgtgagtgt gtccaagtgt gtgtgcgtgt gtccatgtgt 3840

gtgcaagtgt gtgcatgtgt gcgagtgtgt gcatgtgtgt gctcaggggc gtgtggctca 3900

cgtgtgtgac tcagatgtct ctggcgtgtg ggtaggtgac ggcagcgtag cctctccggc 3960

agaagggaac tgcctgggct cccttgtgcg tgggtgaagc cgctgctggg ttttcctccg 4020

ggagagggga cggtcaatcc tgtgggtgaa gacagaggga aacacagcag cttctctcca 4080

ctgaaagaag tgggacttcc cgtcgcctgc gagcctgcgg cctgctggag cctgcgcagc 4140

ttggatggag actccatgag aagccgtggg tggaaccagg aacctcctcc acaccagcgc 4200

tgatgcccaa taaagatgcc cactgaggaa tgatgaagct tcctttctgg attcatttat 4260

tatttcaatg tgactttaat tttttggatg gataagcttg tctatggtac aaaaatcaca 4320

aggcattcaa gtgtacagtg aaaagtctcc ctttccagat attcaagtca cctccttaaa 4380

ggtagtcaag attgtgtttt gaggtttcct tcagacagat tccaggcgat gtgcaagtgt 4440

atgcacgtgt gcacacacac cacacataca cacacacaag cttttttaca caaatggtag 4500

catactttat attggtctgt atcttgcttt ttttcaccaa tatttctcag acatcggttc 4560

atattaagac ataaattact ttttcattct tttataccgc tgcatagtat tccattgtgt 4620

gagtgtacca taatgtattt aaccagtctt cttttgatat actattttca ttctcttgtt 4680

attgcatcaa tgctgagtta ataaatcaaa tatatgtcat ttttgcatat atgtaaggat 4740

aa 4742

<210> 31
<211> 4666
<212> DNA
<213> Homo sapiens

<400> 31
agtaccttgg tccagctctt cctgcaacgg cccaggagct cagagctcca catctgacct 60

tctagtcatg accaggacca gggcagcact cctcctgttc acagccttag caacttctct 120
aggtttcaac ttggacacag aggagctgac agccttccgt gtggacagcg ctgggtttgg 180
agacagcgtg gtccagtatg ccaactcctg ggtggtggtt ggagcccccc aaaagataac 240
agctgccaac caaacgggtg gcctctacca gtgtggctac agcactggtg cctgtgagcc 300
catcggcctg caggtgcccc cggaggccgt gaacatgtcc ctgggcctgt ccctggcgtc 360
taccaccagc ccttcccagc tgctggcctg cggccccacc gtgcaccacg agtgcgggag 420
gaacatgtac ctcaccggac tctgcttcct cctgggcccc acccagctca cccagaggct 480
cccggtgtcc aggcaggagt gcccaagaca ggagcaggac attgtgttcc tgatcgatgg 540
ctcaggcagc atctcctccc gcaactttgc cacgatgatg aacttcgtga gagctgtgat 600
aagccagttc cagagaccca gcacccagtt ttccctgatg cagttctcca acaaattcca 660
aacacacttc actttcgagg aattcaggcg cagctcaaac cccctcagcc tgttggcttc 720
tgttcaccag ctgcaagggt ttacatacac ggccaccgcc atccaaaatg tcgtgcaccg 780
attgttccat gcctcatatg gggcccgtag ggatgccgcc aaaattctca ttgtcatcac 840
tgatgggaag aaagaaggcg acagcctgga ttataaggat gtcatcccca tggctgatgc 900
agcaggcatc atccgctatg caattggggt tggattagct tttcaaaaca gaaattcttg 960
gaaagaatta aatgacattg catcgaagcc ctcccaggaa cacatattta aagtggagga 1020
ctttgatgct ctgaaagata ttcaaaacca actgaaggag aagatctttg ccattgaggg 1080
tacggagacc acaagcagta gctccttcga attggagatg gcacaggagg gcttcagcgc 1140
tgtgttcaca cctgatggcc ccgttctggg ggctgtgggg agcttcacct ggtctggagg 1200
tgccttcctg taccccccaa atatgagccc taccttcatc aacatgtctc aggagaatgt 1260
ggacatgagg gactcttacc tgggttactc caccgagctg gccctctgga aaggggtgca 1320
gagcctggtc ctgggggccc cccgctacca gcacaccggg aaggctgtca tcttcaccca 1380
ggtgtccagg caatggagga tgaaggccga agtcacgggg actcagatcg gctcctactt 1440
cggggcctcc ctctgctccg tggacgtaga cagcgacggc agcaccgacc tggtcctcat 1500
cggggccccc cattactacg agcagacccg agggggccag gtgtctgtgt gtcccttgcc 1560
cagggggtgg agaaggtggt ggtgtgatgc tgttctctac ggggagcagg gccacccctg 1620
gggtcgcttt ggggcggctc tgacagtgct gggggatgtg aatggggaca agctgacaga 1680
cgtggtcatc ggggccccag gagaggagga gaaccggggt gctgtctacc tgtttcacgg 1740
agtcttggga cccagcatca gcccctccca cagccagcgg atcgcgggct cccagctctc 1800
ctccaggctg cagtattttg ggcaggcact gagcgggggt caagacctca cccaggatgg 1860
actggtggac ctggctgtgg gggcccgggg ccaggtgctc ctgctcagga ccagacctgt 1920
gctctgggtg ggggtgagca tgcagttcat acctgccgag atccccaggt ctgcgtttga 1980
gtgtcgggag caggtggtct ctgagcagac cctggtacag tccaacatct gcctttacat 2040

tgacaaacgt tctaagaacc tgcttgggag ccgtgacctc caaagctctg tgaccttgga 2100

cctggccctc gaccctggcc gcctgagtcc ccgtgccacc ttccaggaaa caaagaaccg 2160

gagtctgagc cgagtccgag tcctcgggct gaaggcacac tgtgaaaact tcaacctgct 2220

gctcccgagc tgcgtggagg actctgtgac ccccattacc ttgcgtctga acttcacgct 2280

ggtgggcaag cccctccttg ccttcagaaa cctgcggcct atgctggccg ccgatgctca 2340

gagatacttc acggcctccc taccctttga gaagaactgt ggagccgacc atatctgcca 2400

ggacaatctc ggcatctcct tcagcttccc aggcttgaag tccctgctgg tggggagtaa 2460

cctggagctg aacgcagaag tgatggtgtg gaatgacggg gaagactcct acggaaccac 2520

catcaccttc tcccaccccg caggactgtc ctaccgctac gtggcagagg gccagaaaca 2580

agggcagctg cgttccctgc acctgacatg tgacagcgcc ccagttggga gccagggcac 2640

ctggagcacc agctgcagaa tcaaccacct catcttccgt ggcggcgccc agatcacctt 2700

cttggctacc tttgacgtct cccccaaggc tgtcctggga gaccggctgc ttctgacagc 2760

caatgtgagc agtgagaaca acactcccag gaccagcaag accaccttcc agctggagct 2820

cccggtgaag tatgctgtct acactgtggt tagcagccac gaacaattca ccaaatacct 2880

caacttctca gagtctgagg agaaggaaag ccatgtggcc atgcacagat accaggtcaa 2940

taacctggga cagagggacc tgcctgtcag catcaacttc tgggtgcctg tggagctgaa 3000

ccaggaggct gtgtggatgg atgtggaggt ctcccacccc cagaacccat cccttcggtg 3060

ctcctcagag aaaatcgcac ccccagcatc tgacttcctg gcgcacattc agaagaatcc 3120

cgtgctggac tgctccattg ctggctgcct gcggttccgc tgtgacgtcc cctccttcag 3180

cgtccaggag gagctggatt tcaccctgaa gggcaacctc agctttggct gggtccgcca 3240

gatattgcag aagaaggtgt cggtcgtgag tgtggctgaa attacgttcg acacatccgt 3300

gtactcccag cttccaggac aggaggcatt tatgagagct cagacgacaa cggtgctgga 3360

gaagtacaag gtccacaacc ccacccccct catcgtaggc agctccattg ggggtctgtt 3420

gctgctggca ctcatcacag cggtactgta caaagttggc ttcttcaagc gtcagtacaa 3480

ggaaatgatg gaggaggcaa atggacaaat tgccccagaa aacgggacac agacccccag 3540

cccgcccagt gagaaatgat cccctctttg ccttggactt cttctccccc gcgagttttc 3600

cccacttact taccctcacc tgtcaggcct gacggggagg aaccactgca ccaccgagag 3660

aggctgggat gggcctgctt cctgtctttg ggagaaaacg tcttgcttgg gaaggggcct 3720

ttgtcttgtc aaggttccaa ctggaaaccc ttaggacagg gtccctgctg tgttccccaa 3780

aggacttgac ttgcaatttc tacctagaaa tacatggaca atacccccag gcctcagtct 3840

cccttctccc atgaggcacg aatgatcttt ctttcctttc tttttttttt tttttctttt 3900

cttttttttt tttttgagac ggagtctcgc tctgtcaccc aggctggagt gcaatggcgt 3960

gatctcggct cactgcaacc tccgcctccc gggttcaagt aattctgctg tctcagcctc 4020

ctgagtagct gggactacag gcacacgcca cctcgcccgg cccgatcttt ctaaaataca 4080

gttctgaata tgctgctcat ccccacctgt cttcaacagc tccccattac cctcaggaca 4140

atgtctgaac tctccagctt cgcgtgagaa gtccccttcc atcccagagg gtgggcttca 4200

gggcgcacag catgagaggc tctgtgcccc catcaccctc gtttccagtg aattagtgtc 4260

atgtcagcat cagctcaggg cttcatcgtg gggctctcag ttccgatttc ccaggctgaa 4320

ttgggagtga gatgcctgca tgctgggttc tgcacagctg gcctcccgcg ttgggcaaca 4380

ttgctggctg gaagggagga gcgccctcta gggagggaca tggccccggt gcggctgcag 4440

ctcacccagc cccaggggca gaagagaccc aaccacttct attttttgag gctatgaata 4500

tagtacctga aaaaatgcca agacatgatt atttttttaa aaagcgtact ttaaatgttt 4560

gtgttaataa attaaaacat gcacaaaaag atgcatctac cgctcttggg aaatatgtca 4620

aaggtctaaa aataaaaaag ccttctgtga aaaaaaaaaa aaaaaa 4666

<210> 32
<211> 2291
<212> DNA
<213> Homo sapiens

<400> 32
ctcgccctgg tggggctgct ctccctcggg tgcgtcctct ctcaggagtg cacgaagttc 60

aaggtcagca gctgccggga atgcatcgag tcggggcccg gctgcacctg gtgccagaag 120

ctgaacttca cagggccggg ggatcctgac tccattcgct gcgacacccg gccacagctg 180

ctcatgaggg gctgtgcggc tgacgacatc atggacccca caagcctcgc tgaaacccag 240

gaagaccaca atgggggcca gaagcagctg tccccacaaa aagtgacgct ttacctgcga 300

ccaggccagg cagcagcgtt caacgtgacc ttccggcggg ccaagggcta ccccatcgac 360

ctgtactatc tgatggacct ctcctactcc atgcttgatg acctcaggaa tgtcaagaag 420

ctaggtggcg acctgctccg ggccctcaac gagatcaccg agtccggccg cattggcttc 480

gggtccttcg tggacaagac cgtgctgccg ttcgtgaaca cgcaccctga taagctgcga 540

aacccatgcc ccaacaagga gaaagagtgc ccgcccccgt ttgccttcag gcacgtgctg 600

aagctgacca acaactccaa ccagtttcag accgaggtcg ggaagcagct gatttccgga 660

aacctggatg cacccgaggg tgggctggac gccatgatgc aggtcgccgc ctgcccggag 720

gaaatcggct ggcgcaacgt cacgcggctg ctggtgtttg ccactgatga cggcttccat 780

ttcgcgggcg acggaaagct gggcgccatc ctgaccccca acgacggccg ctgtcacctg 840

gaggacaact tgtacaagag gagcaacgaa ttcgactacc catcggtggg ccagctggcg 900

cacaagctgg ctgaaaacaa catccagccc atcttcgcgg tgaccagtag gatggtgaag 960

acctacgaga aactcaccga gatcatcccc aagtcagccg tgggggagct gtctgaggac 1020

tccagcaatg tggtccatct cattaagaat gcttacaata aactctcctc cagggtcttc 1080

ctggatcaca acgccctccc cgacaccctg aaagtcacct acgactcctt ctgcagcaat 1140

ggagtgacgc acaggaacca gcccagaggt gactgtgatg gcgtgcagat caatgtcccg 1200

atcaccttcc aggtgaaggt cacggccaca gagtgcatcc aggagcagtc gtttgtcatc 1260

cgggcgctgg gcttcacgga catagtgacc gtgcaggtcc ttccccagtg tgagtgccgg 1320

tgccgggacc agagcagaga ccgcagcctc tgccatggca agggcttctt ggagtgcggc 1380

atctgcaggt gtgacactgg ctacattggg aaaaactgtg agtgccagac acagggccgg 1440

agcagccagg agctggaagg aagctgccgg aaggacaaca actccatcat ctgctcaggg 1500

ctgggggact gtgtctgcgg gcagtgcctg tgccacacca gcgacgtccc cggcaagctg 1560

atatacgggc agtactgcga gtgtgacacc atcaactgtg agcgctacaa cggccaggtc 1620

tgcggcggcc cggggagggg gctctgcttc tgcgggaagt gccgctgcca cccgggcttt 1680

gagggctcag cgtgccagtg cgagaggacc actgagggct gcctgaaccc gcggcgtgtt 1740

gagtgtagtg gtcgtggccg gtgccgctgc aacgtatgcg agtgccattc aggctaccag 1800

ctgcctctgt gccaggagtg ccccggctgc ccctcaccct gtggcaagta catctcctgc 1860

gccgagtgcc tgaagttcga aaagggcccc tttgggaaga actgcagcgc ggcgtgtccg 1920

ggcctgcagc tgtcgaacaa ccccgtgaag ggcaggacct gcaaggagag ggactcagag 1980

ggctgctggg tggcctacac gctggagcag caggacggga tggaccgcta cctcatctat 2040

gtggatgaga gccgagagtg tgtggcaggc cccaacatcg ccgccatcgt cgggggcacc 2100

gtggcaggca tcgtgctgat cggcattctc ctgctggtca tctggaaggc tctgatccac 2160

ctgagcgacc tccgggagta caggcgcttt gagaaggaga agctcaagtc ccagtggaac 2220

aatgataatc cccttttcaa gagcgccacc acgacggtca tgaaccccaa gtttgctgag 2280

agttaggagc a 2291

<210> 33

<400> 33
000

<210> 34
<211> 1038
<212> PRT
<213> Homo sapiens

<400> 34

Met Phe Pro Thr Glu Ser Ala Trp Leu Gly Lys Arg Gly Ala Asn Pro
1               5                   10                  15

Gly Pro Glu Ala Ala Val Arg Glu Thr Val Met Leu Leu Leu Cys Leu
            20                  25                  30

Gly Val Pro Thr Gly Arg Pro Tyr Asn Val Asp Thr Glu Ser Ala Leu
        35                  40                  45

Leu Tyr Gln Gly Pro His Asn Thr Leu Phe Gly Tyr Ser Val Val Leu
    50                  55                  60

His Ser His Gly Ala Asn Arg Trp Leu Leu Val Gly Ala Pro Thr Ala
65                  70                  75                  80

Asn Trp Leu Ala Asn Ala Ser Val Ile Asn Pro Gly Ala Ile Tyr Arg
                85                  90                  95

Cys Arg Ile Gly Lys Asn Pro Gly Gln Thr Cys Glu Gln Leu Gln Leu
            100                 105                 110

Gly Ser Pro Asn Gly Glu Pro Cys Gly Lys Thr Cys Leu Glu Glu Arg
        115                 120                 125

Asp Asn Gln Trp Leu Gly Val Thr Leu Ser Arg Gln Pro Gly Glu Asn
    130                 135                 140

Gly Ser Ile Val Thr Cys Gly His Arg Trp Lys Asn Ile Phe Tyr Ile
145                 150                 155                 160

Lys Asn Glu Asn Lys Leu Pro Thr Gly Gly Cys Tyr Gly Val Pro Pro
                165                 170                 175

Asp Leu Arg Thr Glu Leu Ser Lys Arg Ile Ala Pro Cys Tyr Gln Asp
            180                 185                 190

Tyr Val Lys Lys Phe Gly Glu Asn Phe Ala Ser Cys Gln Ala Gly Ile
        195                 200                 205

Ser Ser Phe Tyr Thr Lys Asp Leu Ile Val Met Gly Ala Pro Gly Ser
    210                 215                 220

Ser Tyr Trp Thr Gly Ser Leu Phe Val Tyr Asn Ile Thr Thr Asn Lys
225                 230                 235                 240

Tyr Lys Ala Phe Leu Asp Lys Gln Asn Gln Val Lys Phe Gly Ser Tyr
                245                 250                 255

Leu Gly Tyr Ser Val Gly Ala Gly His Phe Arg Ser Gln His Thr Thr
            260                 265                 270

Glu Val Val Gly Gly Ala Pro Gln His Glu Gln Ile Gly Lys Ala Tyr
        275                 280                 285

Ile Phe Ser Ile Asp Glu Lys Glu Leu Asn Ile Leu His Glu Met Lys

290                 295                 300

Gly Lys Lys Leu Gly Ser Tyr Phe Gly Ala Ser Val Cys Ala Val Asp
305                 310                 315                 320

Leu Asn Ala Asp Gly Phe Ser Asp Leu Leu Val Gly Ala Pro Met Gln
                325                 330                 335

Ser Thr Ile Arg Glu Glu Gly Arg Val Phe Val Tyr Ile Asn Ser Gly
            340                 345                 350

Ser Gly Ala Val Met Asn Ala Met Glu Thr Asn Leu Val Gly Ser Asp
        355                 360                 365

Lys Tyr Ala Ala Arg Phe Gly Glu Ser Ile Val Asn Leu Gly Asp Ile
    370                 375                 380

Asp Asn Asp Gly Phe Glu Asp Val Ala Ile Gly Ala Pro Gln Glu Asp
385                 390                 395                 400

Asp Leu Gln Gly Ala Ile Tyr Ile Tyr Asn Gly Arg Ala Asp Gly Ile
                405                 410                 415

Ser Ser Thr Phe Ser Gln Arg Ile Glu Gly Leu Gln Ile Ser Lys Ser
            420                 425                 430

Leu Ser Met Phe Gly Gln Ser Ile Ser Gly Gln Ile Asp Ala Asp Asn
        435                 440                 445

Asn Gly Tyr Val Asp Val Ala Val Gly Ala Phe Arg Ser Asp Ser Ala
    450                 455                 460

Val Leu Leu Arg Thr Arg Pro Val Val Ile Val Asp Ala Ser Leu Ser
465                 470                 475                 480

His Pro Glu Ser Val Asn Arg Thr Lys Phe Asp Cys Val Glu Asn Gly
                485                 490                 495

Trp Pro Ser Val Cys Ile Asp Leu Thr Leu Cys Phe Ser Tyr Lys Gly
            500                 505                 510

Lys Glu Val Pro Gly Tyr Ile Val Leu Phe Tyr Asn Met Ser Leu Asp
        515                 520                 525

Val Asn Arg Lys Ala Glu Ser Pro Pro Arg Phe Tyr Phe Ser Ser Asn
    530                 535                 540

Gly Thr Ser Asp Val Ile Thr Gly Ser Ile Gln Val Ser Ser Arg Glu
545                 550                 555                 560

Ala Asn Cys Arg Thr His Gln Ala Phe Met Arg Lys Asp Val Arg Asp
                565                 570                 575

Ile Leu Thr Pro Ile Gln Ile Glu Ala Ala Tyr His Leu Gly Pro His
            580                 585                 590

Val Ile Ser Lys Arg Ser Thr Glu Glu Phe Pro Pro Leu Gln Pro Ile
        595                 600                 605

Leu Gln Gln Lys Lys Glu Lys Asp Ile Met Lys Lys Thr Ile Asn Phe
    610                 615                 620

Ala Arg Phe Cys Ala His Glu Asn Cys Ser Ala Asp Leu Gln Val Ser
625                 630                 635                 640

Ala Lys Ile Gly Phe Leu Lys Pro His Glu Asn Lys Thr Tyr Leu Ala
                645                 650                 655

Val Gly Ser Met Lys Thr Leu Met Leu Asn Val Ser Leu Phe Asn Ala
            660                 665                 670

Gly Asp Asp Ala Tyr Glu Thr Thr Leu His Val Lys Leu Pro Val Gly
        675                 680                 685

Leu Tyr Phe Ile Lys Ile Leu Glu Leu Glu Glu Lys Gln Ile Asn Cys
    690                 695                 700

Glu Val Thr Asp Asn Ser Gly Val Val Gln Leu Asp Cys Ser Ile Gly
705                 710                 715                 720

Tyr Ile Tyr Val Asp His Leu Ser Arg Ile Asp Ile Ser Phe Leu Leu
                725                 730                 735

Asp Val Ser Ser Leu Ser Arg Ala Glu Glu Asp Leu Ser Ile Thr Val
            740                 745                 750

His Ala Thr Cys Glu Asn Glu Glu Glu Met Asp Asn Leu Lys His Ser
        755                 760                 765

Arg Val Thr Val Ala Ile Pro Leu Lys Tyr Glu Val Lys Leu Thr Val
    770                 775                 780

His Gly Phe Val Asn Pro Thr Ser Phe Val Tyr Gly Ser Asn Asp Glu
785                 790                 795                 800

Asn Glu Pro Glu Thr Cys Met Val Glu Lys Met Asn Leu Thr Phe His
                805                 810                 815

Val Ile Asn Thr Gly Asn Ser Met Ala Pro Asn Val Ser Val Glu Ile
            820                 825                 830

Met Val Pro Asn Ser Phe Ser Pro Gln Thr Asp Lys Leu Phe Asn Ile
        835                 840                 845

Leu Asp Val Gln Thr Thr Thr Gly Glu Cys His Phe Glu Asn Tyr Gln
    850                 855                 860

Arg Val Cys Ala Leu Glu Gln Gln Lys Ser Ala Met Gln Thr Leu Lys
865                 870                 875                 880

Gly Ile Val Arg Phe Leu Ser Lys Thr Asp Lys Arg Leu Leu Tyr Cys
                885                 890                 895

Ile Lys Ala Asp Pro His Cys Leu Asn Phe Leu Cys Asn Phe Gly Lys
            900                 905                 910

Met Glu Ser Gly Lys Glu Ala Ser Val His Ile Gln Leu Glu Gly Arg
        915                 920                 925

Pro Ser Ile Leu Glu Met Asp Glu Thr Ser Ala Leu Lys Phe Glu Ile
    930                 935                 940

Arg Ala Thr Gly Phe Pro Glu Pro Asn Pro Arg Val Ile Glu Leu Asn
945                 950                 955                 960

Lys Asp Glu Asn Val Ala His Val Leu Leu Glu Gly Leu His His Gln
                965                 970                 975

Arg Pro Lys Arg Tyr Phe Thr Ile Val Ile Ile Ser Ser Ser Leu Leu
            980                 985                 990

Leu Gly Leu Ile Val Leu Leu Leu Ile Ser Tyr Val Met Trp Lys Ala
        995                 1000                1005

Gly Phe Phe Lys Arg Gln Tyr Lys Ser Ile Leu Gln Glu Glu Asn
    1010                1015                1020

Arg Arg Asp Ser Trp Ser Tyr Ile Asn Ser Lys Ser Asn Asp Asp
    1025                1030                1035

<210> 35
<211> 3567
<212> DNA
<213> Homo sapiens

<400> 35
cgccatcccg cgctctgcgg actgggaggc ccgggccagg acgcgagtct gcgcagccga 60

ggttccccag cgccccctgc agccgcgcgt aggcagagac ggagcccggc cctgcgcctc 120
cgcaccacgc ccgggacccc acccagcggc ccgtacccgg agaagcagcg cgagcacccg 180
aagctcccgg ctcggcggca gaaaccggga gtggggccgg gcgagtgcgc ggcatcccag 240
gccggcccga acgtccgccc gcggtgggcc gacttcccct cctcttccct ctctccttcc 300
tttagcccgc tggcgccgga cacgctgcgc ctcatctctt ggggcgttct tccccgttgg 360
ccaaccgtcg catcccgtgc aactttgggg tagtggccgc ttagtgttga atgttcccca 420
ccgagagcgc atggcttggg aagcgaggcg cgaacccggg ccccgaagcc gccgtccggg 480
agacggtgat gctgttgctg tgcctggggg tcccgaccgg ccgcccctac aacgtggaca 540
ctgagagcgc gctgctttac cagggccccc acaacacgct gttcggctac tcggtcgtgc 600
tgcacagcca cggggcgaac cgatggctcc tagtgggtgc gcccactgcc aactggctcg 660
ccaacgcttc agtgatcaat cccggggcga tttacagatg caggatcgga aagaatcccg 720
gccagacgtg cgaacagctc cagctgggta gccctaatgg agaaccttgt ggaaagactt 780
gtttggaaga gagagacaat cagtggttgg gggtcacact ttccagacag ccaggagaaa 840
atggatccat cgtgacttgt gggcatagat ggaaaaatat attttacata aagaatgaaa 900
ataagctccc cactggtggt tgctatggag tgccccctga tttacgaaca gaactgagta 960
aaagaatagc tccgtgttat caagattatg tgaaaaaatt tggagaaaat tttgcatcat 1020
gtcaagctgg aatatccagt ttttacacaa aggatttaat tgtgatgggg gccccaggat 1080
catcttactg gactggctct ctttttgtct acaatataac tacaaataaa tacaaggctt 1140
ttttagacaa acaaaatcaa gtaaaatttg gaagttattt aggatattca gtcggagctg 1200
gtcattttcg gagccagcat actaccgaag tagtcggagg agctcctcaa catgagcaga 1260
ttggtaaggc atatatattc agcattgatg aaaaagaact aaatatctta catgaaatga 1320
aaggtaaaaa gcttggatcg tactttggag cttctgtctg tgctgtggac ctcaatgcag 1380
atggcttctc agatctgctc gtgggagcac ccatgcagag caccatcaga gaggaaggaa 1440
gagtgtttgt gtacatcaac tctggctcgg gagcagtaat gaatgcaatg gaaacaaacc 1500
tcgttggaag tgacaaatat gctgcaagat ttggggaatc tatagttaat cttggcgaca 1560
ttgacaatga tggctttgaa gatgttgcta tcggagctcc acaagaagat gacttgcaag 1620
gtgctattta tatttacaat ggccgtgcag atgggatctc gtcaaccttc tcacagagaa 1680
ttgaaggact tcagatcagc aaatcgttaa gtatgtttgg acagtctata tcaggacaaa 1740
ttgatgcaga taataatggc tatgtagatg tagcagttgg tgcttttcgg tctgattctg 1800
ctgtcttgct aaggacaaga cctgtagtaa ttgttgacgc ttctttaagc caccctgagt 1860
cagtaaatag aacgaaattt gactgtgttg aaaatggatg gccttctgtg tgcatagatc 1920
taacactttg tttctcatat aagggcaagg aagttccagg ttacattgtt ttgttttata 1980
acatgagttt ggatgtgaac agaaaggcag agtctccacc aagattctat ttctcttcta 2040

atggaacttc tgacgtgatt acaggaagca tacaggtgtc cagcagagaa gctaactgta 2100

gaacacatca agcatttatg cggaaagatg tgcgggacat cctcacccca attcagattg 2160

aagctgctta ccaccttggt cctcatgtca tcagtaaacg aagtacagag gaattcccac 2220

cacttcagcc aattcttcag cagaagaaag aaaaagacat aatgaaaaaa acaataaact 2280

ttgcaaggtt ttgtgcccat gaaaattgtt ctgctgattt acaggtttct gcaaagattg 2340

ggtttttgaa gccccatgaa aataaaacat atcttgctgt tgggagtatg aagacattga 2400

tgttgaatgt gtccttgttt aatgctggag atgatgcata tgaaacgact ctacatgtca 2460

aactacccgt gggtctttat ttcattaaga ttttagagct ggaagagaag caaataaact 2520

gtgaagtcac agataactct ggcgtggtac aacttgactg cagtattggc tatatatatg 2580

tagatcatct ctcaaggata gatattagct ttctcctgga tgtgagctca ctcagcagag 2640

cggaagagga cctcagtatc acagtgcatg ctacctgtga aaatgaagag gaaatggaca 2700

atctaaagca cagcagagtg actgtagcaa tacctttaaa atatgaggtt aagctgactg 2760

ttcatgggtt tgtaaaccca acttcatttg tgtatggatc aaatgatgaa aatgagcctg 2820

aaacgtgcat ggtggagaaa atgaacttaa ctttccatgt tatcaacact ggcaatagta 2880

tggctcccaa tgttagtgtg gaaataatgg taccaaattc ttttagcccc caaactgata 2940

agctgttcaa cattttggat gtccagacta ctactggaga atgccacttt gaaaattatc 3000

aaagagtgtg tgcattagag cagcaaaaga gtgcaatgca gaccttgaaa ggcatagtcc 3060

agttcttgtc caagactgat aagaggctat tgtactgcat aaaagctgat ccacattgtt 3120

taaatttctt gtgtaatttt gggaaaatgg aaagtggaaa agaagccagt gttcatatcc 3180

aactggaagg ccggccatcc attttagaaa tggatgagac ttcagcactc aagtttgaaa 3240

taagagcaac aggttttcca gagccaaatc caagagtaat tgaactaaac aaggatgaga 3300

atgttgcgca tgttctactg gaaggactac atcatcaaag acccaaacgt tatttcacca 3360

tagtgattat ttcaagtagc ttgctacttg gacttattgt acttctgttg atctcatatg 3420

ttatgtggaa ggctggcttc tttaaaagac aatacaaatc tatcctacaa gaagaaaaca 3480

gaagagacag ttggagttat atcaacagta aaagcaatga tgattaagga cttctttcaa 3540

attgagagaa tggaaaacag cccgccc 3567

<210> 36
<211> 591
<212> PRT
<213> Homo sapiens

<400> 36

Arg Thr Leu His Arg Asn Glu Tyr Gly Ile Ala Ser Ile Leu Asp Ser
1               5                   10                  15

Tyr Gln Cys Thr Ala Glu Ile Ser Leu Ala Asp Leu Ala Thr Ile Phe
            20                  25                  30

Phe Ala Gln Phe Val Gln Glu Ala Thr Tyr Lys Glu Val Ser Lys Met
        35                  40                  45

Val Lys Asp Ala Leu Thr Ala Ile Glu Lys Pro Thr Gly Asp Glu Gln
    50                  55                  60

Ser Ser Gly Cys Leu Glu Asn Gln Leu Pro Ala Phe Leu Glu Glu Leu
65                  70                  75                  80

Cys His Glu Lys Glu Ile Leu Glu Lys Tyr Gly His Ser Asp Cys Cys
                85                  90                  95

Ser Gln Ser Glu Glu Gly Arg His Asn Cys Phe Leu Ala His Lys Lys
            100                 105                 110

Pro Thr Pro Ala Ser Ile Pro Leu Phe Gln Val Pro Glu Pro Val Thr
        115                 120                 125

Ser Cys Glu Ala Tyr Glu Glu Asp Arg Glu Thr Phe Met Asn Lys Phe
    130                 135                 140

Ile Tyr Glu Ile Ala Arg Arg His Pro Phe Leu Tyr Ala Pro Thr Ile
145                 150                 155                 160

Leu Leu Trp Ala Ala Arg Tyr Asp Lys Ile Ile Pro Ser Cys Cys Lys
                165                 170                 175

Ala Glu Asn Ala Val Glu Cys Phe Gln Thr Lys Ala Ala Thr Val Thr
            180                 185                 190

Lys Glu Leu Arg Glu Ser Ser Leu Leu Asn Gln His Ala Cys Ala Val
        195                 200                 205

Met Lys Asn Phe Gly Thr Arg Thr Phe Gln Ala Ile Thr Val Thr Lys
    210                 215                 220

Leu Ser Gln Lys Phe Thr Lys Val Gln Phe Thr Glu Ile Gln Lys Leu
225                 230                 235                 240

Val Leu Asp Val Ala His Val His Glu His Cys Cys Arg Gly Asp Val
                245                 250                 255

Leu Asp Cys Leu Gln Asp Gly Glu Lys Ile Met Ser Tyr Ile Cys Ser
            260                 265                 270

Gln Gln Asp Thr Leu Ser Asn Lys Ile Thr Glu Cys Cys Lys Leu Thr

275                 280                 285

Thr Leu Glu Arg Gly Gln Cys Ile Ile His Ala Glu Asn Asp Glu Lys
    290                 295                 300

Pro Glu Gly Leu Ser Pro Asn Leu Asn Arg Phe Leu Gly Asp Arg Asp
305                 310                 315                 320

Phe Asn Gln Phe Ser Ser Gly Glu Lys Asn Ile Phe Leu Ala Ser Phe
                325                 330                 335

Val His Glu Tyr Ser Arg Arg His Pro Gln Leu Ala Val Ser Val Ile
            340                 345                 350

Leu Arg Val Ala Lys Gly Tyr Gln Glu Leu Leu Glu Lys Cys Phe Gln
        355                 360                 365

Thr Glu Asn Pro Leu Glu Cys Gln Asp Lys Gly Glu Glu Glu Leu Gln
    370                 375                 380

Lys Tyr Ile Gln Glu Ser Gln Ala Leu Ala Lys Arg Ser Cys Gly Leu
385                 390                 395                 400

Phe Gln Lys Leu Gly Glu Tyr Tyr Leu Gln Asn Ala Phe Leu Val Ala
                405                 410                 415

Tyr Thr Lys Lys Ala Pro Gln Leu Thr Ser Ser Glu Leu Met Ala Ile
            420                 425                 430

Thr Arg Lys Met Ala Ala Thr Ala Ala Thr Cys Cys Gln Leu Ser Glu
        435                 440                 445

Asp Lys Leu Leu Ala Cys Gly Glu Gly Ala Ala Asp Ile Ile Ile Gly
    450                 455                 460

His Leu Cys Ile Arg His Glu Met Thr Pro Val Asn Pro Gly Val Gly
465                 470                 475                 480

Gln Cys Cys Thr Ser Ser Tyr Ala Asn Arg Arg Pro Cys Phe Ser Ser
                485                 490                 495

Leu Val Val Asp Glu Thr Tyr Val Pro Pro Ala Phe Ser Asp Asp Lys
            500                 505                 510

Phe Ile Phe His Lys Asp Leu Cys Gln Ala Gln Gly Val Ala Leu Gln
        515                 520                 525

Thr Met Lys Gln Glu Phe Leu Ile Asn Leu Val Lys Gln Lys Pro Gln
    530                 535                 540

Ile Thr Glu Glu Gln Leu Glu Ala Val Ile Ala Asp Phe Ser Gly Leu
545                 550                 555                 560

Leu Glu Lys Cys Cys Gln Gly Gln Glu Gln Glu Val Cys Phe Ala Glu
                565                 570                 575

Glu Gly Gln Lys Leu Ile Ser Lys Thr Arg Ala Ala Leu Gly Val
            580                 585                 590

## Description

### Field of the Invention

This invention relates to treatment methods using alpha-fetoprotein, including its biologically active fragments, analogs, and derivatives, in conjunction with administration of an integrin antagonist for the treatment of multiple sclerosis.

### Background of the Invention

Multiple Sclerosis (MS) is a neurological disease first described in Holland by a 14th century physician, and which is characterized by irreversible degeneration of the nerves of the central nervous system (CNS). Although the underlying cause is unclear, the neurodegeneration in MS is the direct result of demyelination, or the stripping of myelin, a protein that normally lines the outer layer and insulates the nerves. As the condition progresses, patches of inflammation and scarring develop, interfering with the function of the nerves. Consequently, an MS patient gradually loses sensory and motor functions of the body. About 400,000 to 500,000 people in the U.S. suffer from MS. Usually, a patient is diagnosed with MS between 20 and 40 years of age, but MS has been diagnosed as early as age 15 and as late as age 60. MS is relentless and progressively destructive unless the patient receives medical therapy that is effective in halting or slowing the deterioration. While some individuals manage well in the short term, MS patients invariably become more significantly impaired by the disease over time.

Current therapies for MS are aimed at alleviating the symptoms of the disease and arresting its progress. Drug treatment usually entails the use of disease-modifying agents, such as the interferons (interferon-β-1a, β-1b, and α), glatiramer acetate, or corticosteroids, such as methylprednisolone and prednisone. Chemotherapeutic agents, such as mitoxantrone, methotrexate, azathioprine, and cladribine cyclophosphamide are also used to treat MS.

Thought to be an autoimmune disease, MS is also treated with various immunologic therapies. For example, cyclosporine, an immunosuppressive agent is used to treat MS. In addition, natalizumab (Tysabri^{®}, Elan Corporation, Inc./Biogen-Idec), a selective adhesion molecule inhibitor introduced in 2005, has also been used for the treatment of MS. Natalizumab, a humanized anti-α4 integrin antibody, has been shown to block autoimmiune encephalomyelitis in a rat and a mouse model (Yednock et al., Nature 356:63, 1992; Baron et al., J. Exp. Med. 177:57, 1993). Many of the current treatments for MS either lack efficacy, or pose serious risks and side effects. For example, natalizumab can increase the risk of progressive multifocal leukoencephalopathy (PML), an opportunistic viral infection of the brain that typically leads to death or severe disability. Thus, there remains a need for new and effective therapeutic approaches for the treatment of MS. The present invention addresses this and other related needs.

### Summary of the Invention

The present invention features compositions and methods for treating, preventing, or reducing one or more of the symptoms of MS (e.g., tingling, numbness, tremors, loss of balance, weakness in one or more limbs, blurred or double vision, slurred speech, swallowing problems, paralysis, lack or coordination, cognitive difficulties, fatigue, muscle spasms, dizziness, breathing problems, and seizures) or the progression of MS, in a patient by co-administering alpha-fetoprotein (AFP) and an integrin antagonist. The integrin antagonist can be, e.g., an antibody, a blocking peptide, a nucleic acid inhibitor, or a small molecule, as is described herein.

In a first aspect, the present invention features a method of treating a patient with MS by administering AFP (or biologically active fragment, derivative, or analog thereof) and an integrin antagonist, each in an amount (e.g., a therapeutically effective amount), to the patient.

In an embodiment, the integrin antagonist is an α4 integrin antagonist. The integrin antagonist can be, e.g., an antibody, such as natalizumab, a blocking peptide, a nucleic acid inhibitor, or a small molecule inhibitor.

Different administration schedules can be followed in the above method. For instance, the AFP (or biologically active fragment, derivative, or analog thereof) or the integrin antagonist can be administered one or more times (e.g., 1, 2, 3, 4, 5, or 10 times or more) hourly, daily, weekly, biweekly, or monthly. In addition, the dosage of the AFP (or biologically active fragment, derivative, or analog thereof) per administration may be the same or different.

In other embodiments of the above method, the AFP (or biologically active fragment, derivative, or analog thereof), and the integrin antagonist are administered coextensively or separately. Many variations of administration schemes are possible, for example, both the AFP (or a biologically active fragment, derivative, or analog thereof) and the integrin antagonist may be administered to the patient during the first treatment phase. Subsequently, the administration of one (e.g., the AFP or the integrin antagonist) may be terminated or the dosage amount may be modified (e.g., increased or decreased) while administration of the other is continued (e.g., at the same dosage level or at a modified level (e.g., increased or decreased)). Alternatively, both the AFP (or biologically active fragment, derivative, or analog thereof) and the integrin antagonist may be administered initially at their maximal or minimal dosages with subsequent dosages of both being reduced or increased, respectively, during the treatment regimen. In addition, the AFP (or biologically active fragment, derivative, or analog thereof) may be administered prior to or following administration of the integrin antagonist.

In an additional embodiment of the method, the AFP (or a biologically active fragment, derivative, or analog thereof) and the integrin antagonist are administered in the same dosage form or separate dosage forms. In additional embodiments of the method, the AFP (or biologically active fragment, derivative, or analog thereof) is administered at a dosage in the range of 0.1 mg to 400 mg and/or the integrin antagonist is administered at a dosage in the range of 0.1 mg to 500 mg.

In another embodiment of the method, the AFP (or a biologically active fragment, derivative, or analog thereof) and the integrin antagonist are administered via the same route of administration or via two different routes of administration.

In an embodiment, the method further includes administering a supplemental agent, such as an antagonist of one or more of the following proteins: CD80, P-selectin, sphingosine-1-phosphate receptor-1, hyaluronate receptor, leukocyte function antigen-1 (LFA-1), CD11/CD18, CD20, CD86, ICOS ligand, CCR2, CXCR3, CCR5, CD40, CD154, CD28, IL-23, IL-17, and IL-6. The supplemental agent can be, e.g., an antibody, a blocking peptide, a nucleic acid inhibitor, or a small molecule inhibitor. The CD80, P-selectin, sphingosine-1-phosphate receptor-1, hyaluratone receptor, LFA-1, CD11/CD18, CD20, CD86, ICOS ligand, CCR2, CXCR3, CCR5, CD40, CD154, CD28, IL-23, IL-17, or IL-6 antagonist can be administered co-extensively with AFP (or a biologically active fragment, derivative, or analog thereof) and without an integrin antagonist; with an integrin antagonist and without AFP; or all three can be administered in combination.

In a second aspect, the invention features a composition that includes an AFP (or a biologically active fragment, derivative, or analog thereof) and an integrin antagonist, each in an amount (e.g., a therapeutically effective amount) to treat, prevent, or reduce one or more of the symptoms of or the progression of, MS in a patient in need thereof.

In a related embodiment, the composition further includes an amount (e.g., a therapeutically effective amount) of an antagonist of one or more of the following proteins: CD80, P-selectin, sphingosine-1-phosphate receptor-1, hyaluronate receptor, LFA-1, CD11/CD18, CD20, CD86, ICOS ligand, CCR2, CXCR3, CCR5, CD40, CD154, CD28, IL-23, IL-17, and IL-6.

In additional embodiments of the composition, the AFP (or a biologically active fragment, derivative, or analog thereof) is in a dose in the range of 0.1 mg to 400 mg and/or the integrin antagonist is in a dose in the range of 0.1 mg to 500 mg. The composition of the invention may be administered to a patient with MS according to the first aspect of the invention.

A third aspect of the invention features a kit that includes 1) an AFP, or a biologically active fragment, derivative, or analog thereof, and an integrin antagonist (e.g., natalizumab), each in an amount (e.g., a therapeutically effective amount) to treat, prevent, or reduce one or more of the symptoms of or the progression of, MS in a patient in need thereof, and 2) instructions for administration of the AFP and the integrin antagonist to the patient.

In additional embodiments of the kit, the AFP (or a biologically active fragment, derivative, or analog thereof) and the integrin antagonist of the kit are present in the same composition or are present in the kit in separate compositions; the separate compositions can be admixed prior to administration to a patient or they can be administered separately to the patient. In other embodiments, the AFP (or a biologically active fragment, derivative, or analog thereof) and the integrin antagonist of the kit are formulated for the same route of administration or for two different routes of administration.

In several embodiments of all the aspects of the invention, the AFP (or a biologically active fragment, derivative, or analog thereof) is recombinant human AFP having an amino acid sequence that is substantially identical (e.g., at least 60% identical, preferably at least 65%, 70%, 75%, 80%, 85%, 90%, 91%, 92%, 93%, 94%, 95%, 96%, 97%, 98%, or 99% identical, or even 100% identical) to SEQ ID NO: 1. In other embodiments of all aspects of the invention, the AFP (or a biologically active fragment, derivative, or analog thereof) is non-glycosylated. In yet other embodiments of all aspects of the invention, the AFP may be formulated at a dosage in the range of 0.5 mg to 400 mg; the integrin antagonist may be formulated at a dosage in the range of 0.5 mg to 500 mg.

In other embodiments of all aspects of the invention, the integrin antagonist is an antibody (e.g., natalizumab), a blocking peptide, a nucleic acid inhibitor, or a small molecule inhibitor. In yet other embodiments, the integrin antagonist is an α4 integrin antagonist.

The AFP (or biologically active fragment, derivative, or analog thereof) and integrin antagonist can be formulated for or administered by one or more of a variety of routes of administration, including, but not limited to, intravenous, intramuscular, oral, by inhalation, parenteral, intraperitoneal, intraarterial, transdermal, sublingual, nasal, through use of suppositories, transbuccal, liposomal, adiposal, intraocular, subcutaneous, intrathecal, topical, or through local administration.

In additional embodiments of all aspects of the invention, the co-administration of the AFP (or a biologically active fragment, derivative, or analog thereof) and the integrin antagonist exhibits a therapeutic effect that is greater than that observed when the AFP (or biologically active fragment, derivative, or analog thereof) and the integrin antagonist are administered alone. In additional embodiments of all aspects of the invention, the AFP, the integrin antagonist, or both can be administered at a lower dosage than that normally required for achieving a therapeutic effect when either are administered alone (e.g., the AFP or the integrin antagonist can be administered at a dosage that is at least 10%, 15%, 20%, 25%, 30%, 35%, 40%, 45%, 50%, 60%, or 90% lower). In other embodiments of all aspects of the invention, co-administration of an AFP (or biologically active fragment, derivative, or analog thereof) and an integrin antagonist reduces the toxicity of the integrin antagonist (e.g., by at least 5%, 10%, 15%, 20%, 25%, 30%, 35%, 40%, 45%, 50%, 60%, 70% or more) relative to the toxicity of the integrin antagonist when administered at the same concentration in the absence of the AFP. In yet other embodiments of all aspects of the invention, the integrin antagonist can be administered in combination with the AFP at a dosage that is higher (e.g., at least 5%, 10%, 15%, 20%, 25%, 30%, 35%, 40%, 45%, 50%, 55%, 60%, 65%, 70%, 75%, 85%, 90%, 95%, 100% or more) than the normal dosage of an integrin antagonist, when administered for treating MS, without the toxicity normally expected or observed at the increased dose of the integrin antagonist when it is administered alone.

### Definitions

In this application, when therapeutic agents (e.g., an AFP and an integrin antagonist) are "administered coextensively," the administration time periods of the agents may completely overlap or at least in part overlap. When the therapeutic agents are "administered separately," the therapeutic agents are administered in time periods that do not overlap. In certain embodiments of separate administration, the therapeutic agents are administered in time periods that do not overlap, but are within the bioactive period for each respective agent, i.e., an earlier administered agent retains at least a substantial portion of its biological activity in the patient at the time when the latter administered agent is delivered. In other cases of separate adminstration, the agents are administered outside of their respective bioactive periods.

As used herein, the term "alpha-fetoprotein" or "AFP" refers to a polypeptide having an amino acid sequence that is substantially identical to the mature human AFP (SEQ ID NO: 1) or to a polypeptide that is encoded by a nucleic acid sequence that is substantially identical to the nucleic acid sequence that encodes human AFP (NCBI Accession No. NM_001134; SEQ ID NO: 2). Mature human AFP is a protein of 591 amino acids (see, SEQ ID NO:1), resulting from cleavage of a precursor of 609 amino acids (GenBank Accession No. NP_001125) to remove an 18-amino acid signal sequence. An AFP of this invention has an amino acid sequence that is substantially identical to SEQ ID NO: 1. The AFP is not limited to the full-length sequence; it also includes biologically active fragments of AFP. An AFP of the invention also includes any recombinant human AFP (whether or not having the same post-translational modifications as the naturally occurring version) and biologically active variants of human AFP (e.g., a non-glycosylated form of AFP; see, e.g., U.S. Patent No. 7,208,576, incorporated by reference herein).

An AFP of this invention may contain modifications of the amino acid sequence of SEQ ID NO: 1, including substitution (e.g., conservative substitution), deletion, or addition of one or more amino acid residues. For instance, a recombinant human AFP is described in U.S. Patent No. 7,208,576, incorporated herein by reference, which contains an asparagine to glutamine substitution at position 233 of SEQ ID NO: 1. The term "alpha-fetoprotein" also encompasses derivatives or analogs of AFP, such as those described herein.

An AFP of this invention exhibits one or more of the biological activities of the native human AFP, including, for example, the ability to bind to human leukocytes, the ability to suppress autoimmune reactions, and the ability to reduce the production of inflammatory cytokines. The leukocyte binding assay used for testing AFP activity is described herein and in, e.g., Parker et al., Protein Express. Purification 38:177-183, 2004. The autoimmune suppression activity for an AFP of this invention can be demonstrated by assaying the ability of the AFP to suppress human autologous mixed lymphocyte reactions (AMLR) or by assaying the ability of the AFP to suppress experimental autoimmune encephalomyelitis (EAE) in a mouse model using the methods described herein. The ability to reduce production of inflammatory cytokines can be assayed using the splenocyte assay described herein. A functional AFP of the invention demonstrates at least 40%, 50%, 60%, 70%, 80%, 85%, 90%, 95%, or 100% of the biological activity of the native human.

A biologically active fragment of AFP, for use in the compositions and methods of the invention, can be identified using one or more assays described herein (e.g., AMLR assays, AFP-binding to monocyte assays, experiments using the EAE mouse model, and splenocyte assays). A typical biologically active AFP fragment contains at least 5 contiguous amino acids of SEQ ID NO: 1, or at least 8 contiguous amino acids, preferably at least 10, 20, or 50 contiguous amino acids, more preferably at least 100 contiguous amino acids, and most preferably at least 200, 300, 400, or more contiguous amino acids in length. For instance, U.S. Patent No. 6,818,741 (herein incorporated by reference) discloses an 8-amino acid fragment of human AFP (amino acids 471-478; EMTPVNPG; SEQ ID NO: 3) as well as other related AFP fragments. An active AFP fragment of this invention may further contain amino acid substitutions, deletions, or additions at a limited number of positions, so long as the AFP fragment has at least 90% identity to its corresponding sequence within SEQ ID NO: 1. For sequence comparison purposes in this application, the corresponding sequence of SEQ ID NO: 1 is deemed to have the same number of amino acids as a given AFP fragment. For instance, a 34-mer AFP peptide corresponding to the 446-479 segment of SEQ ID NO: 1 (LSEDKLLACGEGAADIIIGHLCIRHEMTPVNPGV; SEQ ID NO: 4) may contain up to 3 amino acids altered from the 446-479 segment of SEQ ID NO: 1. One such example of sequence deviation in biologically active AFP fragments is found in U.S. Patent No. 5,707,963 (herein incorporated by reference), which discloses a 34-amino acid fragment of human AFP (SEQ ID NO: 4) with flexibility at two amino acid residues (amino acid 9 and 22 of SEQ ID NO: 4). Some other examples of AFP fragments include Domain I (amino acids 2-198 of mature human AFP; SEQ ID NO: 5), Domain II (amino acids 199-390 of mature human AFP; SEQ ID NO: 6), Domain III (amino acids 391-591 of mature human AFP; SEQ ID NO: 7), Domain I+II (amino acids 2-390 of mature human AFP; SEQ ID NO: 8), Domain II+III (amino acids 199-591 of mature human AFP; SEQ ID NO: 9), and human AFP Fragment I (amino acids 267-591 of mature human AFP; SEQ ID NO: 10).

In this application, the term "amino acid" refers to naturally occurring and synthetic amino acids, as well as amino acid analogs and amino acid mimetics that function in a manner similar to the naturally occurring amino acids. Naturally occurring amino acids are those encoded by the genetic code, as well as those amino acids that are later modified, e.g., hydroxyproline, γ-carboxyglutamate, and O-phosphoserine. Amino acid analogs refers to compounds that have the same basic chemical structure as a naturally occurring amino acid, i.e., an α carbon that is bound to a hydrogen, a carboxyl group, an amino group, and an R group, e.g., homoserine, norleucine, methionine sulfoxide, and methionine methyl sulfonium. Such analogs have modified R groups (e.g., norleucine) or modified peptide backbones (e.g., peptide mimetics, such as an AFP peptoid), but retain the same basic chemical structure as a naturally occurring amino acid. Amino acid mimetics are chemical compounds that have a structure that is different from the general chemical structure of an amino acid, but that are capable of functioning in a manner that is similar to a naturally occurring amino acid. An AFP of the invention can include naturally occurring or synthetic amino acids or amino acid mimetics.

As to amino acid sequences, one of skill will recognize that individual substitutions, deletions, or additions to a polypeptide sequence that alter, add, or delete a single amino acid or a small percentage of amino acids in the sequence constitute a "conservatively modified variant," when the alterations result in the substitution of one or more amino acids with other, chemically similar amino acids. Conservative substitution tables providing functionally similar amino acids are well known in the art. Such conservatively modified variants are in addition to and do not exclude polymorphic variants, interspecies homologs, and alleles of the invention.

The term "antibody" herein is used in the broadest sense and specifically covers monoclonal antibodies, polyclonal antibodies, multispecific antibodies (e.g., bi-specific antibodies) formed from at least two intact antibodies, and antibody fragments so long as they exhibit the desired biological activity. The term also incorporates antibody fragments (e.g., Fab, Fab', Fv fragments, diabodies, linear antibodies, and single chain antibody molecules). The term antibody also includes monoclonal antibodies that are chimeric, primatized, or humanized, and antibody mimics.

By "antibody mimic" is meant a protein or compound that can specifically bind to a target protein (e.g., α4 integrin). Examples include molecules comprising non-immunoglobulin protein scaffolds for the variable regions of antibodies. Antibody mimics may include proteins (e.g., Adnectins), RNA molecules, unnatural oligomers (e.g., protease inhibitors, benzodiazepines, purine derivatives, and β-turn mimics).

By the term "biologically active" is meant having one or more activities known to be associated with a naturally occurring or synthetic peptide, polypeptide, protein, antibody, compound, small molecule, or fragment, derivative, or analog thereof (e.g., an AFP or fragment, derivative, or analog thereof, or an integrin antagonist).

By "blocking peptide" is meant a peptide that antagonizes the activity of a target protein by binding to the target protein and preventing its interaction with other proteins or receptors or by binding to the receptor of a target protein and blocking the interaction between the receptor and the target protein. In an embodiment, a blocking peptide is one that antagonizes the activity of α4 integrin. Examples of blocking peptides are described herein.

By "CD11/CD18" is meant a family of three heterodimeric glycoproteins with one of three α subunits (i.e., CD11a, CD11b, and CD11c) that include a sequence substantially identical to SEQ ID NO: 25 (NCBI Accession No. P20701; Larson et al., J. Cell. Biol. 108:703-712, 1989), SEQ ID NO: 26 (NCBI Accession No. P11215; Corbi et al., J. Biol. Chem. 263: 12403-12411, 1988), or SEQ ID NO: 27 (NCBI Accession No. P20702; Corbi et al., EMBO J. 6:4023-4028, 1987), or having an mRNA nucleic acid sequence that includes a nucleic acid sequence substantially identical to SEQ ID NO: 29 (NCBI Accession No. NM_02209; Nishida et al., Immunity 25:583-594, 2006), SEQ ID NO: 30 (NCBI Accession No. NM_000632; Arnaout et al., Proc. Natl. Acad. Sci. U.S.A. 85:2776-2780, 1988), or SEQ ID NO: 31 (NCBI Accession No. NM_000887; Corbi et al., 1987, *supra*); and having a β subunit (CD18) which includes an amino acid sequence substantially identical to SEQ ID NO: 28 (NCBI Accession No. P05107; Kishimoto et al., Cell 48:681-690, 1987), or having a nucleic acid sequence that includes an mRNA nucleic acid sequence substantially identical to SEQ ID NO: 32 (NCBI Accession No. Y00057; Law et al., EMBO J. 6:915-919, 1987).

By "CD80" is meant a polypeptide that includes an amino acid sequence substantially identical to SEQ ID NO: 13 (NCBI Accession No. P33681; Freeman et al., J. Immunol. 143:2714-2722, 1989) or having an mRNA nucleic acid sequence comprising a nucleic acid sequence substantially identical to SEQ ID NO: 14 (NCBI Accession No. NM_005191; Freeman et al., *supra*). CD80 is also referred to in the art as "B-lymphocyte activation antigen," or "B7-1."

By "dosage form" is meant the physical form of a dose of an agent of the invention (e.g., an APF and integrin antagonist). Non-limiting examples of dosage forms include a tablet, capsule, gel, cream, paste, liquid, suspension or emulsion, and spray. The dosage form can be chosen based on the intended route of administration. Alternatively, the route of administration may be dictated by the dosage form of an agent. A dosage form according to the present invention includes those that may be administered intravenously, intramuscularly, orally, parenterally, intraperitoneally, intraarterially, transdermally, sublingually, nasally, transbuccally, liposomally, adiposally, intraocularly, subcutaneously, intrathecally, topically, or through local administration.

By "hyaluronate receptor" is meant a polypeptide that includes an amino acid sequence substantially identical to SEQ ID NO: 19 (NCBI Accession No. P16070; Screaton et al., Proc. Natl. Acad. Sci. U.S.A. 89:12160-12164, 1992) or having an mRNA nucleic acid sequence that includes a nucleic acid sequence substantially identical to SEQ ID NO: 20 (NCBI Accession No. AJ251595; Gunthert, Curr. Top. Microbiol. Immunol. 184:47-63, 1993). Hyaluronate receptor is also referred to in the art as "CD44," "phagocytic glycoprotein-1" or "PGP-1," "lymphocyte antigen-24" or "Ly-24," or "extracellular matrix receptor III" or "ECMR III."

As used here, "integrin antagonist" refers to an agent that suppresses or inhibits the biological activity of an integrin molecule, such as the α4 subunit of an integrin molecule. The agent may act directly or indirectly on the α4 integrin subunit (NCBI Accession No. P13612; SEQ ID NO: 11; Takada et al., EMBO J. 8:1361-1368, 1989; or SEQ ID NO: 34) by inhibiting the activity or expression of the α4 integrin subunit, or may act on the target to which the intact integrin containing an α4 subunit binds. For example, an antibody or blocking peptide that binds to vascular cell adhesion molecule-1 (VCAM-1), thus preventing the binding of α4β1 integrin to VCAM-1 is considered an integrin antagonist for purposes of the present invention. Non-limiting exemplary integrin antagonists suitable for use with the present invention may include proteins, blocking peptides, antibodies, such as natalizumab, small molecule inhibitors, and nucleic acid inhibitors. Examples of nucleic acid inhibitors used as integrin antagonists are those that include a sequence which is complimentary to a sequence substantially identical to all or part of the mRNA sequence of human α4 integrin (NCBI Accession No. NM_000885; SEQ ID NO: 12; Takada et al. *supra*; or SEQ ID NO: 35). For example, a sequence that is complementary to nucleotides 1-25 of SEQ ID NO: 12 or SEQ ID NO: 35.

Examples of α4 integrin antagonists include, but are not limited to, natalizumab (Elan/Biogen Idec; see, e.g., U.S. Patent Nos. 5,840,299; 6,033,665; 6,602,503; 5,168,062; 5,385,839; and 5,730,978), oMEPUPA-V (Biogen; U.S. Patent No. 6,495,525; incorporated by reference herein), CDP-323 (Celltech); firategrast (SB-68399; GlaxoSmithKline); TR-9109 (Pfizer); ISIS-107248 (Antisense Therapeutics); R-1295 (Roche); and TBC-4746 (Schering-Plough).

Additional non-limiting examples of α4 integrin antagonists include the small molecules described in U.S. Patent Nos. 5,821,231; 5,869,448; 5,936,065; 6,265,572; 6,288,267; 6,365,619; 6,423,728; 6,426,348; 6,458,844; 6.479,666; 6,482,849; 6,596,752; 6,667,331; 6,668,527; 6,685,617; 6,903,128; and 7,015,216 (each herein incorporated by reference); in U.S. Patent Application Publication Nos. 2002/0049236; 2003/0004196; 2003/0018016; 2003/0078249; 2003/0083267; 2003/0100585; 2004/0039040; 2004/0053907; 2004/0087574; 2004/0102496; 2004/0132809; 2004/0229858; 2006/0014966; 2006/0030553; 2006/0166866; 2006/0166961; 2006/0241132; 2007/0054909; and 2007/0232601 (each herein incorporated by reference); in European Patent Nos. EP 0842943; EP 0842944; EP 0842945; EP 0903353; and EP 0918059; and in PCT Publication Nos. WO 95/15973; WO 96/06108; WO 96/40781; WO 98/04247; WO 98/04913; WO 98/42656; WO 98/53814; WO 98/53817; WO 98/53818; WO 98/54207; WO 98/58902; WO 99/06390; WO 99/06431; WO 99/06432; WO 99/06433; WO 99/06434; WO 99/06435; WO 99/06436; WO 99/06437; WO 99/10312; WO 99/10313; WO 99/20272; WO 99/23063; WO 99/24398; WO 99/25685; WO 99/26615; WO 99/26921; WO 99/26922; WO 99/26923; WO 99/35163; WO 99/36393; WO 99/37605; WO 99/37618; WO 99/43642; WO 01/42215; and WO 02/28830; all of which are incorporated by reference herein.

Additional examples of α4 integrin antagonists include the phenylalanine derivatives described in: U.S. Patent Nos. 6,197,794; 6,229,011; 6,329,372; 6,388,084; 6,348,463; 6,362,204; 6,380,387; 6,445,550; 6,806,365; 6,835,738; 6,855,706; 6,872,719; 6,878,718; 6,911,451; 6,916,933; 7,105,520; 7,153,963; 7,160,874; 7,193,108; 7,250,516; and 7,291,645 (each herein incorporated by reference). Additional amino acid derivatives that are α4 integrin antagonists include those described in, e.g., U.S. Patent Application Publication Nos. 2004/0229859 and 2006/0211630 (herein incorporated by reference), and PCT Publication Nos. WO 01/36376; WO 01/47868; and WO 01/70670; all of which are incorporated by reference herein.

Other examples of α4 integrin antagonists include the peptides, and the peptide and semi-peptide compounds described in, e.g., PCT Publication Nos. WO 94/15958; WO 95/15973; WO 96/00581; WO 96/06108; WO 96/22966 (Leu-Asp-Val tripeptide; Biogen, Inc.); WO 97/02289; WO 97/03094; and WO 97/49731. An additional example of an α4 integrin antagonist is the pegylated molecule described in U.S. Patent Application Publication No. 2007/066533 (herein incorporated by reference).

Examples of antibodies that are α4 integrin antagonists include those described in, e.g., PCT Publication Nos. WO 93/13798; WO 93/15764; WO 94/16094; and WO 95/19790. Additional examples of α4 integrin antagonists are described herein.

By "leukocyte function antigen-1" or "LFA-1" is meant a polypeptide that includes an α subunit having an amino acid sequence substantially identical to SEQ ID NO: 21 (NCBI Accession No. P20701; Larsen et al., J. Cell. Biol. 108:703-712, 1989) and a β subunit having an amino acid sequence substantially identical to SEQ ID NO: 22 (NCBI Accession No. P05107; Kishimoto et al., Cell 48:681-690, 1987); or having an mRNA nucleic acid sequence that includes a nucleic acid sequence substantially identical to SEQ ID NO: 23 (NCBI Accession No. Y00796; Larsen et al., *supra*) or SEQ ID NO: 24 (NCBI Accession No. Y00057; Law et al., EMBO J 6:915-919, 1987).

By "nucleic acid inhibitor" is meant any nucleic acid sequence (DNA or RNA) or peptide-nucleic acid sequence that contains a sequence complimentary to a nucleic acid sequence that is substantially identical to all or part of the mRNA of a targeted protein (e.g., for α4 integrin, a sequence complimentary to SEQ ID NO: 12 or SEQ ID NO: 35; for CD80, a sequence complimentary to SEQ ID NO: 14; for P-selectin, a sequence complimentary to SEQ ID NO: 16; for sphingosine-1-phosphate receptor-1, a sequence complimentary to SEQ ID NO: 18; for hyaluronate receptor, a sequence complimentary to SEQ ID NO: 20; for LFA-1, a sequence complimentary to SEQ ID NOS: 23 or 24; and for CD11/CD18, a sequence complimentary to SEQ ID NOS: 29, 30, 31, or 32), which, when administered to a cell or subject, results in decreased activity or expression of the target protein (e.g., α4 integrin, CD80, P-selectin, sphingosine-1-phosphate receptor-1, hyaluronate receptor, LFA-1, CD11/18, CD20, CD86, ICOS ligand, CCR2, CXCR3, CCR5, CD40, CD154, CD28, IL-23, IL-17, or IL-6.) relative to a cell or subject not administered the nucleic acid inhibitor. Examples of nucleic acid inhibitors include RNAi, antisense RNA, siRNA, miRNA, and peptide-nucleic acids. One example of a nucleic acid inhibitor is a nucleic acid sequence that results in a decrease in the activity or expression of α4 integrin.

Examples of antisense nucleic acid molecules include RNA or DNA molecules having a sequence that is complementary to the mRNA nucleic acid sequences of target proteins; the sequence of an antisense nucleic acid molecule of the invention has a length of at least 10, 20, 25, 30, 40, 50, 60, 70, 80, 90, or 100, or 100 or more nucleotides. Other examples of antisense nucleic acid molecules are interfering RNA or RNAi molecules (e.g., small interfering RNAs or siRNAs). RNAi molecules contain sequences that are complementary to the mRNA nucleic acid sequences of target proteins (as described above), and may include heterologous sequences which facilitate hairpin formation. RNAi molecules may be at least 10, 20, 25, 30, 40, 50, or even greater than 50 nucleotides in length; preferably the RNAi molecule is 21 or 25 nucleotides in length. Typically, RNAi molecules have at least 25 nucleotides and are complementary to any 25 nucleotides of the target protein mRNA sequence (e.g., the mRNA sequence of α4 integrin set forth in SEQ ID NO: 12 or SEQ ID NO: 35). Additional examples of RNAi molecules are those that are complementary to nucleotides 1-25, 50-75, 50-100, 50-150, 50-1000, or 50-2000 of SEQ ID NO: 12 or SEQ ID NO: 35.

By "P-selectin" is meant a polypeptide having an amino acid sequence substantially identical to SEQ ID NO: 15 (NCBI Accession No. NP_002966; Johnston et al., Cell 56:1033-1044, 1989) or having an mRNA nucleic acid sequence that includes a nucleic acid sequence substantially identical to SEQ ID NO: 16 (NCBI Accession No. NM_003005; Johnston et al., *supra*). P-selectin is also referred to in the art as "CD62P," "Granule Membrane Protein-140" or "GMP-140," and "Platelet Activation-Dependent Granule to External Membrane Protein" or "PADGEM."

By "small molecule inhibitor" is meant any small molecule which is identified as an antagonist of a target protein (e.g., α4 integrin, CD80, P-selectin, sphingosine-1-phosphate receptor-1, hyaluronate receptor, LFA-1, CD11/18, CD20, CD86, ICOS ligand, CCR2, CXCR3, CCR5, CD40, CD154, CD28, IL-23, IL-17, or IL-6) using screening or biological assays (e.g., ligand binding assays, protein or receptor activity assays, and other assays as known in the art). The compound may be identified by screening commercially available chemical or small molecule libraries.

By "sphingosine-1-phosphate receptor-1" or "S1P1" is meant a polypeptide having an amino acid sequence substantially identical to SEQ ID NO: 17 (NCBI Accession No. P21453; Hla and Maciag, J. Biol. Chem. 265:9308-9313, 1990) or having an mRNA nucleic acid sequence that includes a nucleic acid sequence substantially identical to SEQ ID NO: 18 (NCBI Accession No. BC018650; Strausberg et al., *supra*). Sphingosine-1-phosphate receptor-1 is also referred to in the art as "endothelial differentiation, sphingolipid G-protein coupled receptor-1" or "Edg1."

The term "substantial identity" or "substantially identical," when used in the context of comparing a polynucleotide or polypeptide sequence to a reference sequence, refers to the fact that the polynucleotide or polypeptide sequence is the same as the reference sequence or has a specified percentage of nucleotides or amino acid residues that are the same at the corresponding locations within the reference sequence when the two sequences are optimally aligned. For instance, an amino acid sequence that is "substantially identical" to a reference sequence has at least about 60% identity, preferably at least 65%, 70%, 75%, 80%, 85%, 90%, 91%, 92%, 93%, 94%, 95%, 96%, 97%, 98%, or 99% identity, or higher percentage identity (up to 100%) to a reference sequence (e.g., the mature human AFP amino acid sequence as set forth in SEQ ID NO: 1, the human AFP mRNA nucleic acid sequence set forth in SEQ ID NO: 2, or a pre-determined segment of SEQ ID NOS: 1 or 2), when compared and aligned for maximum correspondence over the full length of the reference sequence as measured using BLAST or BLAST 2.0 sequence comparison algorithms with default parameters, or by manual alignment and visual inspection (see, e.g., NCBI web site).

A "therapeutically effective amount" of a therapeutic agent (e.g., an AFP or an integrin antagonist) is an amount of the agent that is sufficient to treat or reduce one or more symptoms of MS (e.g., tingling, numbness, tremors, loss of balance, weakness in one or more limbs, blurred or double vision, slurred speech, swallowing problems, paralysis, lack of coordination, cognitive difficulties, fatigue, muscle spasms, dizziness, breathing problems, or seizures) or the severity of one or more symptoms of MS (e.g., by at least about 5%, 10%, 15%, 20%, 25%, 30%, 40%, 50%, 60%, 70%, or 80% relative to an untreated control patient). The treatment of MS or reduction in one or more of the symptoms of MS (or their severity) can be determined by using one of several methods known in the art (see, e.g., the Expanded Disability Status Scale (EDSS), Kurtzke, Neurology 33:1444-1452, 1983; and the Multiple Sclerosis Severity Score (MSSS), Roxburgh et al., Neurology 64:1144-1151, 2005). Such amount may vary depending on the effect to be achieved. For instance, a "therapeutically effective amount" of an integrin antagonist for treating MS when used in combination with AFP (or a biologically fragment thereof) may be different from the "therapeutically effective amount" of an integrin antagonist when used alone for treating MS. In different embodiments, the therapeutic effect is to reduce the symptoms (e.g., muscle weakness and demylination of nerves) or progression of MS in a patient.

By "treating" is meant the reduction (e.g., by at least 10%, 15%, 20%, 25%, 30%, 35%, 40%, 45%, 50%, 60%, 70%, 80%, 90%, 95%, 99%, or even 100%) in the progression, severity, or frequency of one or more symptoms of MS (e.g., tingling, numbness, tremors, loss of balance, weakness in one or more limbs, blurred or double vision, slurred speech, swallowing problems, paralysis, lack of coordination, cognitive difficulties (e.g., decreased memory and concentration), fatigue, muscle spasms, dizziness, breathing problems, and seizures) or the prevention or decrease (e.g., by at least 10%, 15%, 20%, 25%, 30%, 35%, 40%, 45%, 50%, 60%, 70%, 80%, 90%, 95%, 99%, or even 100%) in the progression of MS in a human patient (e.g., demyelination of nerves and the frequency or severity of one or more symptoms of MS).

### Brief Description of the Figures

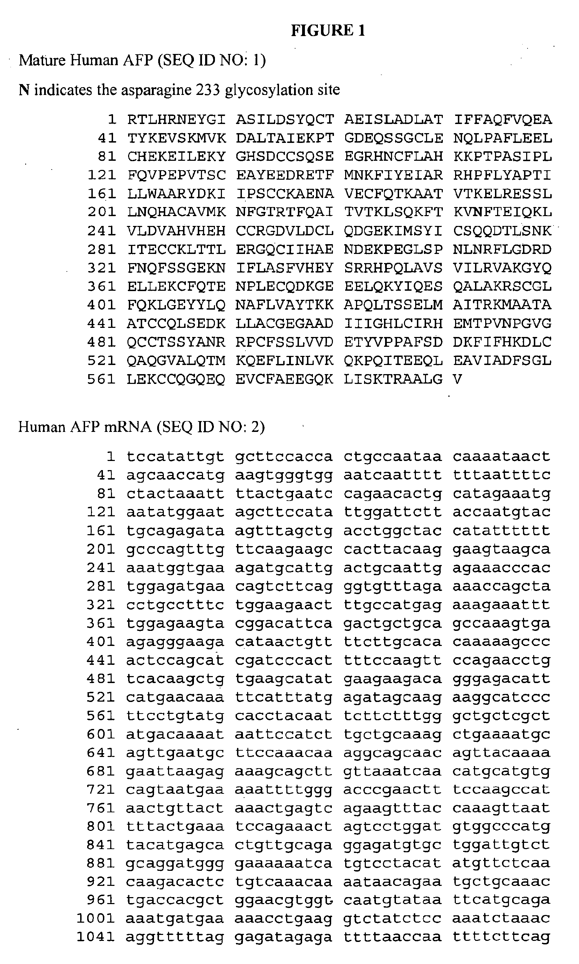

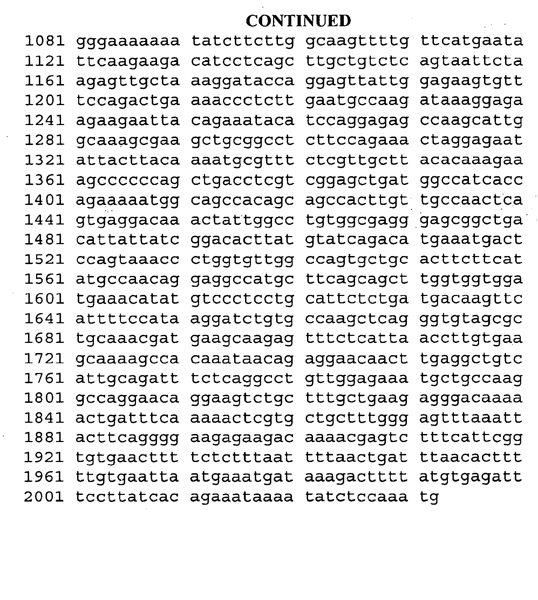

**Figure 1** shows the amino acid sequence of mature human AFP (SEQ ID NO: 1) and the mRNA nucleic acid sequence of human AFP (SEQ ID NO: 2). N indicates the asparagine 233 glycosylation site in the mature human AFP amino acid sequence.
**Figure 2** shows the amino acid sequences of biologically active fragments of AFP including amino acids 2-198 (Domain I; SEQ ID NO: 5), amino acids 199-390 (Domain II; SEQ ID NO: 6), amino acids 391-591 (Domain III; SEQ ID NO: 7), amino acids 2-390 (Domains I+II; SEQ ID NO: 8), amino acids 199-591 (Domains II+III; SEQ ID NO: 9), and amino acids 261-591 of mature human AFP (Human AFP Fragment 1; SEQ ID NO: 10).

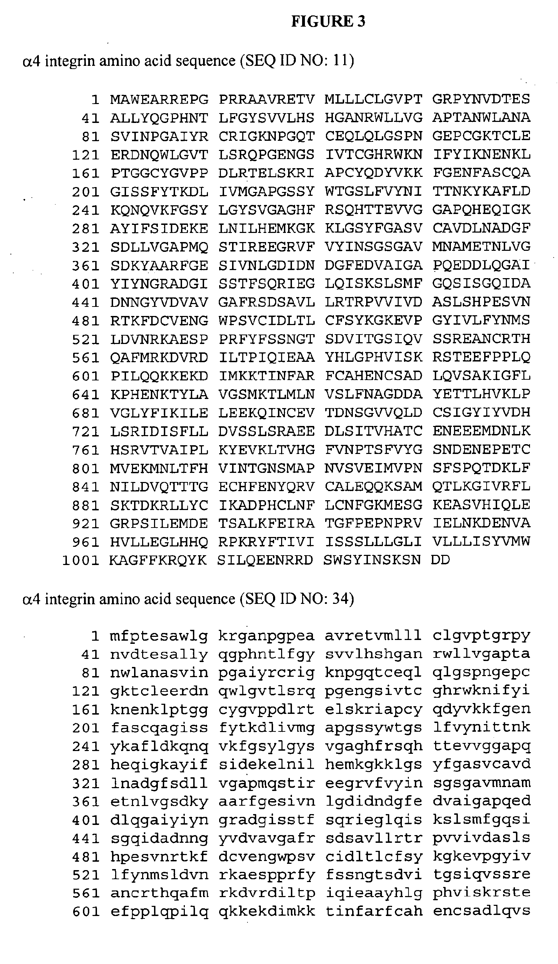

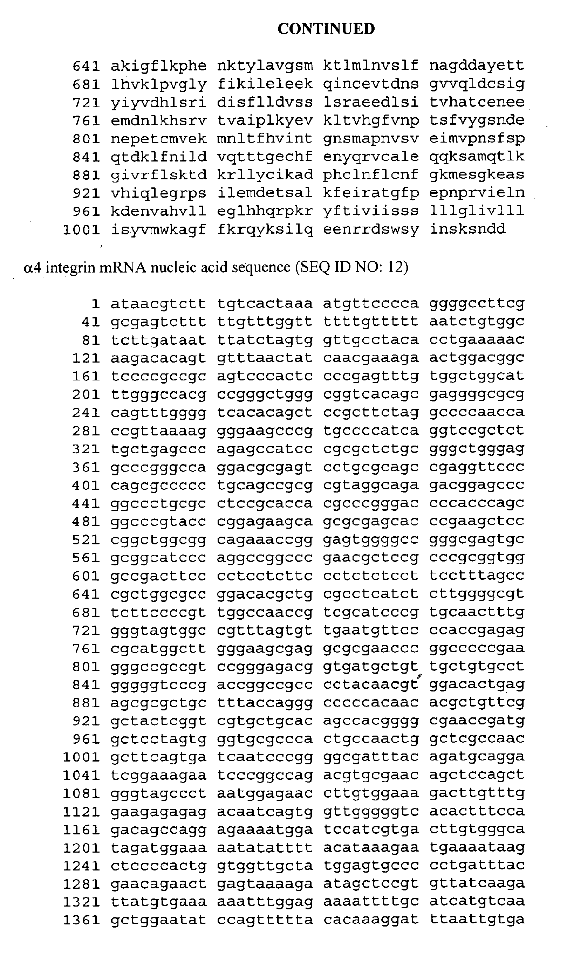

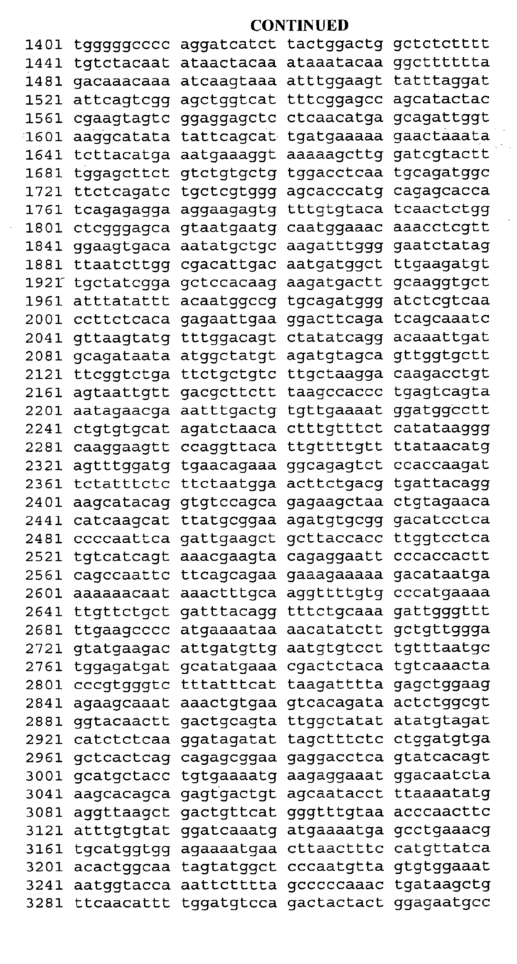

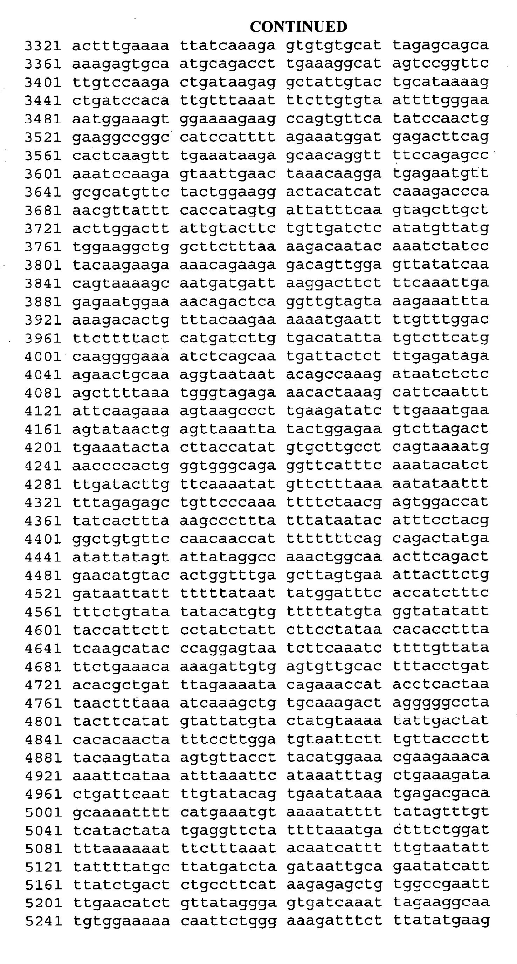

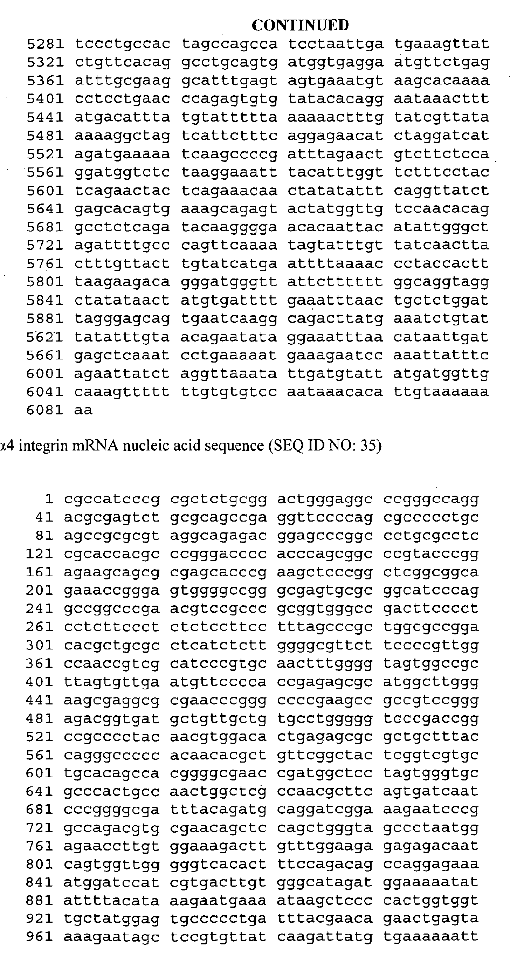

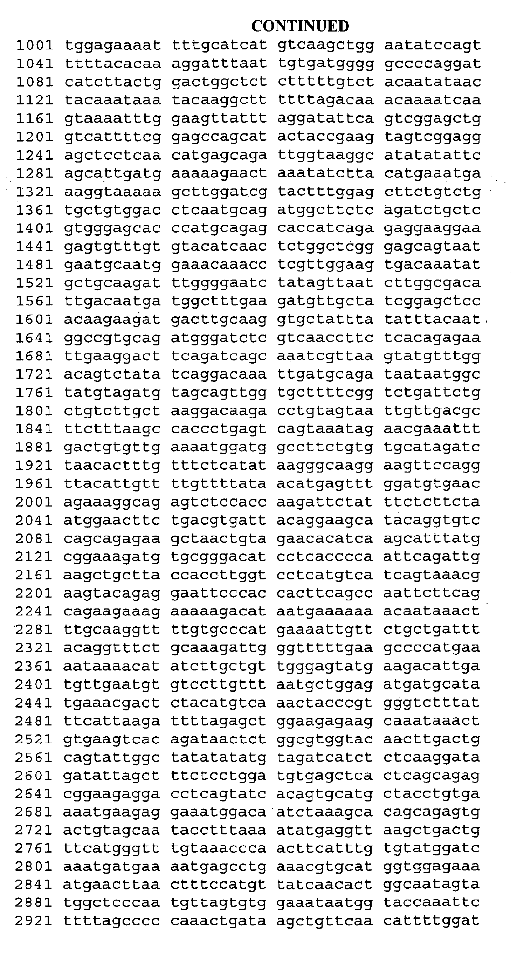

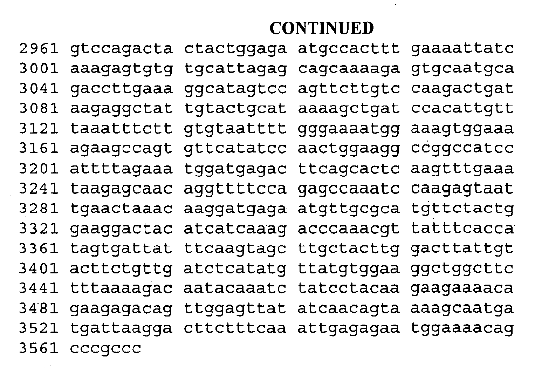

**Figure 3** shows two different amino acid sequences for α4 integrin protein (SEQ ID NOS: 11 and 34) and two different nucleic acid sequences for α4 integrin mRNA (SEQ ID NOS: 12 and 35).

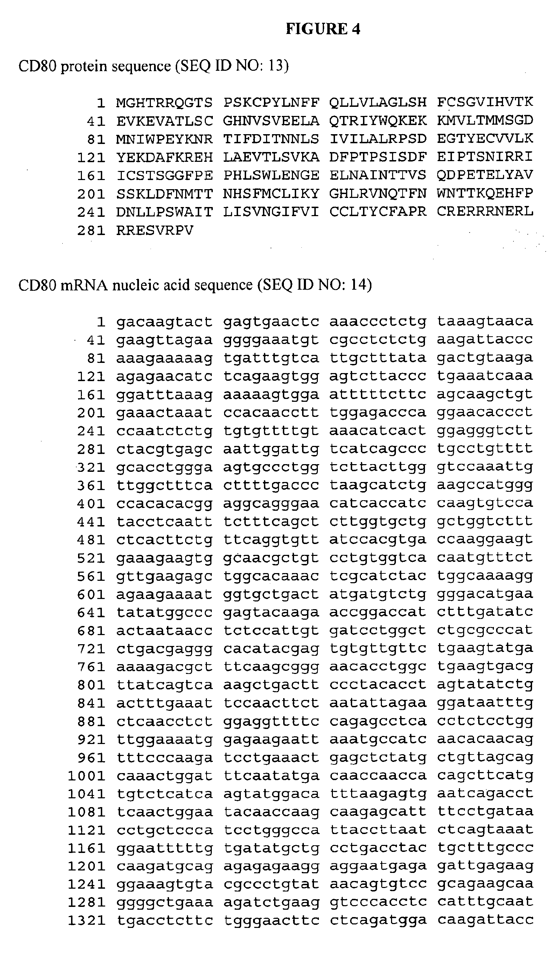

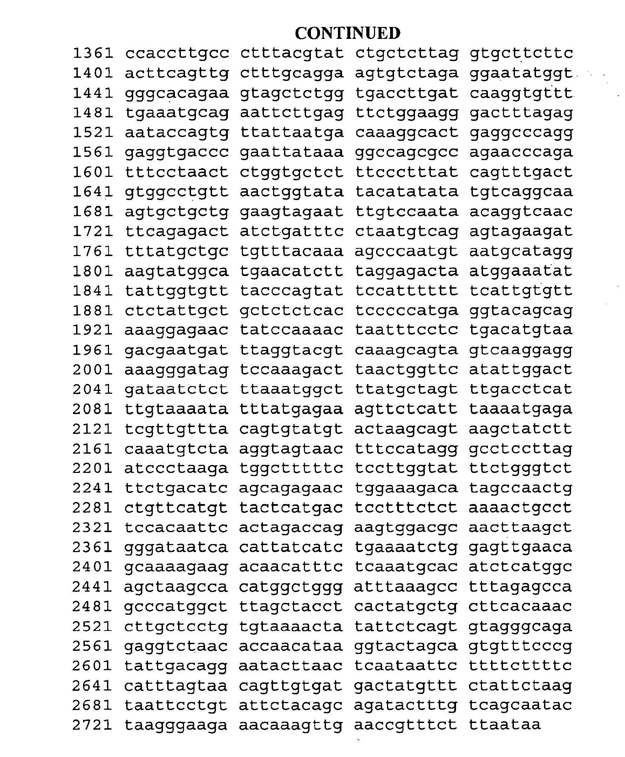

**Figure 4** shows the amino acid sequence (SEQ ID NO: 13) and the nucleic acid sequence (SEQ ID NO: 14) of CD80 protein and mRNA, respectively.

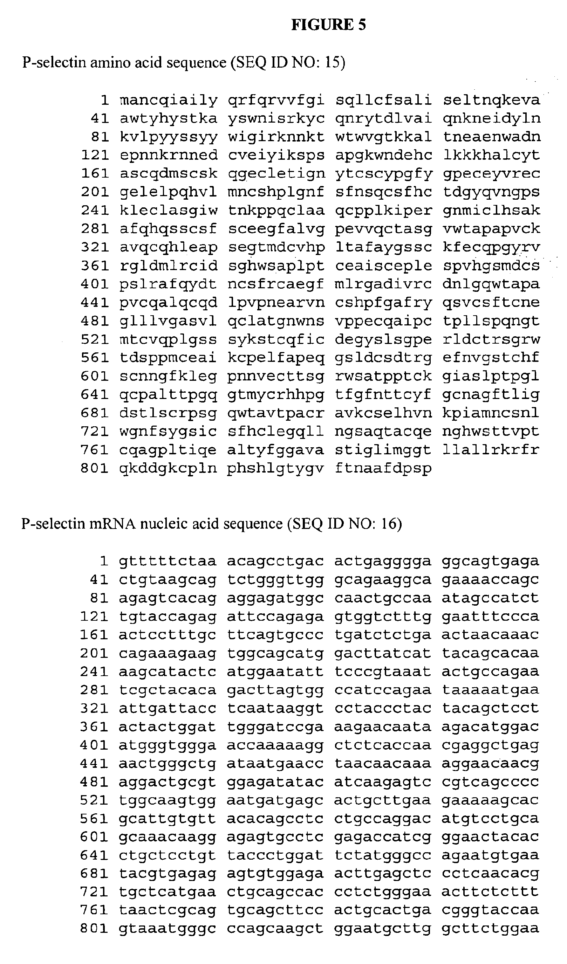

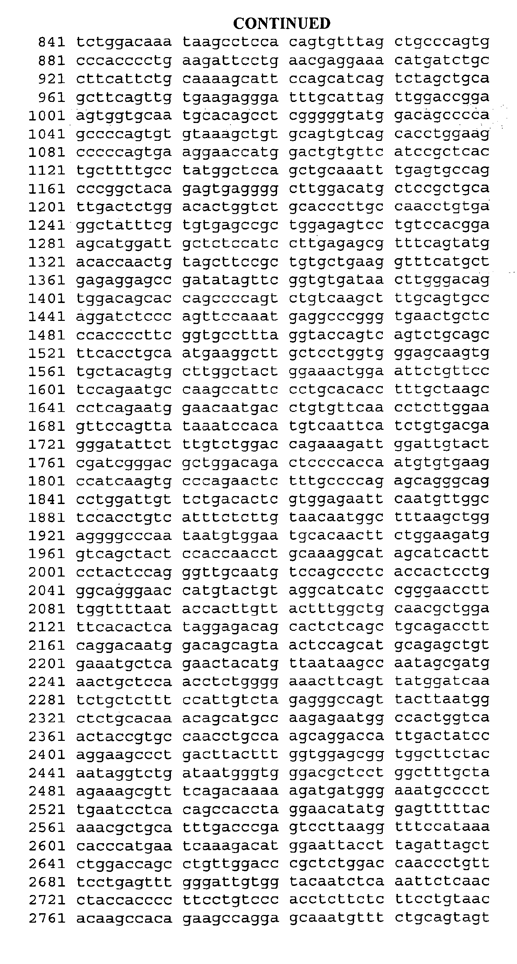

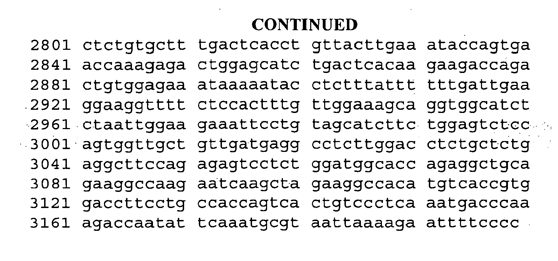

**Figure 5** shows the amino acid sequence (SEQ ID NO: 15) and the nucleic acid sequence (SEQ ID NO: 16) of P-selectin protein and mRNA, respectively.

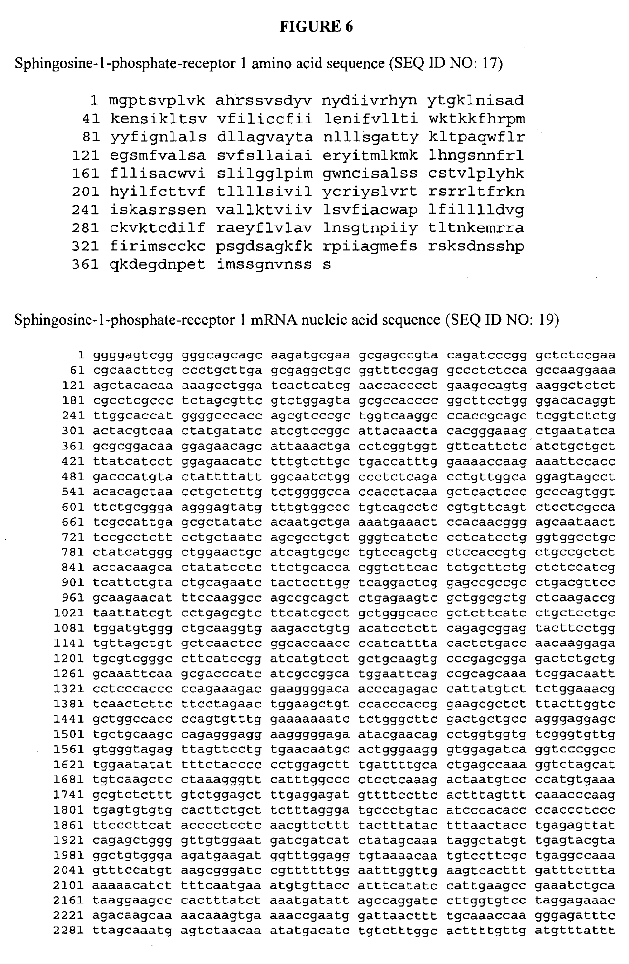

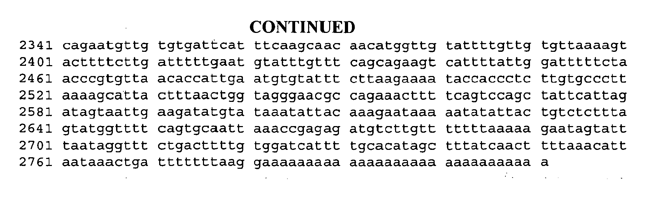

**Figure 6** shows the amino acid sequence (SEQ ID NO: 17) and the nucleic acid sequence (SEQ ID NO: 18) of sphingosine-1-phosphate receptor-1 protein and mRNA, respectively.

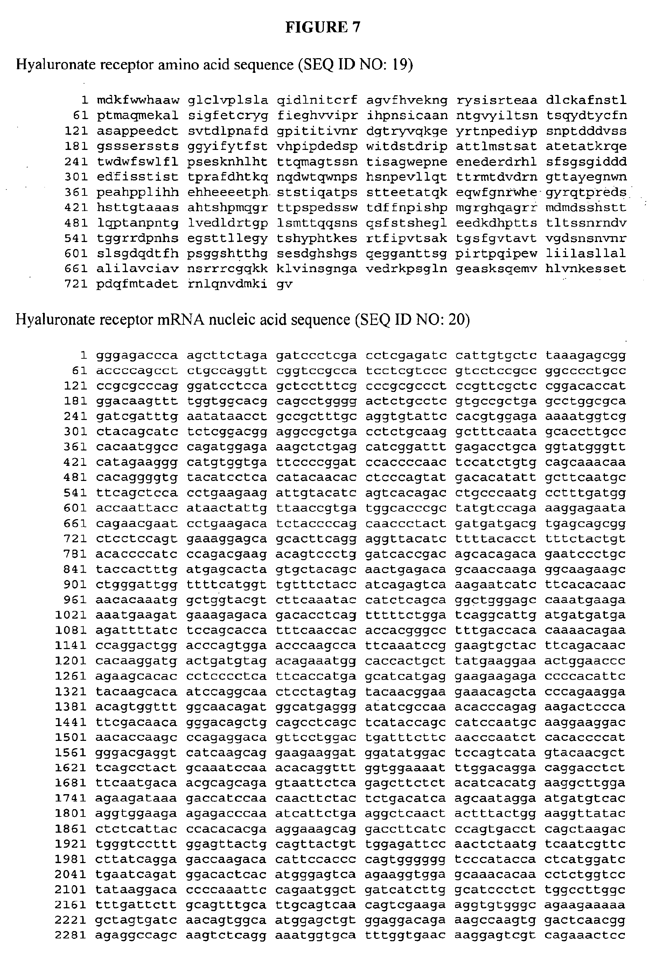

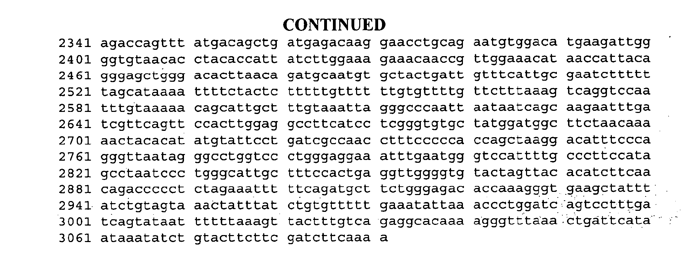

**Figure 7** shows the amino acid sequence (SEQ ID NO: 19) and the nucleic acid sequence (SEQ ID NO: 20) of hyaluronate receptor protein and mRNA, respectively.

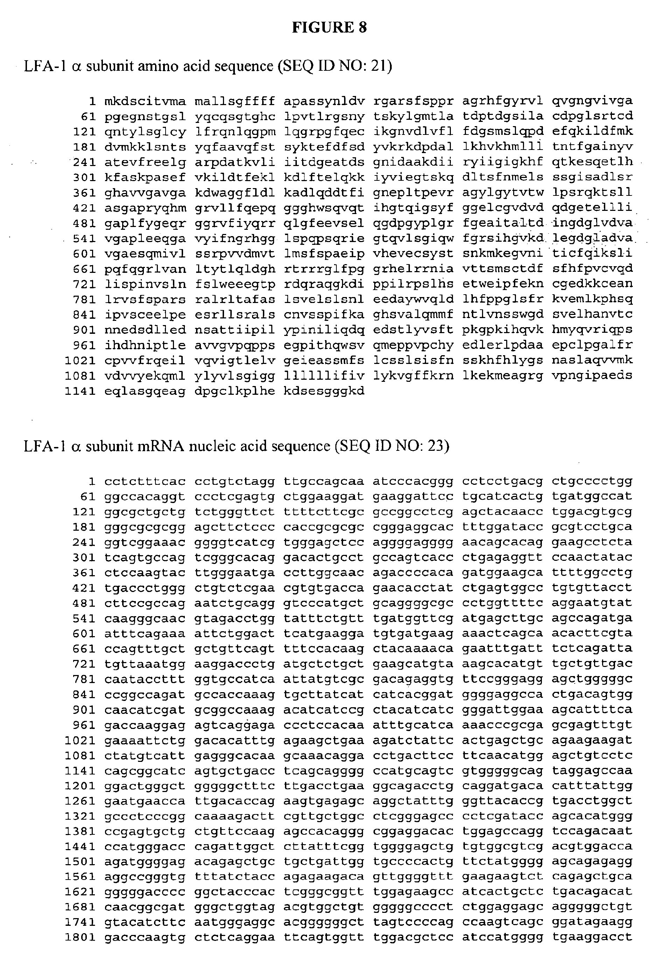

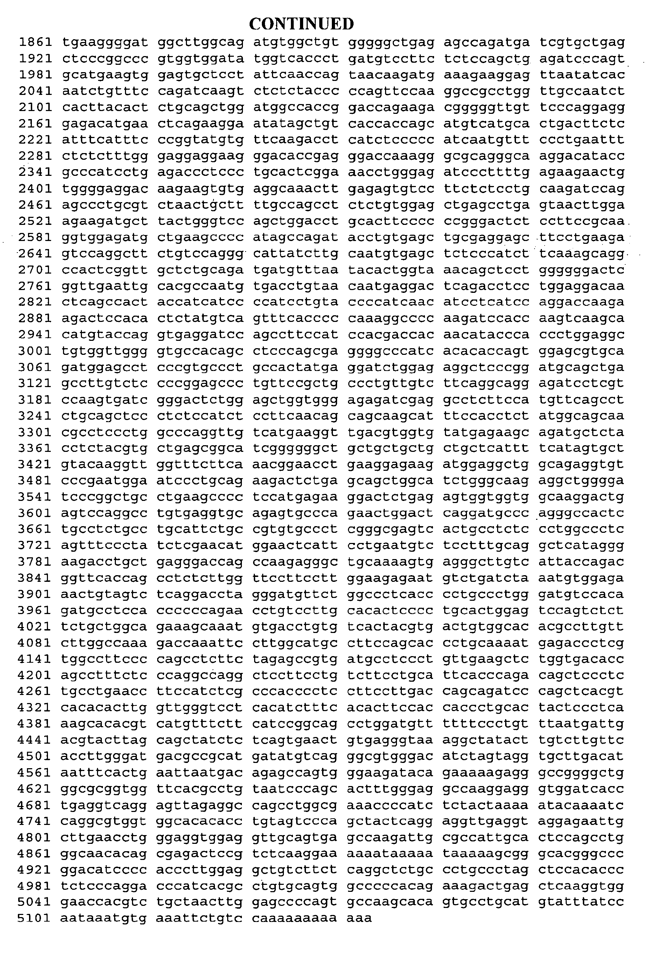

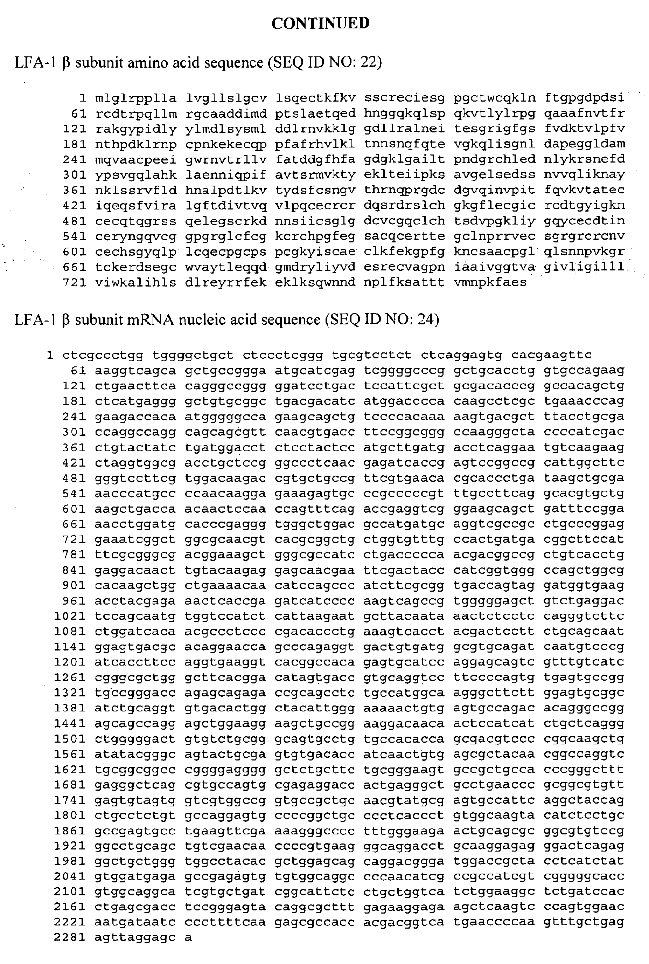

**Figure 8** shows the amino acid sequence (SEQ ID NO: 21) and the nucleic acid sequence (SEQ ID NO: 23) of LFA-1 a subunit protein and mRNA, respectively; and the amino acid sequence (SEQ ID NO: 22) and the nucleic acid sequence (SEQ ID NO: 24) of LFA-1 β subunit protein and mRNA, respectively.

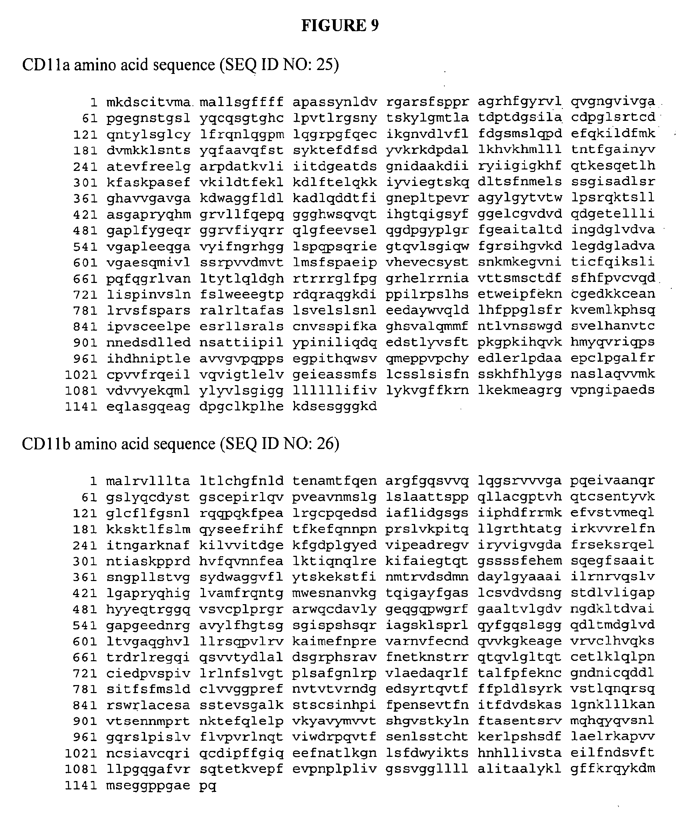

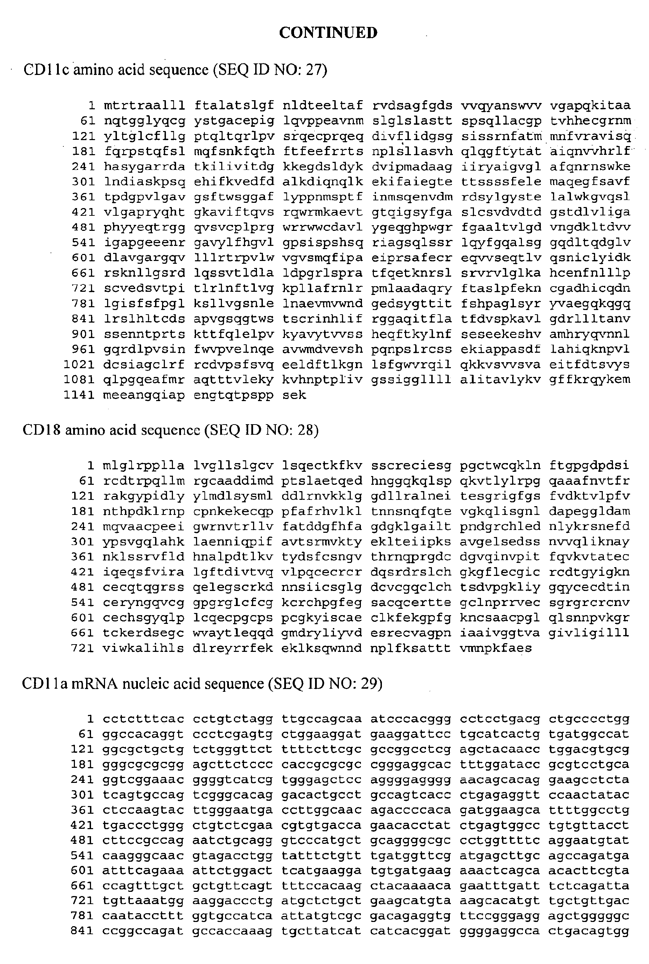

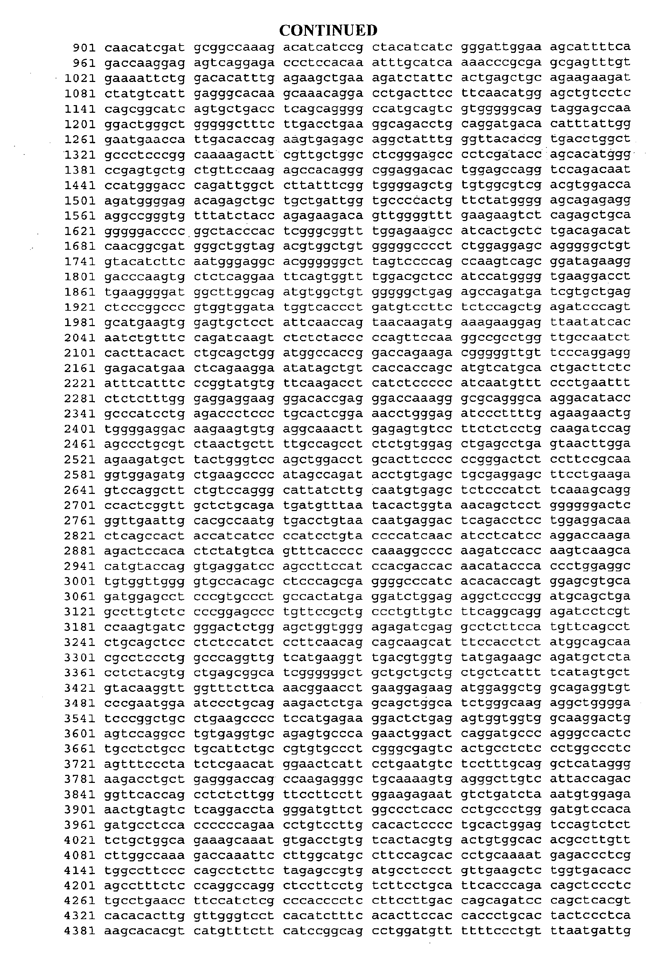

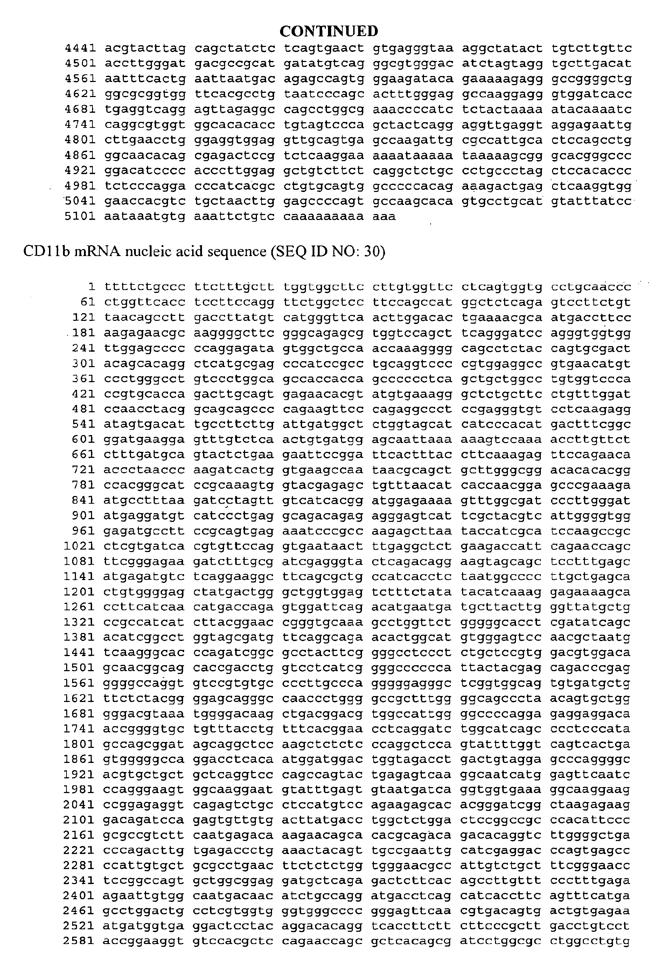

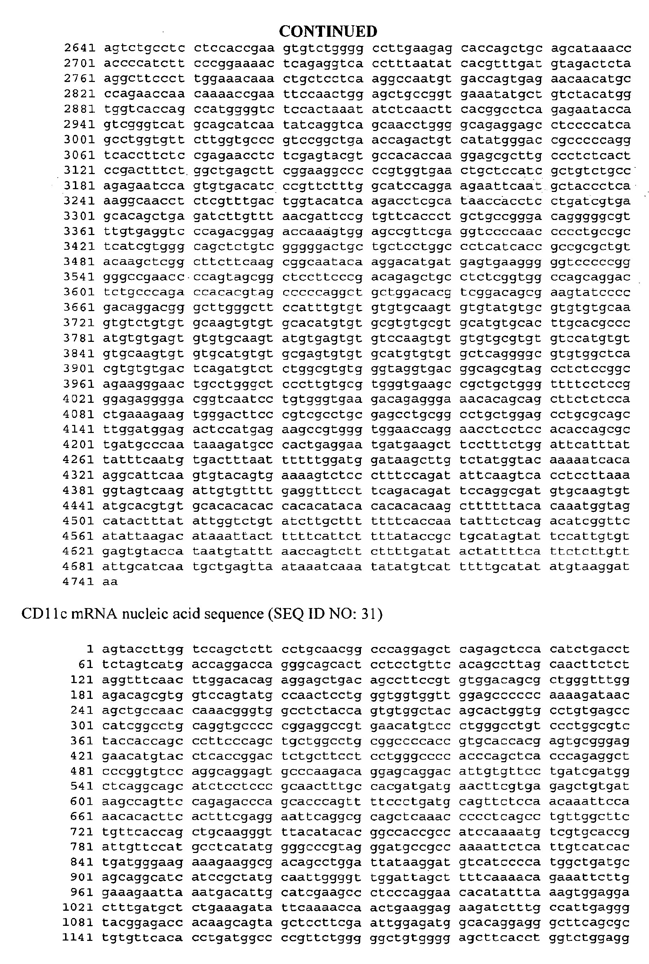

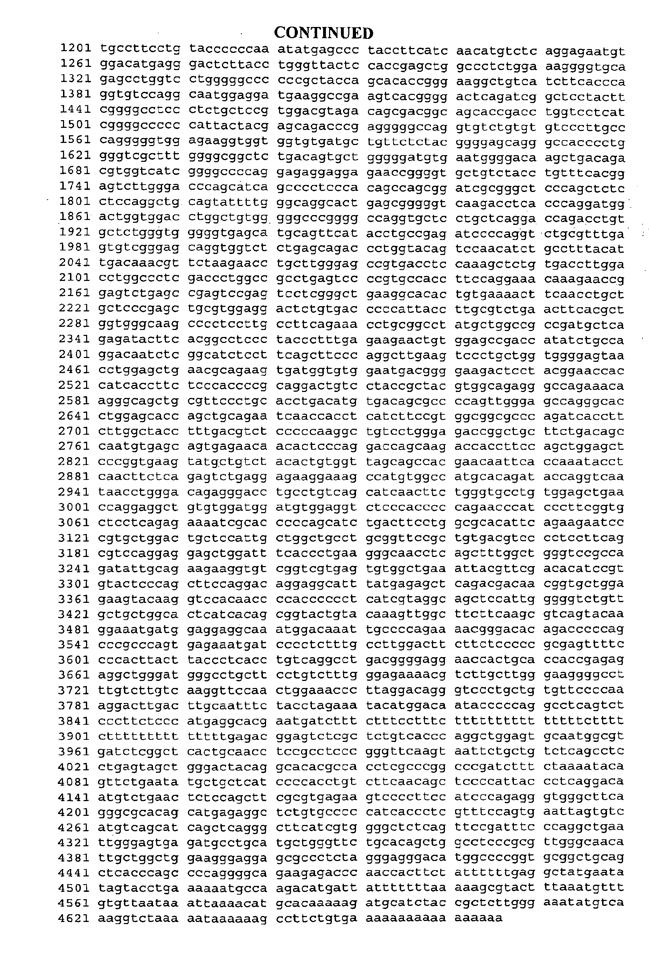

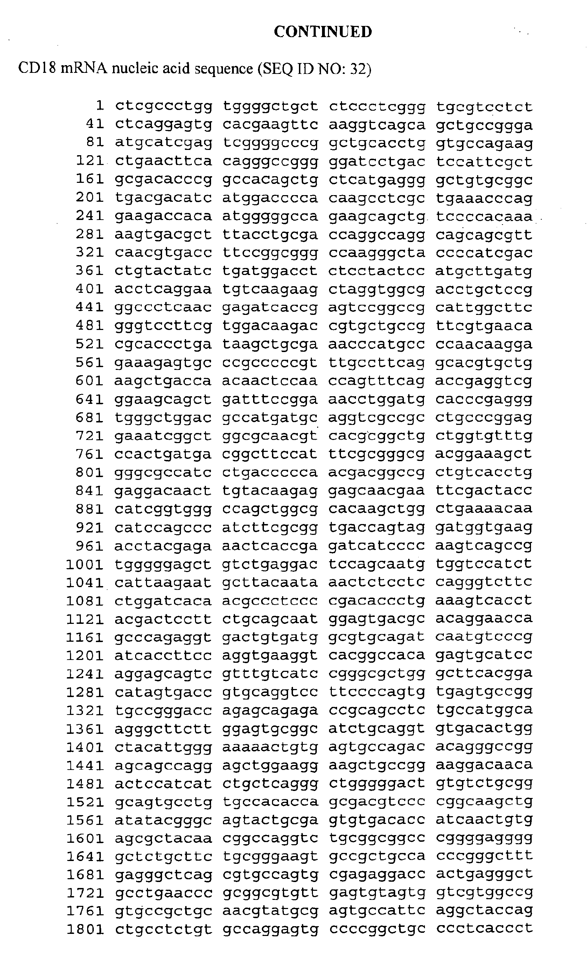

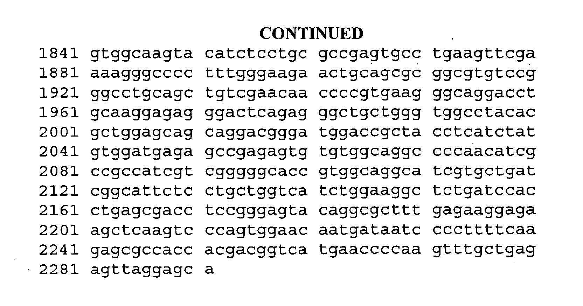

**Figure 9** shows the amino acid sequences of CD11a (SEQ ID NO: 25), CD11b (SEQ ID NO: 26), CD11c (SEQ ID NO: 27), and CD18 (SEQ ID NO: 28); and the nucleic acid sequences of CD11a (SEQ ID NO: 29), CD11b (SEQ ID NO: 30), CD11c (SEQ ID NO: 31), and CD18 (SEQ ID NO: 32) mRNA.

### Detailed Description

The invention features a combination therapy for treating MS that involves the co-administration of an integrin antagonist and an AFP (or a biologically active fragment thereof), each in a therapeutically effective amount, to an MS patient in need thereof.

The invention also features a pharmaceutical composition that includes both an integrin antagonist and an AFP (or a biologically active fragment thereof), each in a therapeutically effective amount for treating, preventing, or reducing one or more of the symptoms of or the progression of, MS. Such a composition optionally contains one or more pharmaceutically acceptable excipients and is formulated to be administered intravenously, intramuscularly, orally, by inhalation, parenterally, intraperitoneally, intraarterially, transdermally, sublingually, nasally, through use of suppositories, transbuccally, liposomally, adiposally, intraocularly, subcutaneously, intrathecally, topically, or through local administration.

The invention also features a kit for treating, preventing, or reducing one or more of the symptoms of or the progression of, MS, which includes a therapeutically effective amount of an integrin antagonist and AFP (or a biologically active fragment thereof), along with proper instructions for using the kit.

The integrin antagonist may be an antibody, a blocking peptide, a nucleic acid inhibitor, or a small molecule inhibitor. Examples of integrin antagonists are described herein. The AFP can be full length AFP, a biologically active fragment, derivative, or analog thereof, or a mutein thereof having one or more amino acid substitutions, deletions, or additions. Examples of AFP agents of the invention are described herein. The AFP and the integrin antagonist may be formulated for or administered in a single dosage form or they may be formulated or administered in different dosage forms. The AFP and integrin antagonist of the invention can be administered coextensively or separately. In addition, the AFP or integrin antagonist may be administered one or more times hourly, daily, weekly, biweekly, or monthly.

An antagonist to CD80, P-selectin, sphingosine-1-phosphate receptor-1, hyaluronate receptor, LFA-1, CD11/CD18, CD20, CD86, ICOS ligand, CCR2, CXCR3, CCR5, CD40, CD154, CD28, IL-23, IL-17, or IL-6 may also be administered in the methods and compositions of the invention.

### Diagnosis and Monitoring of Multiple Sclerosis

MS can be diagnosed by observing one or more symptoms in a patient. Symptoms of MS may be single or multiple and may range from mild to severe in intensity and short to long in duration. Complete or partial remission from symptoms occurs early in about 70% of MS patients. Visual disturbances often are the first symptoms of MS, but they usually subside. A patient may notice blurred or double vision, red-green distortion, or sudden blindness. Muscle weakness leading to difficulties with coordination and balance commonly is noticed early. Muscle spasms, fatigue, numbness, and prickling pain are common symptoms. There may be a loss of sensation, speech impediment, tremors, dizziness, or occasionally hearing loss. Fifty percent of patients experience mental changes such as decreased concentration, attention deficits, some degree of memory loss, or impairment in judgment. Other symptoms may include depression, manic depression, paranoia, or an uncontrollable urge to laugh and weep called laughing-weeping syndrome. As the disease worsens, patients may experience sexual dysfunction or reduced bowel and bladder control. Heat appears to intensify MS symptoms for about 60% of patients, and relief is found with cold baths or swimming. Pregnancy seems to reduce the number of attacks.

There is no single test for MS. Physicians, particularly neurologists, can take into consideration detailed medical histories and can perform complete physical and neurological examinations in order to diagnose MS. Testing for MS can include, e.g., magnetic resonance imaging (MRI) with intravenous gadolinium or magnetic resonance scanning (MRS), both of which help to identify, describe, and date lesions in the brain (i.e., plaques) that occur in MS patients. Another electro-physiological test, evoked potentials, examines the impulses traveling through the nerves to determine if the impulses are moving normally or too slowly; slower than normal movement of impulses through the nerves is indicative of MS. Finally, examination of the cerebro-spinal fluid that surrounds the spinal cord may be used to identify abnormal chemicals or cells floating in the brain or spinal cord that suggest the presence of MS. Collectively, these three tests strengthen the diagnosis of MS. MS can also be diagnosed by identifying one or more of the following symptoms in a patient: tingling, numbness, tremors, loss of balance, weakness in one or more limbs, blurred or double vision, slurred speech, swallowing problems, paralysis, lack of coordination, cognitive difficulties (e.g., decreased memory and concentration), fatigue, muscle spasms, dizziness, breathing problems, and seizures.

All of the methodologies described above are also useful for monitoring the progression of MS in patients, as well as for monitoring the resolution of MS following treatment using the compositions and methods of the invention (e.g., the resolution or decrease in the severity or frequency of one or more symptoms of MS), such that the effectiveness of the treatment received by the patient can be assessed. In addition, a patient can be assessed for an improvement in MS following treatment (e.g., an improvement in one or more symptoms of MS (e.g., a decrease in the occurrence, length, or severity of one or more of the symptoms of MS), or for an improvement in motor neural function) using one of several methods known in the art (see, e.g., the Expanded Disability Status Scale (EDSS), Kurtzke, Neurology 33:1444-1452, 1983; and the Multiple Sclerosis Severity Score (MSSS), Roxburgh et al., Neurology 64:1144-1151, 2005).

### Methods of Treatment of MS by Administration of the Compositions of the Present Invention

The present invention features methods of treating MS in a patient by co-administering a therapeutically effective amount of an AFP (or biologically active fragment thereof) and an integrin antagonist; the compositions of the invention may, but need not, also include additional therapeutic agents, such as those described below. The compositions of the invention can be administered to a patient to treat, prevent, ameliorate, inhibit the progression of, or reduce the severity of one or more symptoms of MS in a human patient. The AFP (or biologically active fragment thereof) and an integrin antagonist may be administered coextensively or separately, in a single dose or in multiple doses. The AFP (or biologically active fragment thereof) and integrin antagonist may be formulated for the same route of administration or formulated for different routes of administration.

Examples of the symptoms of MS that can be treated using the compositions of the invention include: tingling, numbness, tremors, loss of balance, weakness in one or more limbs, blurred or double vision, slurred speech, swallowing problems, paralysis, lack of coordination, cognitive difficulties (e.g., decreased memory and concentration), fatigue, muscle spasms, dizziness, breathing problems, and seizures. These symptoms of MS, and their resolution during treatment, may be measured by a physician during a physical examination or by using one or more of the tests described above.

A physician may adjust the dose (e.g., increase or decrease the dose) of the AFP or of the integrin antagonist administered to the patient based on the severity of, occurrence of, or progression of MS in the patient. For example, a physician can increase the dose of the AFP or of the integrin antagonist if necessary to alleviate one or more symptoms of MS in a patient. Alternatively, a physician can decrease the dose of the AFP or of the integrin antagonist based on an improvement in one or more symptoms of MS in the patient or to avoid toxicity associated with, e.g., the administration of an integrin antagonist.

The combination therapies of the present invention, which include, e.g., an AFP and an integrin antagonist, preferably exhibit a greater therapeutic effect (e.g., improved efficacy or reduced toxicity at higher doses) than that observed when either agent is administered alone.

### Compositions of the Present Invention

The present invention provides compositions including an AFP (or a biologically active fragment, derivative, or analog thereof) and an integrin antagonist (e.g., an α4 integrin antagonist) for the treatment of MS. The compositions of the invention may be formulated for any route of administration (e.g., the formulations described herein) and may be administered in a single dose or in multiple doses to a subject in need thereof. The compositions of the invention may also include supplemental agents, e.g., a CD80, P-selectin, sphingosine-1-phosphate receptor-1, hyaluronate receptor, leukocyte function antigen-1 (LFA-1), CD11/CD18, CD20, CD86, ICOS ligand, CCR2, CXCR3, CCR5, CD40, CD154, CD28, IL-23, IL-17, or IL-6 antagonist. The supplemental agents can be antibodies, blocking peptides, nucleic acid inhibitors, or small molecule inhibitors that antagonist CD80, P-selectin, sphingosine-1-phosphate receptor-1, hyaluronate receptor, leukocyte function antigen-1 (LFA-1), CD11/CD18, CD20, CD86, ICOS ligand, CCR2, CXCR3, CCR5, CD40, CD154, CD28, IL-23, IL-17, or IL-6.

### AFPs for Use in the Compositions and Methods of the Invention

An alpha-fetoprotein for use in the compositions and methods of the present invention are described below. For the purposes of the present invention, both naturally occurring human AFP and recombinantly produced AFP polypeptides or biologically active fragments thereof can be used. Naturally occurring human AFP can be obtained by, e.g., purification from umbilical cord blood or umbilical cord serum. Recombinant AFP polypeptide or biologically active fragment thereof can be obtained, e.g., by using a prokaryotic or eukaryotic expression system, such as those described in, e.g., U.S. Patent No. 5,384,250 and U.S. Patent Application Publication No. 20040098755 (each of which is herein incorporated by reference). These methods include the purification of AFP from a biological fluid of transgenic mammal that has been engineered to express AFP into the biological fluid, as well as other methods known in the art. These AFPs can be used in the present invention notwithstanding the fact that the use of these different expression systems (e.g., production in a prokaryotic host cell, a eukaryotic host cell, or a transgenic animal or plant) may result in a recombinant AFP or fragment thereof having different post-translational modifications than that in the wild-type AFP (e.g., a different number of attached sugar residues (e.g., at least 1, 2, 3, 4, 5, 6, 7, or 10 sugar molecules), a different type of glycosylation (e.g., O-linked glycosylation or N-linked glycosylation, or the lack thereof), a different type of sugar residues (e.g., mannose, galactose, N-acetyl-galactosamine, N-acetyl-glucosamine, glucuronate, sialic acid, or xylose, or different combinations thereof), or different amino acids glycosylated). For instance, naturally occurring human AFP is a variably glycosylated protein (e.g., glycosylation at asparagine 233 of SEQ ID NO: 1). In contrast, the recombinant AFP or fragment may be unglycosylated when produced by a prokaryotic host cell or may be somewhat differently glycosylated when produced by a eukaryotic host cell. Alternatively, a recombinant AFP can be genetically modified to eliminate glycosylation (e.g., by removing a glycosylation site, for instance replacing asparagine 233 of SEQ ID NO: 1 with any amino acid other than asparagine), regardless of the expression system in which it is produced. Human AFP is available through various commercial suppliers, including Fitzgerald Industries International (Concord, MA), Cell Sciences (Canton, MA), and Biodesign International (Saco, ME).

Furthermore, it is possible to employ well-known chemical synthesis methods to synthesize an AFP polypeptide or fragment, particularly when the AFP fragment is a peptide of a relatively short length, e.g., with less than 50 or 100 amino acids.

Any AFP polypeptide or fragment thereof, regardless of its origin or status of post-translational modification, can be used in the present invention if the polypeptide or fragment has the same or substantially the same biological activity (e.g., at least about 40%, desirably at least about 50%, 60%, 70%, and more desirably at least about 80%, 90%, 100%, or 100% or more of the biological activity) of native human AFP (e.g., as determined based on the ability of the AFP to bind to human leukocytes, to suppress human autologous mixed lymphocyte reactions (AMLR), to suppress EAE in a mouse model, or to inhibit release of inflammatory cytokines in a splenocyte assay).

Similarly, fragments of the human AFP can also be used in the compositions and methods of the present invention, so long as the fragments retain the same or substantially the same biological activity of naturally occurring human AFP (as determined using one or more of the assays for AFP biological activity described below). Fragments of human AFP can be generated by methods known to those skilled in the art, e.g., proteolytic cleavage or recombinant expression, or may result from normal protein processing (e.g., removal from a nascent polypeptide of amino acids that are not required for biological activity). Fragments of human AFP can also be produced recombinantly using the techniques described above. Chemical methods can also be useful for synthesizing active AFP fragments.

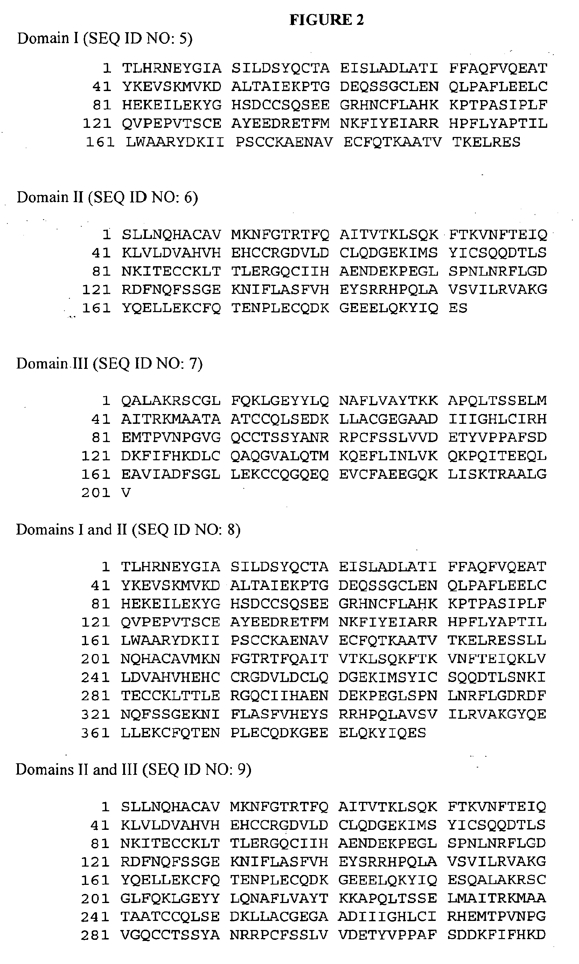

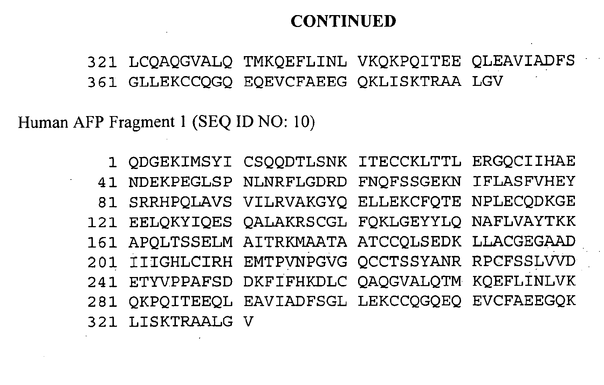

Examples of human AFP fragments suitable for use in practicing the present invention are shown in Figure 2 and include Domain I (amino acids 2-198 of mature human AFP; SEQ ID NO: 5), Domain II (amino acids 199-390 of mature human AFP; SEQ ID NO: 6), Domain III (amino acids 391-591 of mature human AFP; SEQ ID NO: 7), Domain I+II (amino acids 2-390 of mature human AFP; SEQ ID NO: 8), Domain II+III (amino acids 199-591 of mature human AFP; SEQ ID NO: 9), and AFP Fragment I (amino acids 267-591 of mature human AFP; SEQ ID NO: 10). Other examples of known AFP fragments are described herein or can be found in, e.g., U.S. Patent No. 5,707,963 and U.S. Patent No. 6,818,741, herein incorporated by reference.

Also encompassed within the claimed invention is the use of functional derivatives or analogs of full-length native human AFP or fragments thereof. As described in earlier sections, such derivatives or analogs can differ from the full-length native human AFP or portions thereof by amino acid sequence differences (e.g., additions, deletions, conservative or non-conservative substitutions), or by modifications (e.g., post-translational modifications) that do not affect sequence, or by both. The derivatives/analogs of the invention will generally exhibit at least 90%, more preferably at least 95%, or even 99% amino acid identity with all or part of the native human AFP amino acid sequence (SEQ ID NO: 1).

An AFP derivative/analog may differ from a naturally occurring human AFP due to post-translational modifications (which do not normally alter primary sequence), which include *in vivo,* or *in vitro* chemical derivatization of polypeptides, e.g., acetylation, carboxylation, or pegylation; such modifications may occur during polypeptide synthesis or processing, or following treatment with isolated modifying enzymes. Also included are cyclized peptide molecules and analogs that contain residues other than L-amino acids, e.g., D-amino acids, non-naturally occurring, or synthetic amino acids, e.g., β or γ amino acids, or L-amino acids with non-natural side chains (see, e.g., Noren et al., Science 244:182, 1989). Methods for site-specific incorporation of non-natural amino acids into the protein backbone of proteins is described, e.g., in Ellman et al., Science 255:197, 1992. Also included are chemically synthesized polypeptides or peptides with modified peptide bonds (e.g., non-peptide bonds as described in U.S. Patent No. 4,897,445 and U.S. Patent No. 5,059,653; herein incorporated by reference) or modified side chains to obtain the desired pharmaceutical properties as described herein. Useful AFP, AFP fragments, AFP derivatives, and AFP analogs having the same or substantially the same biological activity (e.g., at least about 40%, desirably at least about 50%, 60%, 70%, and more desirably at least about 80%, 90%, 100%, or 100% or more of the biological activity) of wild-type AFP can be identified using art-recognized methods, such as those described below.

Some preferred functional AFP derivatives contain one or more conservative substitutions, in which certain amino acid residues are substituted by other residues having similar chemical structures (e.g., alanine for glycine, arginine for lysine, etc.). The derivatives/analogs mentioned above may include allelic variants, inter-species variants, and genetic variants, both natural and induced (for example, resulting from random mutagenesis by, e.g., site-specific mutagenesis according to methods described in scientific literature such as Sambrook et al., Molecular Cloning, A Laboratory Manual, 3rd ed., 2001; Kriegler, Gene Transfer and Expression. A Laboratory Manual, 1990; and Ausubel et al., eds., Current Protocols in Molecular Biology, 1994.

### AFP Activity Assays

As stated above, AFP polypeptides or AFP fragments suitable for use in the compositions and methods of the present invention may include various derivatives, analogs, or fragments of the naturally occurring human AFP, so long as the polypeptides or fragments retain the same or substantially the same biological activity of mature human AFP. The biological activity of an AFP of the present invention can be demonstrated by using, e.g., one or more of the following assays.

A first assay for testing a candidate AFP for biological activity entails measuring the ability of the AFP to specifically bind to human leukocytes (e.g., peripheral monocytes). A binding assay suitable for this purpose is described in, e.g., Parker et al., Protein Express. Purification 38:177-183, 2004. Briefly, a competitive assay format is used to test a candidate AFP for its ability to specifically bind to U937 cells, a human monocytic cell line. The cells are maintained in RPMI media with 10% fetal bovine serum. Prior to the binding assay, the cells are washed twice with serum-free media and adjusted to 2.5 x 10⁶ cells/ml in phosphate-buffered saline (PBS). Native human AFP (SEQ ID NO: 1) or non-glycosylated human AFP (see, e.g., SEQ ID NO: 36, where, e.g., residue 233 is glutamine) is labeled with a detectable label, e.g., fluorescein, in a proper reaction followed by removal of the unattached labeling material, for instance, by gel filtration. In the case of labeling human AFP with fluorescein, the protein is mixed with a solution of fluorescein-5-isothiocyanate in dimethyl sulfoxide for 1 hour in the dark, followed by gel filtration to remove unbound dye. Labeled human AFP is stored in 20% glycerol at -20 °C until use.

For the binding assay, a certain number of U937 cells (e.g., 40 µl of cell suspension at 2.5 x 10⁶ cells/ml concentration) are mixed with a pre-determined amount of labeled human AFP (e.g., at a final concentration of 0.5 µM) and with unlabeled human AFP or an unlabeled candidate AFP, each at a set of final concentrations (e.g., 20, 10, 5, 2.5, 1.25, and 0.625 µM) to determine the IC₅₀ values for both human AFP and the candidate AFP. At the conclusion of the binding process, the cells are then washed with PBS and suspended in fresh PBS so that the labeled AFP remaining on U937 cells can be measured, e.g., by flow cytometry.

A second assay for testing a candidate AFP for biological activity entails measuring the ability of the AFP to suppress autoimmune reactions, either in AMLR or in a mouse model of EAE. Methods are known in the art for testing AMLR and its inhibition. For instance, U.S. Patent Nos. 5,965,528 and 6,288,034 (each of which is herein incorporated by reference) describe the AMLR system as follows: isolation of human peripheral blood mononuclear cells (PBMC), their fractionation into non-T-cell populations, and the AMLR, performed according to standard procedures. Briefly, responder T-cells are isolated by passing 1.5 x 10⁸ PMBC over a commercial anti-Ig affinity column (US Biotek Laboratories, Seattle, WA) and 2 x 10⁵ responder cells are subsequently cultured with 2 x 10⁵ autologous ¹³⁷Cs-irradiated (2500 rads) non-T stimulator cells from a single donor. The medium employed consists of RPMI-1640 supplemented with 20 mM HEPES (Invitrogen), 5 x 10⁻⁵ M 2-mercaptoethanol (BDH, Montreal, QC), 4 mM L-glutamine (Invitrogen), 100 U/ml penicillin (Invitrogen), and 100 µg/ml streptomycin sulfate, with the addition of 10% fresh human serum autologous to the responder T-cell donor for the AMLR. Varying concentrations of purified recombinant human AFP, human serum albumin, antihuman AFP monoclonal antibody clone #164 (125 µg/ml final concentration in culture) (Leinco Technologies, St. Louis, MO) are added at the initiation of cultures. AMLR cultures are incubated for 4 to 7 days, at 37 °C in 95% air and 5% CO₂. At the indicated intervals, DNA synthesis is assayed by a 6 hour pulse with 1 µCi of ³H-thymidine (specific activity 56 to 80 Ci/mmole; ICN Radioisotopes, Cambridge, MA). The cultures are harvested on a multiple sample harvester (Skatron, Sterling, VA), and the incorporation of ³H-TdR is measured in a Packard 2500 TR liquid scintillation counter. Results are expressed as mean cpm ± the standard error of the mean of triplicate or quadruplicate cultures.

The immunosuppressive activity of a candidate AFP within the scope of the present invention can be assessed by its ability to suppress human autologous mixed lymphocyte reactions (AMLR). Generally, the candidate AFP is tested for its ability to inhibit the proliferative response of autoreactive lymphocytes stimulated by autologous non-T-cells, by measuring lymphocyte autoproliferation throughout a time course of 4 to 7 days. Suppression of AMLR in a dose-dependent manner is demonstrated by results from dose-response studies performed at the peak of T-cell autoproliferation where an AFP is added at the initiation of cultures. Furthermore, parallel viability studies can be used to establish that the inhibitory activity of an AFP polypeptide or fragment on human autoreactive T-cells is not due to non-specific cytotoxic effects.

A third assay for testing a candidate AFP for biological activity involves the use of a myelin oligodendrocyte glycoprotein (MOG) mouse model of experimental autoimmune encephalomyelitis (EAE) (see, e.g., Fritz et al., J. Immunol. 130:1024, 1983; Naiki et al., Int. J. Immunopharmacol. 13:235, 1991; and Goverman, Lab. Anim. Sci., 46:482, 1996). In this *in vivo* assay, genetically susceptible strains of mice are subcutaneously immunized with MOG emulsified in Complete Freund's Adjuvant (CFA), which leads to the development of EAE in the animals. A candidate AFP is administered to a selected group of mice on a daily basis, beginning prior to, at the same time, or subsequent to the start of the administration of MOG to the animals. The symptoms of EAE in these animals are monitored and compared to those in a control group (e.g., those receiving only saline injections) over a certain time period, e.g., 30 days. Severity of EAE in each animal is given a score between 1-5 based on defined clinical symptoms; the average score of animals in a group indicates the disease state of the group. A biologically active AFP will reduce the severity of EAE in animals receiving MOG compared to controls (e.g., at least a 50% reduction in the severity of disease after 30 days of treatment).

A fourth assay that can be used to test a candidate AFP for biological activity examines the ability of the candidate AFP to inhibit or reduce the release of inflammatory cytokines from mitogen-stimulated *in vitro* splenocyte cultures obtained from naive mice (e.g., as described in Hooper and Evans, J. Reprod. Immunol. 16: 83-961,1989; and Kruisbeek, in Current Protocols in Immunology, Vol. 1, Section 3.1.1-3.1.5, 2000). Splenocytes are stimulated with phytohemagglutinin (PHA), concavalin A (ConA), or lipopolysaccharide (LPS) in the presence of increasing concentrations of an AFP for 24 hours. Human serum albumin is used as a negative control for the assays. A 10 point dose response study has shown that biologically active AFP inhibits or substantially inhibits the secretion of PHA induced IFN-γ in a reproducible manner.

### Integrin Antagonists for Use in the Compositions and Methods of the Invention

An integrin antagonist for use in the methods of this invention is an agent that decreases (e.g., by at least 5%, 10%, 15%, 20%, 25%, 30%, 35%, 40%, 45%, 50%, 60%, 70%, 80% or more) the biological activity or expression (transcription or translation) of α4 integrin. Integrin antagonists can include antibodies, blocking peptides, nucleic acid inhibitors, or small molecule inhibitors. As understood in the field of biological science, integrins are integral membrane proteins present in the plasma membrane of cells, that, *inter alia,* play a role in signal transduction and in the attachment of a cell to the extracellular matrix (ECM). There are many types of integrins, and many cells have multiple types on their surface.

Structurally, integrins are non-covalent heterodimeric complexes consisting of an α subunit and a β subunit. See, Hemeler, Ann. Rev. Immunol. 8:365, 1990. About 18 α and 8 β subunits have been characterized, with additional variants formed due to differential splicing. Through the different combinations of α and β subunits, at least 24 unique integrins can be generated.

The α4β1 integrin, or very late antigen-4 (VLA-4), is constituitively expressed by all leukocytes (e.g., monocytes, lymphocytes, basophils, eosinophils, mast cells, macrophages, and neutrophils). The binding of VLA-4 to one of its ligands has a number of known cell adhesion and activation functions. *See,* for example, Hemeler, *supra*; Walsh et al., Clin. and Exp. Allergy, 25:1128, 1995; and Hutala et al., J. Cell Biol. 129:867, 1995. In particular, VLA-4 interacts with the cytokine inducible endothelial cell surface protein known as vascular cell adhesion molecule-1 (VCAM-1), and the alternatively spliced forms of the extracellular matrix protein fibronectin containing the CS-1 domain (Ruegg et al. J Cell Biol. 177:179, 1991) and the extracellular matrix protein osteopontin (Bayless et al., J. Cell Sci. 111:1165-1174, 1988).

The expression of an α4 integrin may be measured using art-known methods including Western blot, ELISA, fluorescence-assisted cell sorting (FACS), proteomics, RT-PCR, Northern blot, and gene chip technology. The biological activity of α4 integrin may be measured in cellular based assays, including, but not limited to: JAK3 activation and phosphorylation, STAT6 activation and phosphorylation, and STAT6-responsive reporter plasmid assays (e.g., assays using a promoter which is activated by STAT6, e.g., IGE, MHC class II, and CD23). Additional assays for integrin antagonists are described below.

Examples of integrin antagonists are described in: U.S. Patent Nos. 5,821,231; 5,869,448; 5,936,065; 6,197,794; 6,229,011; 6,265,572; 6,288,267; 6,329,372; 6,348,463; 6,362,204; 6,365,619; 6,380,387; 6,388,084; 6,423,728; 6,426,348; 6,445,550; 6,458,844; 6.479,666; 6,482,849; 6,596,752; 6,667,331; 6,668,527; 6,685,617; 6,806,365; 6,835,738; 6,855,706; 6,872,719; 6,878,718; 6,903,128; 6,911,451; 6,916,933; 7,015,216; 7,105,520; 7,153,963; 7,160,874; 7,193,108; 7,250,516; and 7,291,645 (each herein incorporated by reference); in U.S. Patent Application Publication Nos. 2002/0049236; 2003/0004196; 2003/0018016; 2003/0078249; 2003/0083267; 2003/0100585; 2004/0039040; 2004/0053907; 2004/0087574; 2004/0102496; 2004/0132809; 2004/0229858; 2004/0229859; 2006/0014966; 2006/0030553; 2006/0166866; 2006/0166961; 2006/0211630; 2006/0241132; 2007/0054909; and 2007/066533 (each herein incorporated by reference); in European Patent Nos. EP 0842943; EP 0842944; EP 0842945; EP 0903353; and EP 0918059; in PCT Publication Nos. WO 93/13798; WO 93/15764; WO 94/15958; WO 94/16094; WO 95/15973; WO 95/19790; WO 96/00581; WO 96/06108; WO 96/22966; WO 96/40781; WO 97/02289; WO 97/03094; WO 97/49731; WO 98/04913; WO 98/04247; WO 98/42656; WO 98/53814; WO 98/53817; WO 98/53818; WO 98/54207; WO 98/58902; WO 99/06390; WO 99/06431; WO 99/06432; WO 99/06433; WO 99/06434; WO 99/06435; WO 99/06436; WO 99/06437; WO 99/10312; WO 99/10313; WO 99/20272; WO 99/23063; WO 99/24398; WO 99/25685; WO 99/26615; WO 99/26921; WO 99/26922; WO 99/26923; WO 99/35163; WO 99/36393; WO 99/37605; WO 99/37618; WO 99/43642; WO 01/42215; WO 01/47868; WO 01/70670; and WO 02/28830. Examples of antibodies, blocking peptides, nucleic acid inhibitors, and small molecule inhibitors that can be used as integrin antagonists are described below.

### Antibodies

An integrin antagonist can be an antibody. Examples of antibodies include monoclonal antibodies, polyclonal antibodies, multispecific antibodies (e.g. bi-specific antibodies) formed from at least two intact antibodies, and antibody fragments so long as they function as an integrin antagonist. Other kinds of antibody included in the invention are antibody fragments (e.g., Fab, Fab', Fv fragments, diabodies, linear antibodies, and single chain antibody molecules). Additional examples of antibodies include monoclonal antibodies that are chimeric, primatized, or humanized. The term antibody herein used also encompasses proteins or molecules which contain the amino acid sequence of the variable region of an immunoglobulin gene (e.g., an antibody mimic). Methods for the production of the various antibodies described above are known in the art.

Studies using a specific monoclonal antibody antagonist of α4 integrin have demonstrated that inhibitors of VLA-4 cell adhesion can inhibit or reduce the severity of numerous pathological conditions, including inflammatory, respiratory, and autoimmune conditions (Chisholm et al., Eur. J. Immunol. 23:682, 1993; Richards et al., Am. J. Resp. Cell Mol. Biol. 15:172, 1996; Fryer et al., J. Clin. Invest. 99:2036, 1997; Soiluhanninen et al., J. Neuroimmunol. 72:95, 1997). These pathological processes can also be inhibited with agents other than antibodies, as evidenced in animal model studies using synthetic CS-1 peptide or small molecule peptide inhibitors of VLA-4 (Ferguson et al., Proc. Natl. Acad. Sci., 88:8072, 1991; Wahl et al., J. Clin. Invest. 94:655, 1994; Molossi et al., J. Clin. Invest. 95:2601, 1995; Abraham et al., J. Resp. Crit. Care. Med. 156:696, 1997; and Jackson et al., J. Med. Chem. 40:3359, 1997).

An exemplary antibody that binds and blocks the biological activity of α4 integrin is natalizumab, a recombinant humanized IgG4k monoclonal antibody produced in murine myeloma cells (reviewed in Rudick and Sandrock, Expert Rev. Neurother. 4:571-580, 2004). Natalizumab contains human antibody framework regions and the complementarity-determining regions of a murine antibody that binds to α4-integrin.

Other compounds have been developed that can target and bind to α4 integrin in a manner similar to antibodies. Certain of these "antibody mimics" use non-immunoglobulin protein scaffolds as alternative protein frameworks for the variable regions of antibodies. For example, Ladner et al. (U.S. Patent No. 5,260,203, herein incorporated by reference) describe single polypeptide chain binding molecules with binding specificity similar to that of the aggregated, but molecularly separate, light and heavy chain variable region of antibodies. The single-chain binding molecule contains the antigen binding sites of both the heavy and light variable regions of an antibody connected by a peptide linker and will fold into a structure similar to that of the two peptide antibody. The single-chain binding molecule displays several advantages over conventional antibodies, including, smaller size, greater stability, and are more easily modified.

The technique of Lipovsek et al. (U.S. Patent Nos: 6,818,418 and 7,115,396, herein incorporated by reference) can also be used to create integrin antagonists. Lipovsek et al. (U.S. Patent Nos: 6,818,418 and 7,115,396) discloses an antibody mimic featuring a fibronectin or fibronectin-like protein scaffold and at least one variable loop. Known as Adnectins, these fibronectin-based antibody mimics exhibit many of the same characteristics of natural or engineered antibodies, including high affinity and specificity for any targeted ligand.

The structure of these fibronectin-based antibody mimics is similar to the structure of the variable region of the IgG heavy chain. Therefore, these mimics display antigen binding properties similar in nature and affinity to those of native antibodies. Further, these fibronectin-based antibody mimics exhibit certain benefits over antibodies and antibody fragments. For example, these antibody mimics do not rely on disulfide bonds for native fold stability, and are, therefore, stable under conditions which would normally break down antibodies. In addition, since the structure of these fibronectin-based antibody mimics is similar to that of the IgG heavy chain, a process for loop randomization and shuffling may be employed *in vitro* that is similar to the process of affinity maturation of antibodies *in vivo.*

Beste et al. (Proc. Natl. Acad. Sci. U.S.A. 96:1898-1903, 1999) also discloses techniques that can be used to create integrin antagonists. Beste et al. (*supra*) discloses an antibody mimic based on a lipocalin scaffold (i.e., an Anticalin^{®}). Lipocalins are composed of a β-barrel with four hypervariable loops at the terminus of the protein. Beste et al. (*supra*) subjected the loops to random mutagenesis and selected for binding with, for example, fluorescein. Three variants exhibited specific binding with fluorescein, with one variant showing binding similar to that of an anti-fluorescein antibody. Further analysis revealed that all of the randomized positions are variable, indicating that Anticalin^{®} would be suitable for use as an alternative to antibodies. Anticalins^{®} are small, single chain peptides, typically between 160 and 180 residues, which provide several advantages over antibodies, including decreased cost of production, increased stability in storage, and decreased immunological reaction. Thus, the structural framework of Anticalins^{®} can be used to produce an integrin antagonist according to the present invention.

Hamilton et al. (U.S. Patent No. 5,770,380, herein incorporated by reference) discloses a method of making synthetic antibody mimics that may be used as integrin antagonists. Hamilton et al. (*supra*) describes a method of making a synthetic antibody mimic using the rigid, non-peptide organic scaffold of calixarene, attached with multiple variable peptide loops used as binding sites. The peptide loops all project from the same side of the calixarene, with respect to each other. Because of this geometric confirmation, all of the loops are available for binding, increasing the binding affinity to a ligand. However, in comparison to other antibody mimics, the calixarene-based antibody mimic does not consist exclusively of a peptide, and therefore, it is less vulnerable to attack by protease enzymes. Neither does the scaffold consist purely of a peptide, DNA, or RNA, meaning this antibody mimic is relatively stable in extreme environmental conditions and has a long life span. Further, since the calixarene-based antibody mimic is relatively small, it is less likely to produce an immunogenic response.

Another class of antibody mimics that may be used to produce an integrin antagonists is disclosed in Murali et al., Cell. Mol. Biol. 49:209-216, 2003. Murali et al. (*supra*) discloses a methodology for reducing antibodies into smaller peptidomimetics, which may also be useful as an alternative to antibodies in the practice of the present invention.

In addition to non-immunoglobulin protein frameworks, antibody properties have also been mimicked in compounds that include RNA molecules and unnatural oligomers (e.g., protease inhibitors, benzodiazepines, purine derivatives, and beta-turn mimics), each of which may be used in the preparation of integrin antagonist suitable for use with the present invention.

### Blocking Peptides

Other exemplary integrin antagonists suitable for use with the present invention are blocking peptides that bind to and antagonize the activity of VLA-4 on leukocytes. The VLA-4 binding domain in the CS-1 region of fibronectin comprises the octapeptide: Glu-Ile-Leu-Asp-Val-Pro-Ser-Thr, as well as the overlapping pentapeptides Glu-Ile-Leu-Asp-Val and Leu-Asp-Val-Pro-Ser. Thus, the minimal amino acid sequence required for inhibition would be Leu-Asp-Val (LDV). In fact, the LDV minimal tripeptide sequence has been shown to be equally effective as the full length CS-1 fragment in binding the activated from of VLA-4 (Wayner et al., J. Cell Biol. 116:489, 1992). Another exemplary integrin antagonist suitable for use in the present invention are Arg-Gly-Asp (RGD) based cyclic peptides capable of inhibiting both α4β1 and α5β1 integrins from binding to fibronectin, as disclosed in Nowlin et al., J. Biol. Chem. 268:20352, 1993; and PCT/US91/04862. The tri- and tetra-peptide sequences IDA and REDV are suitable for use as blocking peptides in the present invention, as they have been shown to regulate fibronectin binding to VLA-4, while the pentapeptide QIDSP regulates binding of VLA-4 to VCAM-1 and is also suitable for use with the present invention (Humphries et al., Ciba Found. Symp. 189:177, 1995). Additional VLA-4 and VLA-5 blocking peptides that have been shown to inhibit a hypersensitivity response include GRDGSP and EILDV.

Ku et al. (Proc. Natl. Acad. Sci. U.S.A. 92:6552-6556, 1995) describes a method of creating peptide antagonists that may function as integrin antagonists. Ku et al. (*supra*) discloses a peptide antagonist based on cytochrome b562. In this method, Ku et al. generated a library in which two of the loops of cytochrome b562 were randomized and selected for binding against bovine serum albumin (BSA). The individual mutants were found to bind selectively with BSA similar to anti-BSA antibodies. Similar mutants can be constructed to selectively bind the α4 integrin subunit.

### Nucleic Acid Inhibitors

In some embodiments, the integrin antagonist is a nucleic acid inhibitor. For example, ribozymes, antisense RNA and/or interfering RNA (RNAi) molecules can be used to target α4 integrin subunit.

In some embodiments, RNAi molecules are used to target the α4 integrin subunit. In mammalian cells, the introduction of dsRNAs (e.g., dsRNAs having at least 10, 20, 30, 40, 50, or 50 or more nucleotides in length; and desirably having 21 nucleotides in length) often initiates a potent antiviral response, which is exemplified by nonspecific inhibition of protein synthesis and RNA degradation. The phenomenon of RNA interference is described and discussed, for example, in Bass, Nature 411:428-29, 2001; Elbahir et al., Nature 411:494-98, 2001; and Fire et al., Nature 391:806-11, 1998; wherein methods of making interfering RNA also are discussed. RNAi molecules targeting the α4 integrin may include all or part of a nucleic acid sequence the complement of which is substantially identical to the mRNA sequence of α4 integrin (SEQ ID NO: 12 or SEQ ID NO: 35). The nucleic acid sequence preferably has at least 10, 20, 30, 40, 50, or even 50 or more nucleotides in length (e.g., 21 or 25 nucleotides in length). The RNAi molecules can be made by methods known in the art. Exemplary RNAi molecules according to the invention could have up to 29 bps, 25 bps, 22 bps, 21 bps, 20 bps, 15 bps, 10 bps, 5 bps or any integer thereabout or therebetween. The RNAi molecule may have a sequence which is complementary to any sequence within SEQ ID NO: 12 or SEQ ID NO: 35 (e.g., a sequence that is complementary to nucleotides 1-25, 50-75, 50-100, 50-150, 50-1000, or 50-2000 of SEQ ID NO: 12 or SEQ ID NO: 35).

The RNAi can also comprise two complementary molecules, or can be constructed such that a single transcript has both the sense and complementary antisense sequences from the target sequence (e.g., SEQ ID NO: 12 or SEQ ID NO: 35) so that the RNAi molecule is capable of forming a hairpin.

Methods for designing double stranded RNA to inhibit gene expression in a target cell are known (e.g., US Patent No. 6,506,559; Elbashir et al., Methods 26:199-213, 2002; Chalk et al., Biochem. Biophysy Res. Comm 319:264-274, 2004; Cui et al., Comp. Method Prog. Biomed. 75:67-73, 2004; and Wang et al., Bioinformatics 20:1818-1820, 2004). For example, the design of RNAi molecules (including hairpins) typically follows known thermodynamic rules (see, e.g., Schwarz et al., Cell 115:199-208, 2003; Reynolds et al., Nature Biotechnol. 22:326-30, 2004; and Khvorova et al., Cell 115:209-16, 2003). Many computer programs are available for selecting regions of the α4 integrin sequence that are suitable target sites. These include programs available through commercial sources such as Ambion, Dharmacon, Promega, Invitrogen, Ziagen, and GenScript as well as noncommercial sources, such as EMBOSS, The Wistar Institute, Whitehead Institute, and others.

For example, design can be based on the following considerations. Typically shorter sequences, i.e., less than about 30 nucleotides are selected. The coding region of the mRNA is usually targeted. The search for an appropriate target sequence optionally begins 50-100 nucleotides downstream of the start codon, as untranslated region binding proteins and/or translation initiation complexes may interfere with the binding of the siRNP endonuclease complex. Some algorithms, e.g., based on the work of Elbashir et al., *supra,* search for a 23-nt sequence motif AA(N19)TT (N, any nucleotide) and select hits with approximately 50% G/C-content (30% to 70% G/C-content can also be used for selection). If no suitable sequences are found, the search is extended using the motif NA(N21). The sequence of the sense RNAi corresponds to (N19)TT or N21 (position 3 to 23 of the 23-nt motif), respectively. In the latter case, the 3' end of the sense siRNA is converted to TT.

Other algorithms preferentially select RNAi molecules corresponding to the target motif NAR(N17)YNN, where R is purine (A, G) and Y is pyrimidine (C, U). The respective 21-nt sense and antisense RNAi therefore begin with a purine nucleotide and can also be expressed from polymerase III expression vectors without a change in targeting site; expression of RNAs from polymerase III promoters is only efficient when the first transcribed nucleotide is a purine.

Other nucleic acids, e.g., ribozymes or antisense, can also be designed based on known principles. For example, Sfold (see, for example, Ding et al., Nucleic Acids Res. 32 Web Server issue:W135-W141; Ding and Lawrence, Nucl. Acids Res. 31: 7280-7301, 2003; and Ding and Lawrence, Nucl. Acids Res. 20:1034-1046, 2001) provides programs relating to designing ribozymes and antisense, as well as RNAi molecules. Examples of antisense RNA molecules that target α4 integrin include a nucleic acid sequence that is complementary to a nucleic acid sequence that is substantially identical to all or part of the mRNA sequence of α4 integrin (SEQ ID NO: 12 or SEQ ID NO: 35), may consist of at least 10, 20, 30, 40, 50, 60, 70, 80, 90, 100, or even 100 or more nucleotides; and, in particular, may be complementary to nucleotides 1-25, 50-75, 50-100, 50-150, 50-1000, or 50-2000 of SEQ ID NO: 12 or SEQ ID NO: 35.

An additional class of nucleic acid inhibitors are peptide-nucleic acids. Peptide-nucleic acids are molecules which include a nucleic acid sequence that is complimentary to a sequence substantially identical to all or a part of the mRNA nucleic acid sequence of a target protein (e.g., an α4 integrin). Peptide-nucleic acids that are effective as integrin antagonists, have a nucleic acid sequence that is complimentary to a sequence substantially identical to all or a part of the mRNA of α4 integrin (SEQ ID NO: 12 or SEQ ID NO: 35), contain at least 10, 20, 30, 40, 50, 60, 70, 80, 90, 100, or even greater than 100 nucleotides; and promote a decrease in α4 integrin activity or expression. Representative United States patents that teach the preparation of PNA compounds include, but are not limited to, U.S. Pat. Nos.: 5,539,082; 5,714,331; and 5,719,262, each of which is herein incorporated by reference. Further teaching of PNA compounds can be found in Nielsen et al., Science, 254, 1497 (1991).

### Small Molecule Inhibitors

Another class of integrin antagonists suitable for use with the present invention include small molecule inhibitors. Non-limiting exemplary small molecule inhibitors suitable for use with the present invention include: the CS-1 peptidomimetics disclosed in PCT Publication Nos: WO 96/15973 and WO 96/06108; U.S. Patent Nos: 5,821,231; 5,936,065, and 5,869,448 (each herein incorporated by reference); the semi-peptidic inhibitors disclosed in PCT International Pub. No. WO 97/03094; the VLA-4 inhibitors disclosed in PCT International Pub. No. WO 96/22966, which uses the LDV tripeptide as a core group; cyclic peptides from 5 to 13 residues modeled after a portion of the CS 1 peptide and containing a free acid and the closely related compounds disclosed in PCT International Publication Nos: WO 96/00581, WO 97/49731, and WO 96/20216; and the cyclic tetrapeptide dimers, which comprise cyclic dimeric peptides in which a peptide 1 and peptide 2 independently represent a tetrapeptide juxtaposed in parallel or antiparallel orientation by means of two linking moieties as disclosed in PCT International Pub. No. WO 97/02289.

Another class of integrin antagonists suitable for use with the present invention include the sulfonylated-Pro-Phe compounds as disclosed in U.S. Patent No. 6,489,300 (herein incorporated by reference); sulfonylated dipeptide compounds disclosed in PCT International Publication No. WO 99/06437; dipeptide compounds disclosed in PCT International Publication Nos: WO 99/06432, WO 99/06433, and WO 99/06435; the tyrosine derivatives disclosed in PCT International Publication No. WO 98/54207; and the phenylalanine derivatives disclosed in U.S. Patent No. 6,174,794 (herein incorporated by reference) and PCT International Publication Nos: WO 99/37618, WO 99/35163, and WO 99/43642; the substituted phenylalanine compounds disclosed in PCT International Publication No. WO 99/06431; the 4-amino-phenylalanine type compounds disclosed in PCT International Publication No. WO 99/06434; the N-aroylphenylalanine derivatives and closely related compounds disclosed in PCT International Publication Nos: WO 99/10312 and WO 99/10313; and the cyclic amino acids disclosed in PCT International Publication No. WO 99/26615 and the closely related compounds using β-amino acids disclosed in WO 98/153814, WO 99/26921, and WO 99/33789 are suitable for use in the present invention.

Another exemplary class of small molecule integrin antagonists include the 5-ring heterocycles and related compounds disclosed in European Patent Application Nos: EP 842 943, EP 842 944 and EP 842 945; and the heterocyclic amide compounds as disclosed in PCT International Publication No. WO 98/53814.

Additional small molecule inhibitors for use as integrin antagonist in the present invention are disclosed in, e.g., U.S. Patent Publication Nos: 2003/0130349 and 2002/0049236 (herein incorporated by reference), and PCT International Publication Nos: WO 98/04247, WO 98/04913, WO 99/37605, WO 99/36393, WO 99/24398, WO 98/42656, and WO 96/01544. In addition, the substituted anilides as disclosed in PCT International Publication No. WO 99/23063; the carbamoyloxy compounds disclosed in PCT International Publication No. 99/06390; the benzyl compounds disclosed in PCT International Publication No. WO 99/06436; the imidazolidine derivatives and substituted imidazoline derivatives disclosed in European Patent Application Nos: EP 903 353 and EP 918 059; the biarylalkanoic acids disclosed in WO 98/53817; the sulfonamide compounds as disclosed in WO 98/53818 and the closely related azapeptide acids as disclosed in WO 99/20272; the 4-substituted-4-piperidine carboxamide derivatives disclosed in PCT International Publication No. WO 99/25685; the substituted pyrrole derivatives disclosed in PCT International Publication No WO 99/26922; and the para-aminomethylaryl carboxamide derivatives disclosed in WO 99/26923 are also suitable for use with the present invention.

Another class of integrin antagonists suitable for use with the present invention include the conjugates comprising more than one integrin antagonist covalently attached to a polymer as described in U.S. Patent Publication No. 2006/0013799 (herein incorporated by reference).

Further small molecule inhibitors can be identified using screening or biological assays (e.g., ligand binding assays, protein or receptor activity assays, and other assays as known in the art and described herein). For example, an integrin antagonist may be identified by screening commercially available chemical or small molecule libraries.

Additional antibodies, blocking peptides, nucleic acid inhibitors, or small molecules shown to be potent inhibitors of α4 mediated integrin adhesion to VCAM-1, CS-1, or osteopontin, using the assays disclosed herein are suitable for use with the methods of the invention for the treatment of patients with multiple sclerosis as described herein.

### Functional Assays for Integrin Antagonists

A variety of assays are available to establish the antagonistic activity of an integrin antagonist for use in the compositions and methods of the present invention. Non-limiting exemplary assays include the Jurkat-endothelial cell adhesion assay and the Jurkat-CS-1 assay as disclosed in U.S. Patent Publication No. 2003/0130349 (herein incorporated by reference), and the EAE model disclosed in greater detail in Example 2. PCT International Publication No. WO 98/53817 further discloses an assay for determining antagonism of α4β7 dependent binding to VCAM-Ig fusion protein.

The Jurkat-endothelial cell adhesion assay measures the adhesive interactions of a T-cell line (Jurkat), which express the α4β1 integrin, to endothelial monolayers in the presence of test compounds to identify integrin antagonists suitable for use with the present invention. Briefly, the test compounds are added in increasing concentration to the T-cells, and then the T-cell/compound mixture is added to interleukin-1 stimulated endothelial cell monolayers. The plates are incubated, washed, and the number of attached T-cells is quantitated. The assay directly demonstrates the cell adhesion inhibitory or modulatory activity of integrin antagonists at various concentrations for use with the present invention.

The Jurkat-CS-1 assay, described in U.S. Patent Publication No. 2003/0130349 is a modification of the previously published method of Cardarelli et al. (J. Biol. Chem. 269:18668-18673, 1994; and Proc. Natl. Acad. Sci. U.S.A. 83:2647-2651, 1986). Briefly, a CS-1 peptide, CLHPGEILDVPST, and the scrambled control peptide CLHGPIELVSDPT are immobilized onto microplates using a heterobifunctional crosslinker (e.g., 3-(2-pyridyldithio)propionic acid N-hydroxysuccinimide ester (SPDP) as described by Pierschbacher et al., Proc. Nat. Acad. Sci. U.S.A. 80:1224-1227, 1983). Briefly, the procedure involves coating the microtiter plates with horse-serum albumin (HSA) for 2 hours, washing the plates, and derivitizing with 10 µg/ml SPDP for 1 hour. After washing the derivitized plates, a recently dissolved 100 µg/ml cysteine containing CS-1 or control peptide solution is added and allowed to crosslink to the plates overnight at 4 °C. The unbound peptide is removed by washing and the unreacted sites are blocked with a 2.5 mg/ml solution of bovine-serum albumin (BSA). A known number of Jurkat cells in a defined volume (e.g., 100 µl of cells at 2.5 x 10⁶ cells/ml) are mixed with a desired concentration of the integrin antagonist and added to the peptide-coated dishes and incubated for 1 hour at 37 °C. Following incubation, the plates are washed and attached cells are fixed with 3% paraformaldehyde in PBS and stained with toluidine blue overnight at room temperature. Cell attachment is quantitated via optical density at 590 nm using a vertical pathway spectrophotometer.

Preferred integrin antagonists are those which have low IC50 values in the Jurkat endothelial cell assay or the CS-1 assay, or at least moderate activity in both assays. Typically, an integrin antagonist suitable for use with the present invention has activity at less than 50 µM in the CS-1 assay or at less than 500 µM in the endothelial assay.

### Supplemental Therapeutic Agents for Use in the Combination Therapies of the Invention

In addition to an AFP (or a biologically active fragment, derivative, or analog thereof) and an integrin antagonist, the combination therapies of the invention can also include the administration of an antagonist (e.g., an antibody, blocking peptide, nucleic acid inhibitor, or small molecule) to CD80, P-selectin, sphingosine-1-phosphate receptor-1, hyaluronate receptor, LFA-1, CD11/CD18, CD20, CD86, ICOS ligand, CCR2, CXCR3, CCR5, CD40, CD154, CD28, IL-23, IL-17, or IL-6.

For example, the compositions of the invention may include, or the methods of the invention may involve the administration of, a CD80 antagonist as a supplemental therapeutic agent. CD80 provides a co-stimulatory signal to the T-cell receptor (Lanier et al., J. Immunol. 154:97-105, 1995). Myeloid dendritic cells from MS patients have increased CD80 expression (Karni et al., J. Immunol. 177:4196-4202, 2006); therefore, CD80 is an attractive target for treatment of MS.

The compositions of the invention may also include, and the methods of the invention may also involve the administration of, an antagonist of P-selectin. P-selectin is a protein which plays a role in recruitment of leukocytes to the site of injury and is also a target for MS therapy.

In addition, compositions of the invention may also include, and the methods of the invention may also involve the administration of, a sphingosine-1-phosphate-receptor-1 (S1P1) antagonist. S1P1 plays a role in thymocyte and lymphocyte maturation. An inhibitor of S1P1 has been shown to deprive thymocytes and lymphocytes of the signal to egress from lymphoid organs (Brinkman et al., Am. J. Transplant. 4:1019-1025, 2004). Therefore, an inhibitor of S1P1 can be included in the AFP/integrin antagonist treatment regimen described herein.

A hyaluronate receptor (CD44) antagonist can also be included in the compositions of the invention or administered with an AFP and integrin antagonist in the methods of the invention. Hyaluronate receptor is a protein which plays a role in leukocyte extravasation in inflammatory central nervous system disease (Brennan et al., Immunology 98:427-435, 1999). Hyaluronate receptor is also highly expressed in the T cells of MS patients (Soilu-Hanninen et al., J. Neuroimmunol. 166:189-192, 2005). Therefore, an antagonist of hyaluronate receptor can also be included in the combination therapies of the invention.

In another example, the compositions and methods of the invention may include a leukocyte function antigen-1 (LFA-1) antagonist. LFA-1 is expressed on lymphocytes and plays a major role in the activation and trafficking of T-lymphocytes to the site of inflammation. LFA-1 has also been considered as a therapeutic target for MS (Avolio et al., J. Neurol. Sci. 186:65-73, 2001; Lujan et al., Mult. Sclerosis 4:239-242, 1998); therefore, an antagonist of LFA-1 may also be included in the combination therapies of the invention.

An antagonist of CD11/CD18 may also be used in conjuction with the compositions and methods of the present invention. CD11/CD18 is a heterodimeric integrin receptor which consists of one of three α subunits (CD11a, CD11b, or CD11c) and one β subunit (CD18). The CD11/CD18 receptor is involved in crucial leukocyte adhesion functions, including chemotaxis, phagocytosis, adhesion to the endothelium, aggregation, and cell-mediated cytotoxicity. One humanized monoclonal antibody to CD11/CD18 is already in phase I MS clinical trials (Bowen et al., Clin. Pharmacol. Ther. 64:339-346, 1998).

Additionally, the compositions and methods of the invention may include CD20 antagonist. CD20 is a B-lymphocyte surface molecule which plays a role in the development and differentiation of B-cells into plasma cell. Monoclonal antibodies to CD20 include rituxumab, ibritumomab tiuxetan, and tositumomab. Rituximab (Rituxan^{®}, by Genentech) is currently being studied in a multi-center phase II/III trial involving primary-progressive MS, and a phase II trial in secondary-progressive MS. A case report for the use of rituximab on MS was reported in Stuve et al. (Arch Neurol. 2005; 62:1620-1623). Therefore, an antagonist of CD20 can also be included in the combination therapies of the invention.

In another example, the compositions and methods of the invention may include a CD86 antagonist. CD86 (cluster of differentiation 86) is a protein that provides a costimulatory signal necessary for T cell activation and survival. CD86 principal mode of action is by binding to CD28. CD86 and CD80 provide the necessary stimuli to prime T cells against antigens presented by antigen-presenting cells. The CD86 pathway is currently being evaluated as a potential target for the treatment of MS. Therefore, an antagonist of CD86 can also be used in conjunction with the combination therapies of the invention.

The compositions and methods of the invention may also include an antagonist of ICOS ligand. ICOS ligand is a membrane-protein that is expressed on activated monocytes and dendritic cells. ICOS ligand functions as a costimulatory signal for T-cell proliferation and cytokine secretion and induces B-cell proliferation and differentiation into plasma cells. ICOS ligand may play an important role in mediating local tissue response to inflammatory conditions and may modulate the secondary immune response by co-stimulating memory T-cell function. Therefore, an antagonist to ICOS ligand can also be used in conjunction with the combination therapies of the invention.

The compositions and methods of the invention may also include an CCR2 (chemokine (C-C motif) receptor 2) antagonist. CCR2 is a chemokine receptor that mediates recruitment of both infiltrating macrophages and resident microglia to specific sites of central nervous system inflammation. ChemoCentryx has initiated a Phase 1 for the use of CCX915, a small molecule antagonist of CCR2, in the treatment of MS. Humanized anti-CCR2 antibodies are described in U.S. Patent No. 6,406,865, herein incorporated by reference. Therefore, an antagonist to CCR2 can also be used in conjunction with the combination therapies of the invention.

Additionally, the compositions and methods of the invention may include a CXCR3 antagonist. CXCR3 is a chemokine receptor that is expressed on activated T lymphocytes and NK cells. CXCR3 regulates leukocyte trafficking and the binding of chemokines to CXCR3 induces various cellular responses, most notably integrin activation, cytoskeletal changes and chemotactic migration. CXCR3-ligand interaction attracts Th 1 cells and promotes Th 1 cell maturation. CXCR3 has been implacated for a role in the development of MS. Several antagonists of CXCR3 are known in the art, including, small molecules (e.g., those described in WO 06/088837) and humanized antibodies (e.g., those described in WO 05/030793). Therefore, an antagonist to CXCR3 can also be used in conjunction with the combination therapies of the invention.

Finally, the compositions and methods of the invention may also include a CCR5 antagonist. CCR5 is a chemokine receptor expressed on T cells, macrophages, dendritic cells and microglia. CCR5 may play a role in inflammatory responses to infection. A role for CCR5 in the pathogenesis of MS has been suggested (Trebst et al., Am. J. Pathol. 159:1701-1710, 2001). Several antagonists to CCR5 are known in the art, including small molecule inhibitors (see, e.g., EP 1 539 695) and humanized antibodies (see, e.g., U.S. Patent No. 7,122,185, herein incorporated by reference). Therefore, an antagonist to CCR5 can also be used in conjunction with the combination therapies of the invention.

### Pharmaceutical Compositions

The present invention also relates to a pharmaceutical composition that contains a therapeutically effective amount of an AFP and/or an integrin antagonist. The active ingredients, AFP and an integrin antagonist, may be present in the same pharmaceutical composition (a single dosage form) or separate pharmaceutical compositions (separate dosage forms) to be administered coextensively or separately. In addition, the compsotion can include one or more different AFPs or integrin antagonists. The compositions can be formulated for use in a variety of drug delivery systems. One or more physiologically acceptable excipients or carriers can also be included in the compositions for proper formulation. Suitable formulations for use in the present invention are found in Remington's Pharmaceutical Sciences, Mack Publishing Company, Philadelphia, PA, 17th ed., 1985. For a brief review of methods for drug delivery, see, Langer, Science 249: 1527-1533, 1990.

The pharmaceutical compositions can be formulated for parenteral, intranasal, topical, oral, or local administration, such as by a transdermal means, for prophylactic and/or therapeutic treatment. Commonly, the pharmaceutical compositions are administered parenterally (e.g., by intravenous, intramuscular, or subcutaneous injection), by oral ingestion, or by topical application at areas affected by MS. Thus, the invention features compositions for parenteral administration that include an AFP and/or an integrin antagonist dissolved or suspended in an acceptable carrier, preferably an aqueous carrier, e.g., water, buffered water, saline, PBS, and the like. The compositions may contain pharmaceutically acceptable auxiliary substances as required to approximate physiological conditions, such as pH adjusting and buffering agents, tonicity adjusting agents, wetting agents, detergents, and the like. The invention also features compositions for oral delivery, which may contain inert ingredients such as binders or fillers for the formulation of a tablet, a capsule, and the like. Furthermore, this invention features compositions for local administration, which may contain inert ingredients such as solvents or emulsifiers for the formulation of a cream, an ointment, and the like. In different embodiments of the invention, the AFP and the integrin antagonist may be administered in the same or separate compositions for administration via the same or two different routes of administration.

Compositions of the invention may be sterilized by conventional sterilization techniques or they may be sterile filtered. The resulting aqueous solutions may be packaged for use as is, or lyophilized, the lyophilized preparation being combined with a sterile aqueous carrier prior to administration. The pH of the preparations typically will be between 3 and 11, more preferably between 5 and 9 or between 6 and 8, and most preferably between 7 and 8, such as 7 to 7.5. The resulting compositions in solid form may be packaged in multiple single dose units, each containing a fixed amount of an AFP and/or an integrin antagonist, such as in a sealed package of tablets or capsules (e.g., a blister pack). The composition in solid form can also be packaged in a container for a flexible quantity, such as in a squeezable tube designed for a topically applicable cream or ointment.

The compositions of the invention containing an effective amount of an AFP and/or an integrin antagonist can be administered for prophylactic and/or therapeutic treatments. In prophylactic applications, compositions of the invention containing an AFP and/or an integrin antagonist are administered to a patient susceptible to or otherwise at risk of developing MS. Such an amount is defined to be a "prophylactically effective dose." In this use, the precise amounts again depend on the patient's state of health, but generally range from about 0.5 mg to about 400 mg of an AFP per dose (e.g., 10 mg, 50 mg, 100 mg, 200 mg, 300 mg, or 400 mg per dose) and from about 0.1 mg to about 500 mg of an integrin antagonist per dose (e.g., 10 mg, 50 mg, 100 mg, 200 mg, 300 mg, 400 mg, or 500 mg per dose). A dose of the AFP and/or integrin antagonist can be administered prophylactically to a patient one or more times per hour, day, week, month, or year (e.g., 2, 4, 5, 6, 7, 8, 9, 10, 11, or 12 times per hour, day, week, month, or year). More commonly, a single dose per week of an AFP and/or an integrin antagoist is administered to a patient.

In therapeutic applications, compositions of the invention can be administered to a patient already suffering from MS in an amount sufficient to cure or at least partially arrest one or more of the symptoms of the disease and their complications. An amount adequate to accomplish this purpose is defined as a "therapeutically effective dose." Amounts effective for this use may depend on the severity of the disease or condition and the general state of the patient, but may range from about 0.5 mg to about 400 mg of an AFP per dose (e.g., 10 mg, 50 mg, 100 mg, 200 mg, 300 mg, or 400 mg per dose) and from about 0.1 mg to about 500 mg of an integrin antagonist per dose (e.g., 10 mg, 50 mg, 100 mg, 200 mg, 300 mg, 400 mg, or 500 mg per dose).

In several embodiments, the patient may receive an AFP (with or without an integrin antagonist) in the range of about 0.5 to about 400 mg per dose one or more times per week (e.g., 2, 3, 4, 5, 6, or 7 or more times per week), preferably about 5 mg to about 300 mg per dose one or more times per week, and even more preferably about 5 mg to about 200 mg per dose one or more times per week. The patient may also receive a biweekly dose of an AFP in the range of about 50 mg to about 800 mg or a monthly dose of an AFP in the range of about 50 mg to about 1,200 mg.

In other embodiments, an AFP may be administered to a patient in a typical dosage range of about 0.5 mg to about 400 mg per dose per week, about 1.0 mg to about 300 mg per dose per week, about 5 mg to about 200 mg per dose per week, about 10 mg to about 100 mg per dose per week, about 20 mg to about 80 mg per dose per week, about 100 mg to about 300 mg per dose per week, or about 100 mg to about 200 mg per dose per week. An AFP may be administered in the range of about 0.5 mg to about 100 mg per dose every other day, preferably about 5 mg to about 75 mg per dose every other day, more preferably about 10 mg to about 50 mg per dose every other day, and even more preferably 20 mg to about 40 mg per dose every other day. An AFP may also be administered in the range of about 0.5 mg to about 100 mg per dose three times per week, preferably about 5 mg to about 75 mg per dose three times per week, more preferably about 10 mg to about 50 mg per dose three times per week, and even more preferably about 20 mg to about 40 mg per dose three times per week.

In several embodiments, the patient may receive an integrin antagonist (with or without an AFP) in the range of about 0.1 to about 500 mg per dose per one or more times per week (e.g., 2, 3, 4, 5, 6, or 7 or more times per week), preferably about 0.1 mg to about 400 mg per dose one or more times per week, or about 0.1 mg to about 300 mg per dose one or more times per week, more preferably about 1 mg to about 200 mg per dose one or more times per week, or most preferably about 5 mg to about 100 mg per dose one or more times per week. The patient may also receive a biweekly, triweekly, or monthly dose of an integrin antagonist in the range of about 0.1 mg to about 1.5 g, preferably a dose in the range of about 1 mg to about 1,000 mg, more preferably a dose in the range of about 5 mg to about 800 mg. Preferably, the dose of an integrin antagonist (e.g., natalizumab) is about 300 mg per dose every four weeks.

In some embodiments where the integrin antagonist administered is natalizumab, the patient receives a typical dosage in the range of about 15 µg to about 150 mg per dose per week, preferably about 1 mg to about 120 mg per dose per week, more preferably about 2 mg to about 100 mg per dose per week, and even more preferably about 5 mg to 80 mg per dose per week. In another embodiment, natalizumab is administered in the range of about 200 mg to 400 mg per dose every four weeks. The patient may also receive an AFP polypeptide in the range of about 0.5 mg to about 200 mg per dose per week, preferably about 5 mg to about 100 mg per dose per week, more preferably about 10 mg to about 80 mg per dose per week, and even more preferably about 20 mg to about 70 mg per dose per week.

A dose of the AFP and/or integrin antagonist can be administered therapeutically to a patient one or more times per hour, day, week, month, or year (e.g., 2, 4, 5, 6, 7, 8, 9, 10, 11, or 12 times per hour, day, week, month, or year). More commonly, a single dose per week of an AFP and/or an integrin antagonist is administered to a patient.

In non-limiting embodiments of the methods of the present invention, an AFP and an integrin antagonist are administered to a patient: continuously for 1, 2, 3, or 4 hours; 1, 2, 3, or 4 times a day; every other day or every third, fourth, fifth, or sixth day; 1, 2, 3, 4, 5, 6, 7, 8, 9, or 10 times a week; biweekly; 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 times a month; bimonthly; 1, 2, 3, 4, 5, 6, 7, 8, 9, or 10 times every six months; 1, 2, 3, 4, 5, 6, 7, 8, 9, 10, 11, 12, 13, 14, 15, 16, 17, 18, 19, or 20 times a year; or biannually. The AFP (or biologically active fragment thereof) and the integrin antagonist may be administered at different frequencies during a therapeutic regime (i.e., administered at a higher frequency in the later stages of MS (e.g., administered once a week in the initial stages of MS and administered three times a week a later stage of MS) or administered at a higher frequency in the early stages of MS (e.g., administered three times a week during the initial stages of MS and administered once a week at a later stage of MS)). In additional embodiments, the AFP and the integrin antagonist may be administered to a patient at the same frequency or at a different frequency.

The amount of integrin antagonist and AFP polypeptide required to achieve the desired therapeutic effect depends on a number of factors, such as the specific integrin antagonist(s) chosen, the mode of administration, and clinical condition of the recipient. A skilled artisan will be able to determine the appropriate dosages of integrin antagonist and AFP (or biologically active fragment thereof) to achieve the desired results.

The coadministration of an AFP and an integrin antagonist according to the methods of this invention refers to the use of the two active ingredients in the same general time period or using the same general administration method. It is not always necessary, however, to administer both at the exact same time. For instance, if an AFP and an integrin antagonist are administered to a patient suffering from MS in two separate pharmaceutical compositions, the two compositions need not be delivered to the patient during the same time period or even during two partially overlapping time periods. In some cases, the administration of the second agent (e.g., an AFP) may begin shortly after completion of the administration period for the first agent (e.g., an integrin antagonist, such as natalizumab), or *vice versa.* The time gap between the two administration periods may vary from one or more hours, days, weeks, or months. In some cases, one therapeutic agent (e.g., an AFP) may be administered first with the second (e.g., an integrin antagonist, such as natalizumab) in a separate time period, and subsequently administered without the second in a following period. A typical schedule of this type may require a higher dosage of the first therapeutic agent in the first, co-administration period, and a lower dosage in the second period, and *vice versa.* The same applies for the second agent.

Single or multiple administrations of the compositions of the present invention that include an effective amount of an AFP and/or an integrin antagonist can be carried out with the dose levels and the pattern being selected by the treating physician. The dose and administration schedule can be determined and adjusted based on the severity of MS in a patient, which may be monitored throughout the course of treatment according to the methods commonly practiced by clinicians or those described herein.

In addition to an AFP and/or an integrin antagonist, the composition may also include an antagonist to CD80, P-selectin, sphingosine-1-phosphate receptor-1, hyaluronate receptor, LFA-1, CD11/CD18, CD20, CD86, ICOS ligand, CCR2, CXCR3, CCR5, CD40, CD154, CD28, IL-23, IL-17, or IL-6. The antagonist to CD80, P-selectin, sphingosine-1-phosphate receptor-1, hyaluronate receptor, LFA-1, CD11/CD18, CD20, CD86, ICOS ligand, CCR2, CXCR3, CCR5, CD40, CD154, CD28, IL-23, IL-17, or IL-6 may be an antibody, a binding peptide, a nucleic acid inhibitor, or a small molecule inhibitor; which can be identified according to the same methods described above with regard to an integrin antagonist.

The patient may receive an antagonist to CD80, P-selectin, sphingosine-1-phosphate receptor-1, hyaluronate receptor, LFA-1, CD11/CD18, CD20, CD86, ICOS ligand, CCR2, CXCR3, CCR5, CD40, CD154, CD28, IL-23, IL-17, or IL-6 in the range of about 0.1 to about 500 mg per dose per one or more times per hour, day, week, or month (e.g., 2, 3, 4, 5, 6, or 7 times per hour, day, week, or month), about 0.1 to about 400 mg per dose one or more times per week, about 0.1 to about 300 mg per dose one or more times per week, about 1 to about 200 mg per dose one or more times per week, or about 5 to about 100 mg per dose one or more times per week. The patient may also receive a biweekly, triweekly, or monthly dose of an antagonist to CD80, P-selectin, sphingosine-1-phosphate receptor-1, hyaluronate receptor, LFA-1, CD11/CD18, CD20, CD86, ICOS ligand, CCR2, CXCR3, CCR5, CD40, CD154, CD28, IL-23, IL-17, or IL-6 in the range of about 0.1 mg to about 1.5 g, preferably a dose in the range of about 1 mg to about 1,000 mg, more preferably a dose in the range of about 5 mg to about 800 mg.

### Kits of the Invention

The invention also features kits for treating or reducing the symptoms of, or severity of, MS according to the combination treatment method of the present invention. The kits typically include a pharmaceutical composition containing an AFP and a pharmaceutical composition containing an integrin antagonist, each in a therapeutically effective amount for treating MS. In one example, effective amounts of an AFP and an integrin antagonist can be present in a single pharmaceutical composition. Optionally, the pharmaceutical composition(s) may contain one or more pharmaceutically acceptable excipients.

Preferably, the kits include multiple packages of the single-dose pharmaceutical composition(s) containing an effective amount of an AFP and/or an integrin antagonist. Optionally, instruments or devices necessary for administering the pharmaceutical composition(s) may be included in the kits. For instance, a kit of this invention may provide one or more prefilled syringes containing an effective amount of an AFP and one or more prefilled syringes containing an effective amount of an integrin antagonist. Alternatively, the kit may provide one or more prefilled syringes containing an effective amount of an AFP and tablets containing a dosage of an integrin antagonist. Furthermore, the kits may also include additional components such as instructions or administration schedules for a patient suffering from MS to use the pharmaceutical composition(s) containing an AFP and/or an integrin antagonist.

In addition to an AFP and/or an integrin antagonist, the kit may also include an antagonist of CD80, P-selectin, sphingosine-1-phosphate receptor-1, hyaluronate receptor, LFA-1, CD11/CD18, CD20, CD86, ICOS ligand, CCR2, CXCR3, CCR5, CD40, CD154, CD28, IL-23, IL-17, or IL-6. The antagonist of CD80, P-selectin, sphingosine-1-phosphate receptor-1, hyaluronate receptor, LFA-1, CD11/CD18, CD20, CD86, ICOS ligand, CCR2, CXCR3, CCR5, CD40, CD154, CD28, IL-23, IL-17, or IL-6 may be an antibody, a binding peptide, a nucleic acid inhibitor, or a small molecule inhibitor.

It will be apparent to those skilled in the art that various modifications and variations can be made in the compositions, methods, and kits of the present invention without departing from the spirit or scope of the invention. Thus, it is intended that the present invention cover the modifications and variations of this invention provided they come within the scope of the appended claims and their equivalents.

The following examples are meant to illustrate the invention and should not be construed as limiting.

### EXAMPLES

The following two examples are provided by way of illustration only and not by way of limitation. Those skilled in the art will readily recognize a variety of noncritical parameters that could be changed or modified to yield essentially the same or similar results.

### Example 1

### Functional Test of a Recombinant AFP Using MOG-EAE Mouse Model

Efficacy experiments of a recombinant version of human AFP (recombinant human AFP, rhAFP, produced according to U.S. Patent Application Publication No. 20040098755) were performed in a mouse model in which experimental autoimmune encephalomyelitis (EAE) is induced by immunization of susceptible strains of mice with myelin antigen or peptides (myelin oligodendrocyte protein [MOG] or proteolipid protein [PLP]). This assay system is useful for determining the functionality of an AFP of this invention.

**Purpose of Study:** The purpose of these studies was to test compounds intended as therapeutics for MS, an autoimmune disease directly associated with the major histocompatibility complex (MHC) class II molecule HLA-DR2. The mouse experimental autoimmune encephalomyelitis (EAE) model was chosen for its relevance to human MS.

**EAE Model Description and Features:** Experimental Allergic Encephalomyelitis (EAE) is a demyelinating disease of the central nervous system. It serves as the animal model MS (Goverman, Lab. Anim. Sci. 46:482, 1996; and Paterson, Clin. Immunol. Rev. 1:581, 1981). EAE can assume an acute, chronic, or relapsing-remitting disease course that is dependent upon the method of induction and type of animal used. Disease induction results in escalating degrees of ascending animal paralysis. The resulting paralysis is debilitating, but not painful, and most animals will show some degree of recovery even from advanced stages of EAE. Paralysis usually begins with a weakened tail, gradually followed by hind limb weakness progressing to paralysis, and less frequently front limb paralysis. EAE disease progression can be monitored with a scoring system that starts with the normal condition and ends when the mice become moribund. Since the severity of the disease varies from animal to animal there is no way to reliably predict whether an animal will recover. As a result, close monitoring is needed in this animal model.

EAE can be induced with components of the central nervous system (Levine and Sowinski, J. Immunol. 110:139, 1973; and Fritz et al., J. Immunol. 130:1024, 1983) or peptides (Tuohy et al., J. Immunol. 140:1868, 1988; McFarlin et al., Science 179:478, 1973; and Linington et al., Eur. J. Immunol. 23:1364, 1993) and also via T cell transfer from one animal to another animal (Yamamura et al., J. Neurol. Sci. 76:269, 1986). Complete Freund's adjuvant (CFA) must be used with the proteins or peptides to effectively trigger the autoimmune response. CFA is often used in combination with pertussis toxin (Lee, Proc. Soc. Exp. Biol. Med. 89:263, 1955; and Kamradt et al., J. Immunol. 147:3296, 1991) to increase the efficiency of immunization. It is not possible to administer analgesics to lessen any pain that may be associated with the CFA injections, as most analgesics affect the immune response that is an essential component of the model (Billiau, J. Leukoc. Biol. 70:849, 2001; and Naiki et al., Int. J. Immunopharmacol. 13:235, 1991).

### Experimental Design and Methods

**Induction of experimental MS-like disease syndrome:** 50 female mice (C57BL6) between 6 and 8 weeks of age, were immunized subcutaneously on day 0 (left paralumbar region) and day 7 (right paralumbar region) with an emulsion (125 µg per mouse) of myelin oligodendrocyte glycoprotein (mMOG-35-55 peptide) in CFA containing heat-killed *Mycobacterium tuberculosis* H37RA. In addition, mice were given pertussis toxin (Ptx) intraperitonealy on days 0 and 2 post-immunization.

**Disease monitoring:** The initial signs of disease (weakened tail or paralysis) were observed beginning ∼10 days after the first immunization. Actively immunized mice were assessed daily through day 30 for clinical signs of EAE according to an established scale:
0 No disease
1 Tail weakness
2 One or two weak hind limbs, sufficient to impair righting
3 One of two hind limb paralysis
4 One or two front limb paralysis
5 Moribund or dead

The 50 mice were randomized into 5 groups of 10 mice each. One group of 10 animals received a saline injection to serve as an untreated EAE disease control. Four compounds were evaluated in the remaining 4 groups.

Mice were injected with 100 µl of test rhAFP or control material IP daily. These compounds are: 1-500 µg rhAFP or 1-500 µg human serum albumin (control). Furthermore, depleting antibodies to specific leukocyte subsets (e.g., CD4⁺ cells) are employed as additional control(s) in some studies.

Mice were used in this study to assess the effect of rhAFP on disease progression in an experimental model of MS (EAE). Without treatment it was expected that many of the animals would develop signs and symptoms of EAE, namely progressive encephalopathy and paralysis.

In addition to daily monitoring of the animals for disease progression over a 30-day time course, animals were sacrificed at the end of the study and central nervous system tissues (brain and spinal cord) were harvested for immunohistochemical analysis of infiltrating, disease-causing cells (i.e., CD4⁺ T cells).

Additionally, six to ten-day short-term studies were employed to assess the effect(s) of rhAFP administration on the induction phase of disease. In these shorter studies, draining lymph node cells were harvested for FACs analysis of immunologic cell subsets including but not limited to: T cells, CD4⁺ cells, regulatory T cells, and their activation markers. A fraction of harvested cells from each treatment group were assessed for *in vitro* proliferative response to a panel of stimuli to assess Ag-specific recall response to the immunizing antigen (Ag), MOG35-55, and Ag-nonspecific responses to a panel of mitogens (Concanavalin A, PHA, and LPS). Supernatants from cultures set-up in the same fashion are analyzed for cytokines (e.g., IL-2, IL-4, or IFNγ).

### Example 2

### Effect of AFP and an integrin antagonist in MOG-EAE Mouse Model

The synergistic effect of recombinant human AFP and an integrin antagonist (e.g,. an antibody, such as a surrogate anti-mouse antibody (e.g., an anti-VLA-4 antibody or a rat anti-mouse antibody, such as PS/2)) for treating EAE is tested in a study utilizing the MOG-EAE or PLP-EAE mouse model for MS.

The general experimental design is identical to Example 1. Briefly, 70 female mice (C57BL6) between 6 and 8 weeks of age are immunized subcutaneously on day 0 (left paralumbar region) and day 7 (right paralumbar region) with an emulsion (125 µg per mouse) of myelin oligodendrocyte glycoprotein (mMOG-35-55 peptide) in CFA containing heat-killed *Mycobacterium tuberculosis* H37RA.

The 70 mice are randomized into 7 groups of 10 mice each. One group of 10 animals receives a saline injection to serve as an untreated EAE disease control. Six different formulations are evaluated in the remaining 6 groups. The mice of group 1 receive a placebo; group 2 receives rhAFP at 10 µg/day; group 3 receives rhAFP at 100 µg/day; group 4 receives the integrin antagonist at 10 µg/day; group 5 receives the integrin antagonist at 100 µg/day; group 6 receives both rhAFP and the integrin antagonist at 10 µg/day and 10 µg/day, respectively; and group 7 receives both rhAFP and the integrin antagonist at 100 µg/day and 100 µg/day, respectively. The experimental design can alternatively include modifying (e.g., increasing or decreasing) the dosages of one or both of the rhAFP and the integrin antagonist during the administration period. For example, the mice could initially be administered a constant dose of rhAFP and an escalating dose (e.g., 0.1, 1.0, 10, 20, 50, or 100 µg/mouse given every other day) of the integrin antagonist (e.g., a mouse anti-α4 mAb) to determine if rhAFP enables the administration of a suboptimal dose of the integrin antagonist. As an alternative, the mice could initially be administered a constant dose of the integrin antagonist and an escalating dose of rhAFP.

The administration is by daily injections (interperitoneally or subcutaneously) from day 0 until the end of experiment at between days 40 and 60. All groups are scored daily for disease symptoms according to the scale as described in Example 1 for the duration of the study.

All mice are euthanized between days 40 and 60, and various organs and blood (e.g., spleen, knees, and hind and fore paws) are harvested for immuno-histochemistry and immunological analysis.

### Other Embodiments

While the invention has been described in connection with specific embodiments thereof, it will be understood that it is capable of further modifications and this application is intended to cover any variations, uses, or adaptations of the invention following, in general, the principles of the invention and including such departures from the present disclosure that come within known or customary practice within the art to which the invention pertains and may be applied to the essential features hereinbefore set forth.

All publications and patent applications mentioned in this specification are herein incorporated by reference to the same extent as if each independent publication or patent application was specifically and individually indicated to be incorporated by reference in their entirety.

The invention further includes the subject matter of the claims of WO2008103378 from which this application is derived, the content of which is reproduced below as numbered paragraphs.

Paragraph 1. A method of treating a patient with multiple sclerosis comprising administering to said patient alpha-fetoprotein (AFP) or a biologically active fragment thereof and an integrin antagonist.

Paragraph2. The method of paragraph 1, wherein said AFP or biologically active fragment thereof is recombinant human AFP.

Paragraph 3. The method of paragraph 1, wherein said AFP or biologically active fragment thereof is non-glycosylated.

Paragraph 4. The method of paragraph 1, wherein said integrin antagonist is an antibody, a blocking peptide, a nucleic acid inhibitor, or a small molecule inhibitor.

Paragraph 5. The method of paragraph 4, wherein said antibody is an anti-α4 integrin antibody.

Paragraph 6. The method of paragraph 5, wherein said anti-α4 integrin antibody is natalizumab.

Paragraph 7. The method of paragraph 1, wherein said method further comprises administering an antagonist of one or more of the following proteins: CD80, P-selectin, sphingosine-1-phosphate receptor-1, hyaluronate receptor, leukocyte function antigen-1 (LFA-1), CD11/CD18, CD20, CD86, ICOS ligand, CCR2, CXCR3, CCR5, CD40, CD154, CD28, IL-23, IL-17, and IL-6.

Paragraph 8. The method of paragraph 7, wherein said antagonist of CD80, P-selectin, sphingosine-1-phosphate receptor-1, hyaluronate receptor, leukocyte function antigen-1 (LFA-1), CD11/CD18, CD20, CD86, ICOS ligand, CCR2, CXCR3, CCR5, CD40, CD154, CD28, IL-23, IL-17, or IL-6 is an antibody, a blocking peptide, a nucleic acid inhibitor, or a small molecule inhibitor.

Paragraph 9. The method of paragraph 1, wherein said AFP or biologically active fragment thereof, or said integrin antagonist is administered intravenously, intramuscularly, orally, by inhalation, parenterally, intraperitoneally, intraarterially, transdermally, sublingually, nasally, through use of suppositories, transbuccally, liposomally, adiposally, intraocularly, subcutaneously, intrathecally, topically, or through local administration.

Paragraph 10. The method of paragraph 1, wherein said AFP or biologically active fragment thereof, or said integrin antagonist is administered one or more times hourly, daily, weekly, biweekly, or monthly.

Paragraph 11. The method of paragraph 1, wherein said AFP or biologically active fragment thereof, and said integrin antagonist are administered coextensively or separately.

Paragraph 12. The method of paragraph 1, wherein said AFP or biologically active fragment thereof, and said integrin antagonist are administered in separate dosage forms.

Paragraph 13. The method of paragraph 1, wherein said AFP or biologically active fragment thereof and said integrin antagonist are administered in the same dosage form.

Paragraph 14. The method of paragraph 1, wherein said AFP or biologically active fragment thereof and said integrin antagonist are administered via two different routes of administration.

Paragraph 15. The method of paragraph 1, wherein said AFP or biologically active fragment thereof and said integrin antagonist are administered via the same route of administration.

Paragraph 16. The method of paragraph 1, wherein said AFP or biologically active fragment thereof is administered in the range of 0.5 mg to 400 mg per dose.

Paragraph 17. The method of paragraph 1, wherein said integrin antagonist is administered in the range of 0.1 mg to 500 mg per dose.

Paragraph 18. The method of paragraph 1, wherein said AFP or biologically active fragment thereof is administered prior to said integrin antagonist.

Paragraph 19. The method of paragraph 1, wherein said AFP or biologically active fragment thereof is administered after said integrin antagonist.

Paragraph 20. The method of paragraph 1, wherein said administering results in a loss of or reduction in severity of one or more symptoms of multiple sclerosis.

Paragraph 21. The method of paragraph 20, wherein said one or more symptoms of multiple sclerosis are selected from the group consisting of tingling, numbness, tremors, loss of balance, weakness in one or more limbs, blurred or double vision, slurred speech, swallowing problems, paralysis, lack of coordination, cognitive difficulties, fatigue, muscle spasms, dizziness, breathing problems, and seizures.

Paragraph 22. A composition comprising an AFP or a biologically active fragment thereof and an integrin antagonist.

Paragraph 23. The composition of paragraph 22, wherein said composition further comprises an antagonist to one or more of the following proteins: CD80, P-selectin, sphingosine-1-phosphate receptor-1, hyaluronate receptor, leukocyte function antigen-1 (LFA-1), CD11/CD18, CD20, CD86, ICOS ligand, CCR2, CXCR3, CCR5, CD40, CD154, CD28, IL-23, IL-17, and IL-6.

Paragraph 24. The composition of paragraph 22, wherein said AFP or biologically active fragment thereof is recombinant human AFP.

Paragraph 25. The composition of paragraph 22, wherein said AFP or biologically active fragment thereof is non-glycosylated.

Paragraph 26. The composition of paragraph 22, wherein said integrin antagonist is an antibody, a blocking peptide, a nucleic acid inhibitor, or a small molecule inhibitor.

Paragraph 27. The composition of paragraph 22, wherein said integrin antagonist is natalizumab.

Paragraph 28. The composition of paragraph 22, wherein said composition is formulated for intravenous, intramuscular, oral, parenteral, intraperitoneal, intraarterial, transdermal, sublingual, nasal, transbuccal, liposomal, adiposal, intraocular, subcutaneous, intrathecal, topical, or through suppository, inhalation, or local administration.

Paragraph 29. The composition of paragraph 22, wherein said AFP or biologically active fragment thereof is in a dose of between 0.5 mg and 400 mg and said integrin antagonist is in a dose of between 0.1 mg to 500 mg.

Paragraph 30. A kit comprising an AFP or a biologically active fragment thereof and an integrin antagonist, and instructions for administration to said patient.

Paragraph 31. The kit of paragraph 30, wherein said AFP or biologically active fragment thereof is recombinant human AFP.

Paragraph 32. The kit of paragraph 30, wherein said APF or biologically active fragment thereof is non-glycosylated.

Paragraph 33. The kit of paragraph 30, wherein said integrin antagonist is natalizumab

Paragraph 34. The kit of paragraph 30, wherein said AFP or biologically active fragment thereof or said integrin antagonist is formulated for intravenous, intramuscular, oral, parenteral, intraperitoneal, intraarterial, transdermal, sublingual, nasal, transbuccal, liposomal, adiposal, intraocular, subcutaneous, intrathecal, topical, or through suppository, inhalation, or local administration.

35. The kit of paragraph 30, wherein said AFP or biologically active fragment thereof and said integrin antagonist are present in the same composition.

Paragraph 36. The kit of paragraph 30, wherein said AFP or biologically active fragment thereof and said integrin antagonist are formulated in separate compositions.

Paragraph 37. The kit of paragraph 30, wherein said AFP or biologically active fragment thereof and said integrin antagonist are formulated for two different routes of administration.

Paragraph 38. The kit of paragraph 30, wherein said AFP or biologically active fragment thereof and said integrin antagonist are formulated for the same route of administration.

## Claims

1. A composition comprising an alpha-fetoprotein (AFP) or a biologically active fragment thereof and an integrin antagonist for use in treating, preventing or reducing one or more symptoms or progression of multiple sclerosis in a patient in need thereof.

2. A method for determining the antagonistic activity of a test compound, the method comprising the steps of:
(i) adding the test compound in increasing concentration to Jurkat T- cells;
(ii) adding the mixture of (i) to plates containing interleukin-1 stimulated endothelial cell monolayers;
(iii) incubating and washing said plates; and
(iv) quantitating the number of Jurkat T-cells attached to the plate.

3. A composition comprising an alpha-fetoprotein (AFP) or a biologically active fragment thereof for use in treating multiple sclerosis in a patient in need thereof in combination with a composition comprising an integrin antagonist selected from natalizumab.

4. A composition comprising an integrin antagonist selected from natalizumab for use in treating multiple sclerosis in a patient in need thereof in combination with a composition comprising an alpha-fetoprotein (AFP), or biologically active fragment thereof.

5. The composition of any one of claims 1, 3 or 4, wherein said composition further comprises an antagonist to one or more of the following proteins: CD80, P-selectin, sphingosine-1-phosphate receptor-1, hyaluronate receptor, leukocyte function antigen-1 (LFA-1), CD11/CD18, CD20, CD86, ICOS ligand, CCR2, CXCR3, CCR5, CD40, CD154, CD28, IL,-23, IL-17, and IL-6.

6. The composition of any one of claims 1, 3 or 4, wherein said AFP or biologically active fragment thereof is recombinant human AFP, or is non-glycosylated.

7. The composition of any one of claims 1, 3 or 4, wherein said composition is formulated for intravenous, intramuscular, oral, parenteral, intraperitoneal, intraarterial, transdermal, sublingual, nasal, transbuccal, liposomal, adiposal, intraocular, subcutaneous, intrathecal, topical, or through suppository, inhalation, or local administration.

8. The composition of any one of claims 1, 3 or 4, wherein said one or more symptoms of multiple sclerosis are selected from the group consisting of tingling, numbness, tremors, loss of balance, weakness in one or more limbs, blurred or double vision, slurred speech, swallowing problems, paralysis, lack of coordination, cognitive difficulties, fatigue, muscle spasms, dizziness, breathing problems, and seizures.

9. A medicament comprising the composition of any one of claims 1, 3 or 4 and a pharmaceutically acceptable carrier for use in treating a patient with multiple sclerosis.

10. The composition of claim 3 or claim 4, wherein said AFP, or biologically active fragment thereof, and said integrin antagonist are for use in a single dosage form.

11. The composition of claim 3 or claim 4, wherein said AFP, or biologically active fragment thereof, and said integrin antagonist are for use in separate dosage forms.

12. The composition of claim 3 or claim 4, for use in an hourly, daily, weekly, biweekly, or monthly administration regimen.
